# FASCICULE DE BREVET EUROPEEN

(11) **EP 0 969 798 B1**
(45) Date de publication et mention de la délivrance du brevet: **23.07.2003**
(21) Numéro de dépôt: 98946516.6
(22) Date de dépôt: 28.09.1998
(51) Int. Cl.: A61K 7/13

(54) **COMPOSITION DE TEINTURE D'OXYDATION DES FIBRES KERATINIQUES ET PROCEDE DE TEINTURE METTANT EN OEUVRE CETTE COMPOSITION**
ZUSAMMENSETZUNG ZUR OXIDATIONSFÄRBUNG VON KERATINISCHEN FASERN UND FÄRBEVERFAHREN MIT DIESER ZUSAMMENSETZUNG
OXIDATION DYEING COMPOSITION FOR KERATIN FIBRES AND DYEING METHOD USING SAID COMPOSITION

(30) Priorité: 03.10.1997 FR 9712353
(43) Date de publication de la demande: 12.01.2000
(73) Titulaire: L'OREAL, 75008 Paris (FR)
(72) Inventeur: DE LA METTRIE, Roland, F-78110 Le Vesinet (FR); COTTERET, Jean, F-78480 Verneuil-sur-Seine (FR); DE LABBEY, Arnaud, F-93600 Aulnay sous Bois (FR); MAUBRU, Mireille, F-78400 Chatou (FR)
(74) Mandataire: Goulard, Sophie
(86) Numéro de dépôt international: FR9802075
(87) Numéro de publication internationale: WO99017730

(56) Documents cités:
- EP-A- 0 310 675
- EP-A- 0 716 846
- WO-A-94/00100

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation, au moins un colorant direct cationique, et au moins une enzyme de type oxydo-réductase à 2 électrons en présence d'au moins Un donneur pour ladite enzyme, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les précurseurs de colorants d'oxydation et les coupleurs, des colorants directs, c'ést à dire des substances colorées qui apportent une coloration en l'absence d'agent oxydant.

Ces colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant une base d'oxydation et éventuellement un coupleur, en association avec des enzymes telles que la pyranose-oxydase, la glucose-oxydase ou bien l'uricase, en présence d'un donneur pour lesdites enzymes. Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, conduisent à des colorations ne donnant pas entière satisfaction notamment du point de vue de leur puissance, de leur chromaticité et de leur résistance vis à vis des diverses agressions que peuvent subir les cheveux.

Or, la demanderesse vient maintenant de découvrir qu'il est possible d'obtenir de nouvelles teintures, capables de conduire à des colorations puissantes et chromatiques, sans engendrer de dégradation significative des fibres kératiniques, peu sélectives et résistant bien aux diverses agressions que peuvent subir les fibres, en associant au moins une base d'oxydation, au moins un colorant direct cationique, et au moins une enzyme de type oxydo-réductase à 2 électrons en présence d'au moins un donneur pour ladite enzyme.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation,
- au moins un colorant direct cationique,
- au moins une enzyme de type oxydo-réductase à 2 électrons,
- et au moins un donneur pour ladite enzyme.

La composition tinctoriale prête à l'emploi conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

La ou les oxydo-réductases à 2 électrons utilisées dans la composition tinctoriale prête à l'emploi conforme à l'invention peuvent notamment être choisies parmi les pyranose oxydases, les glucose oxydases, les glycérol oxydases, les lactates oxydases, les pyruvate oxydases, et les uricases.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition tinctoriale prête à l'emploi, et encore plus préférentiellement de 0,1 à 5 % en poids environ de ce poids.

Selon l'invention, on entend par donneur, les différents substrats participant au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; et enfin à titre de donneur pour les uricases, on peut citer l'acide urique et ses sels.

Le ou les donneurs (ou substrats) utilisés conformément à l'invention représentent de préférence de 0,01 à 20 % en poids environ du poids total de la composition tinctoriale prête à l'emploi conforme à l'invention et encore plus préférentiellement de 0,1 à 5 % en environ de ce poids.

La nature de la ou des bases d'oxydation utilisées dans la composition tinctoriale prête à l'emploi n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide : 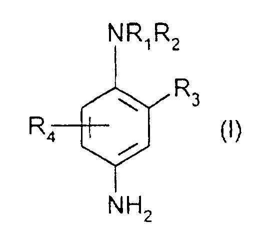 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide: 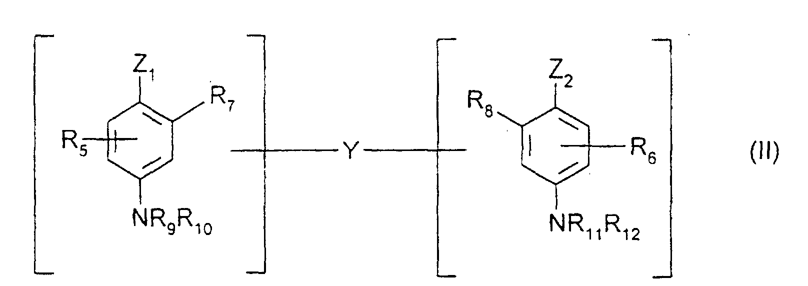 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide : 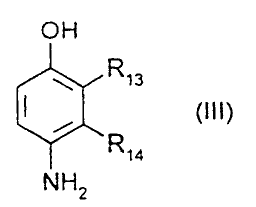 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole. le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique : 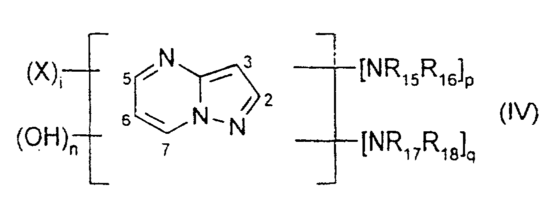 dans laquelle :
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C₄)alkyl ou di-[hydroxy(C₁-C₄ )alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Lorsque les pyrazolo-[1,5-a]-pyrimidines de formule (IV) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

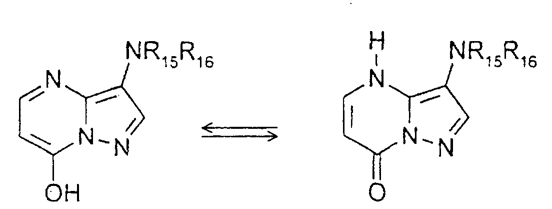

Parmi les pyrazolo-[1,5-a]-pyrimidines de formule (IV) ci-dessus on peut notamment citer
- la pyrazolo-[1,5-a]-pyrimidine-3,7-diamine ;
- la 2,5-diméthyl pyrazolo-[1,5-a]-pyrimidine-37-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-AI, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (IV) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Almera, J. Elguero, J. Heterocyclic Chem., 11(3), 423, 1974.
- K. Saito, I. Hori, M. Higarashi, H. Midorikawa, Bull. Chem. Soc. Japan, 47(2), 476, 1974.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

Le ou les colorants directs cationiques pouvant être utilisés dans la composition tinctoriale prête à l'emploi conforme à l'invention sont de préférence choisis parmi les amino-anthraquinoniques cationiques, les mono- ou di-azoïques cationiques, les naphtoquinones cationiques.

A titre de d'exemple, on peut notamment citer le chlorure de [8-[(p-aminophényl)azol]-7-hydroxy-2-naphtyl]triméthylammonium (également appelé Basic Brown 16 ou Arianor Mahogany 306002 dans le Color Index), le chlorure de 3-[(4-amino-6-bromo-5,8-dihydro-1-hydroxy-8-imino-5-oxo-2-naphtalényl)-amino]-N,N,N-triméthyl-benzénaminium (également dénommé Basic Blue 99 ou Arianor Steel Blue 306004 dans le Color Index), le chlorure de 7-hydroxy-8-[(2-méthoxyphényl)azo]-N,N,N-triméthyl-2-naphtalènaminium (également appelé le Basic Red 76 ou Arianor Madder Red dans le Color Index), le chlorure de [8-[(4-amino-2-nitrophényl)azo]-7-hydroxy-2-naphtyl]triméthylammonium (également appelé Basic Brown 17 ou Arianor Sienna Brown 306001 dans le Color Index) et le chlorure de 3-[(4,5-dihydro-3-méthyl-5-oxo-1-phényl-1H-pyrazol-4-yl)azo]-N,N,N-triméthyl-benzènaminium également appelé Basic Yellow 57 ou Arianor Straw Yellow 306005 dans le Color Index).

Le ou les colorants directs cationiques peuvent également être choisis parmi :
a) les composés de formule (V) suivante 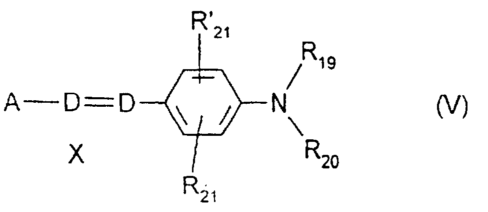 dans laquelle :
   D représente un atome d'azote ou le groupement -CH,
   R₁₉ et R₂₀, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₂₁ et R'₂₁, identiques ou différents, représentent un atome d'hydrogène ou d'halogène choisi parmi le chlore, le brome, l'iode et le fluor, un radical cyano, alcoxy en C₁-C₄ ou acétyloxy,
   X⁻ représente un anion de préférence choisi parmi le chlorure, le méthyl sulfate et l'acétate,
   A représente un groupement choisi par les structures A1 à A19 suivantes : 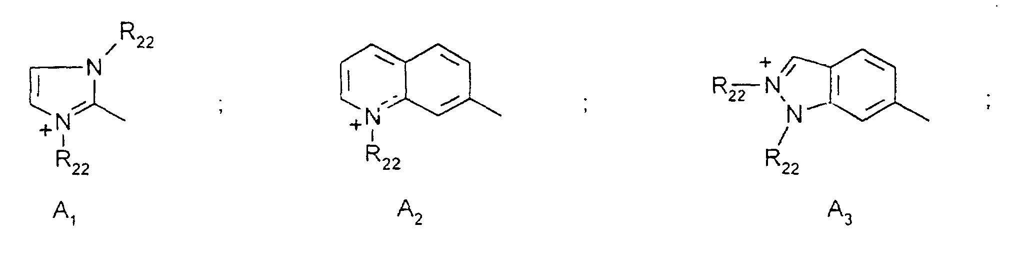 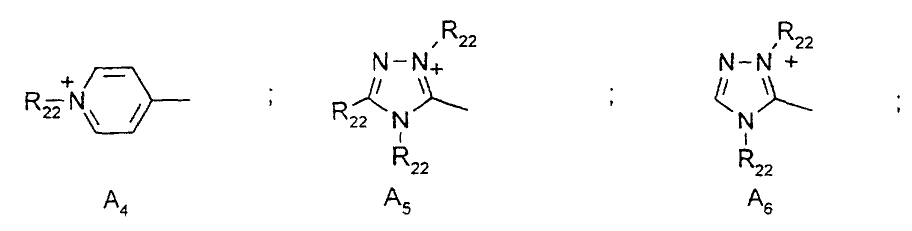 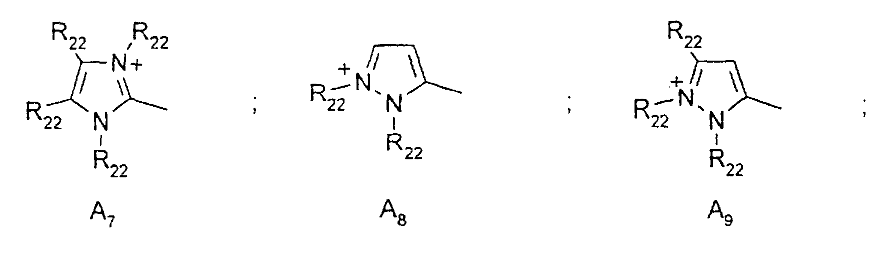 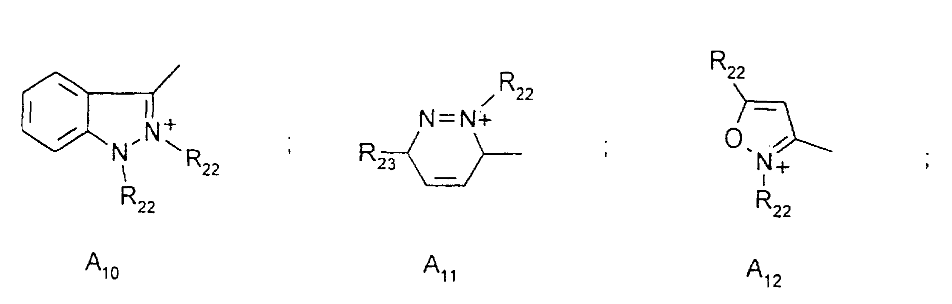 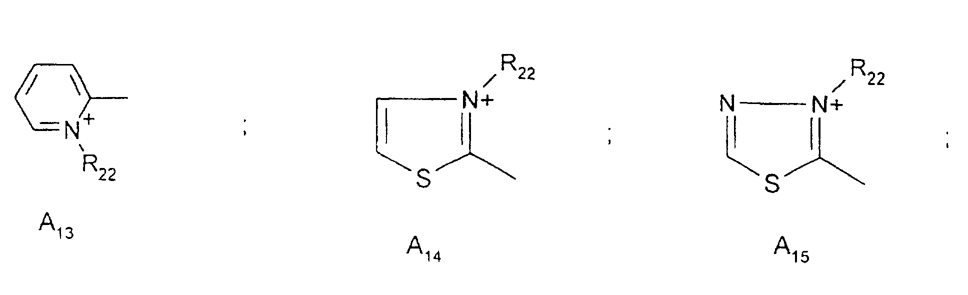 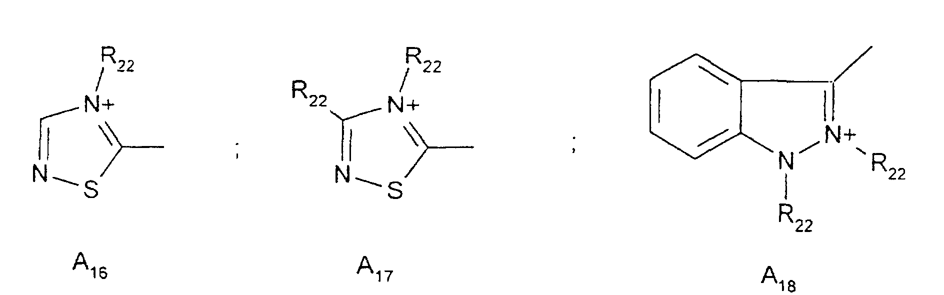 et 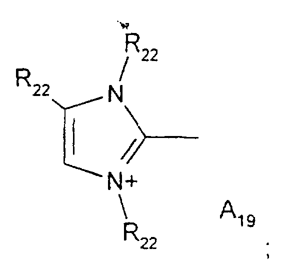
   dans lesquelles R₂₂ représente un radical alkyle en C₁-C₄ pouvant être substitué par un radical hydroxyle et R₂₃ représente un radical alcoxy en C₁-C₄ ;
b) les composés de formule (VI) suivante : 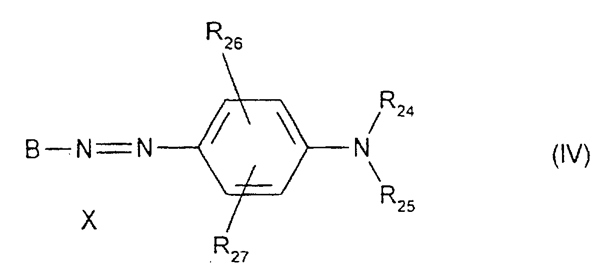 dans laquelle :
   R₂₄ représente un atome d'hydrogène ou un radical alkyle en C₁-C₄,
   R₂₅ représente un atome d'hydrogène, un radical alkyle pouvant être substitué par un radical -CN ou par un groupement amino, un radical 4'-aminophényle ou forme avec R₂₄ un hétérocycle éventuellement oxygéné et/ou azoté pouvant être substitué par un radical alkyle en C₁-C₄,
   R₂₆ et R₂₇, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
   X représente un anion de préférence choisi parmi le chlorure, le méthyl sulfate et l'acétate,
   B représente un groupement choisi par les structures B1 à B6 suivantes : 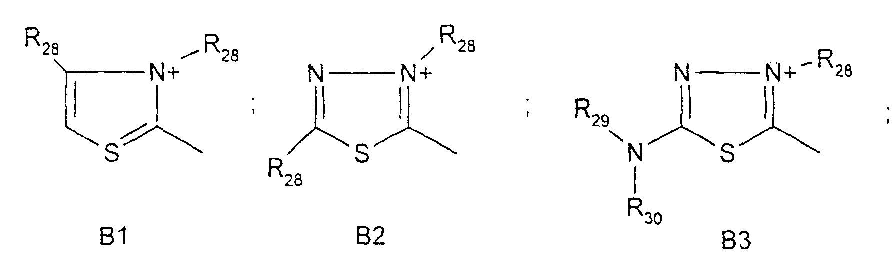 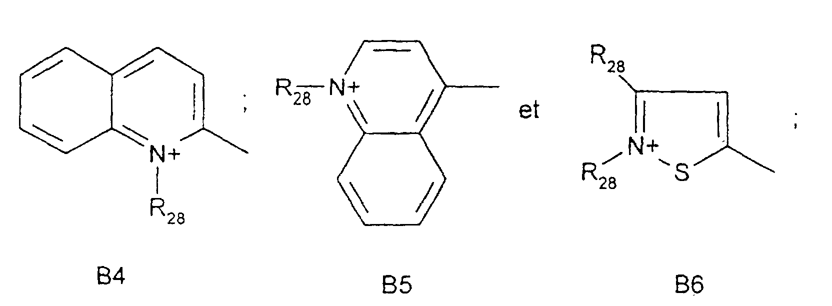
   dans lesquelles R₂₈ représente un radical alkyle en C₁-C₄, R₂₉ et R₃₀, identiques ou différents, représentent un atome d'hydrogène ou un radical alkyle en C₁-C₄ ;
c) les composés de formules (VII) et (VII') suivantes 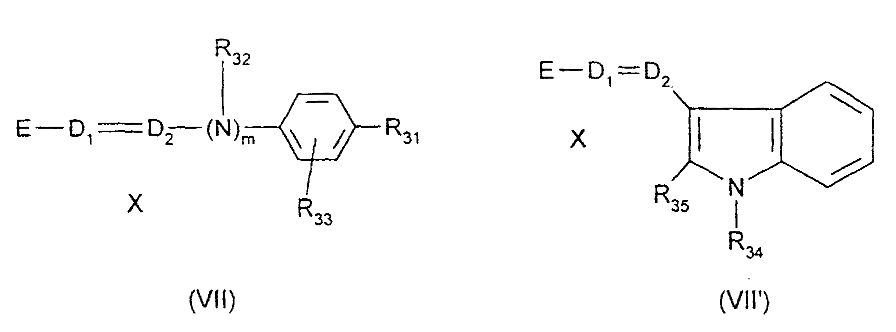 dans lesquelles :
   R₃₁ représente un atome d'hydrogène, un radical alcoxy en C₁-C₄, un atome d'halogène tel que le brome, le chlore, l'iode ou le fluor ou un radical amino,
   R₃₂ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₃₃ représente un atome d'hydrogène ou d'halogène tel que le brome, le chlore, l'iode ou le fluor,
   R₃₄ et R₃₅, identiques ou différents, représentent un atome d'hydrogène ou un radical alkyle en C₁-C₄,
   D₁ et D₂, identiques ou différents, représentent un atome d'azote ou le groupement -CH,
   m = 0 ou 1,
   étant entendu que lorsque R₃₁ représente un groupement amino non substitué, alors D₁ et D₂ représentent simultanément un groupement -CH et m = 0,
   X⁻ représente un anion de préférence choisi parmi le chlorure, le méthyl sulfate et l'acétate,
   E représente un groupement choisi par les structures E1 à E8 suivantes : 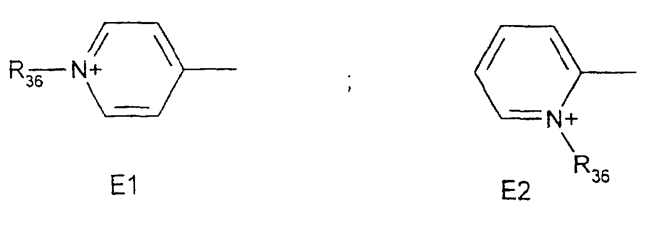 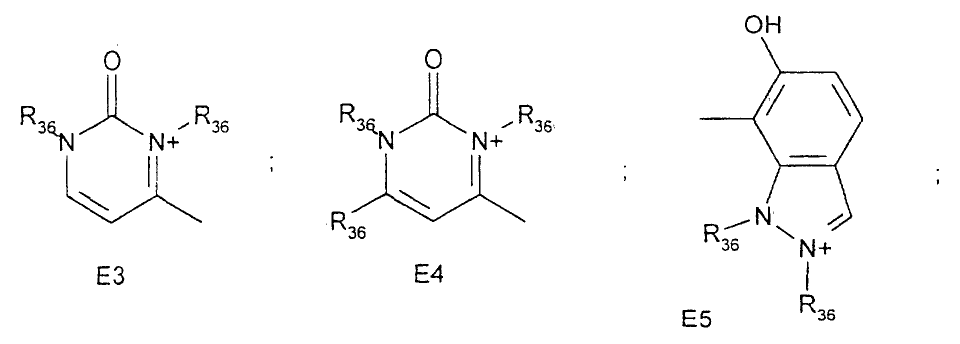 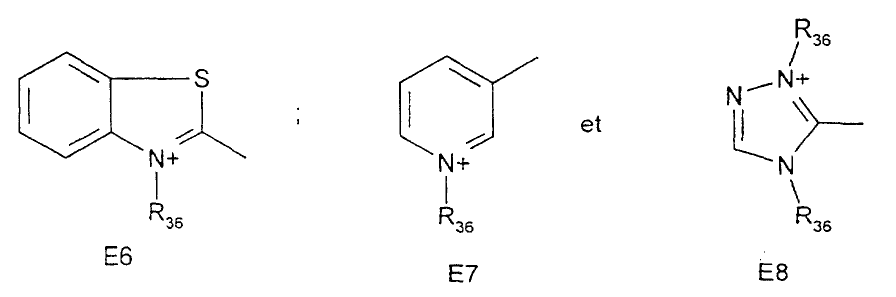
   dans lesquelles R₃₆ représente un radical alkyle en C₁-C₄ ;
   lorsque m = 0 et que D₁ représente un atome d'azote, alors E peut également désigner un groupement de structure E9 suivante : 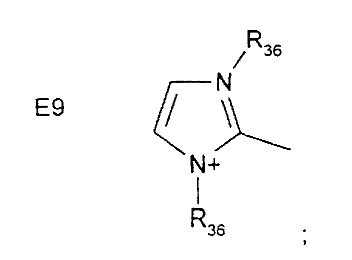 dans laquelle R₃₆ représente un radical alkyle en C₁-C₄.

Les colorants directs cationiques de formules (V), (VI), (VII) et (VII') utilisables dans la composition tinctoriale prête à l'emploi conforme à l'invention, sont des composés connus et sont décrits par exemple dans les demandes de brevets WO 95/01772, WO 95/15144 et EP-A-0 714 954.

Parmi les colorants directs cationiques de formule (V) utilisables dans les compositions tinctoriales prêtes à l'emploi conformes à l'invention, on peut plus particulièrement citer les composés répondant aux structures (V1) à (V52) suivantes : 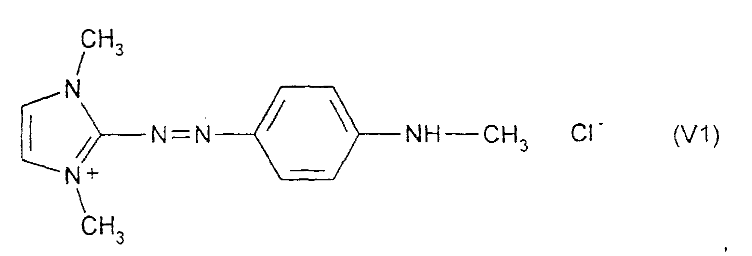 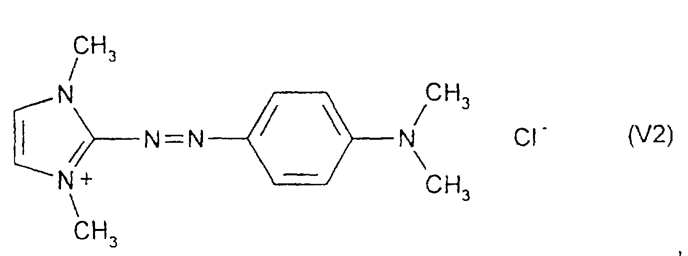 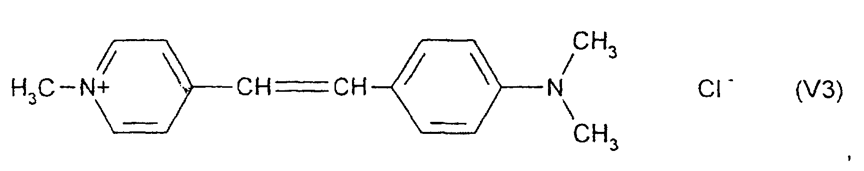 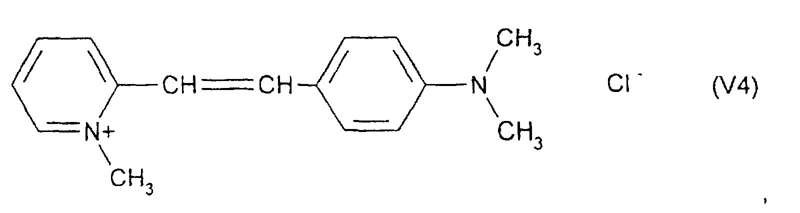 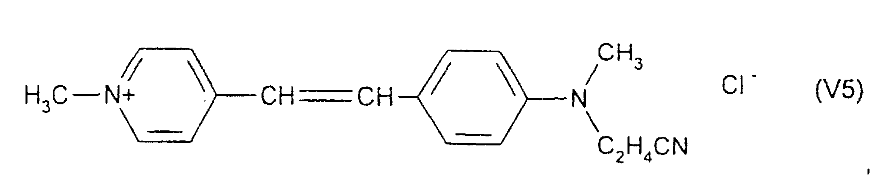 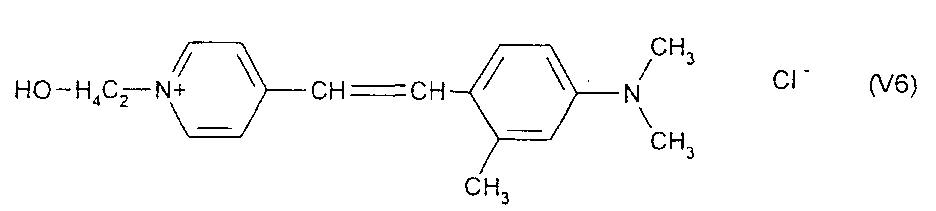 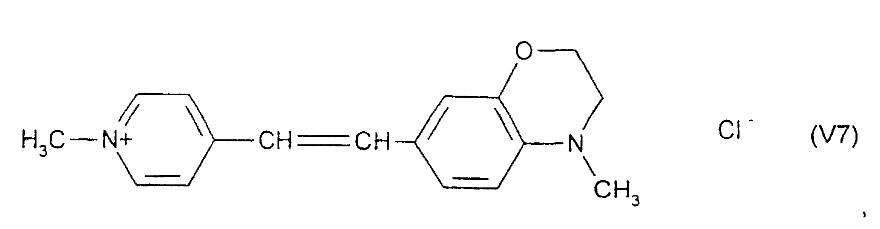 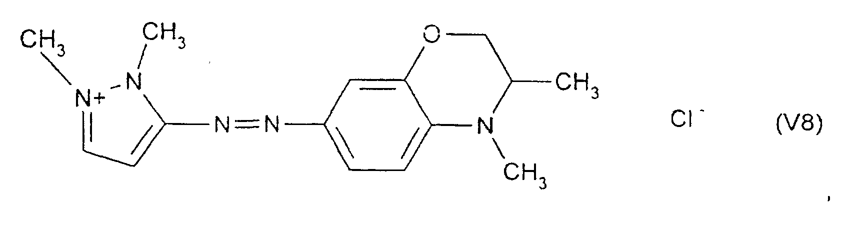 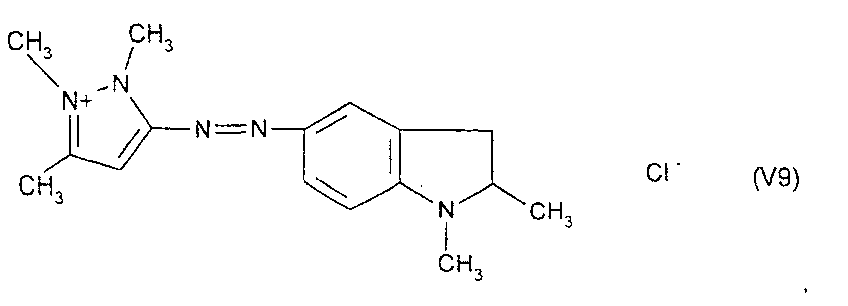 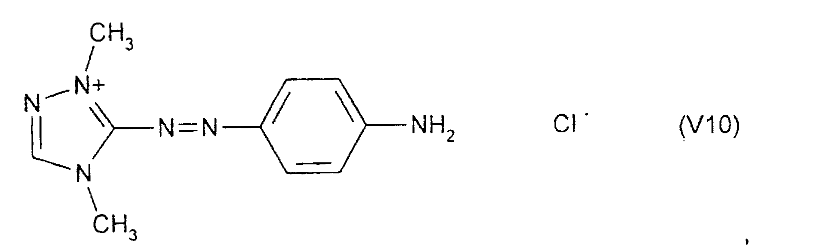 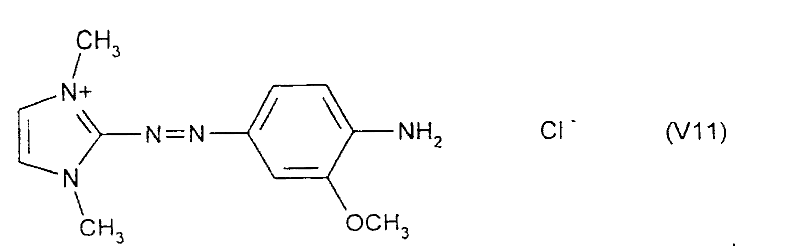 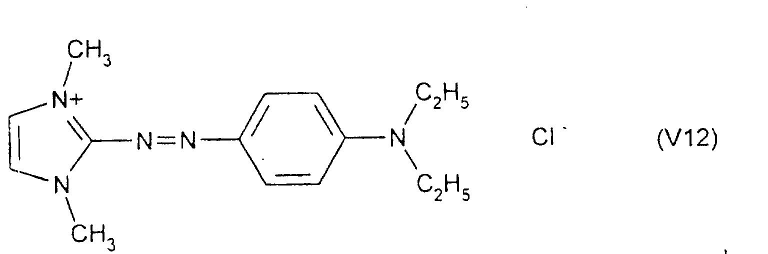 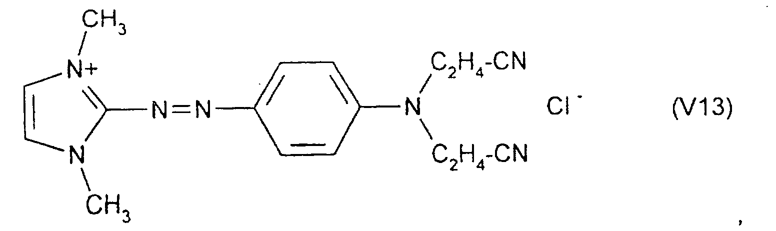 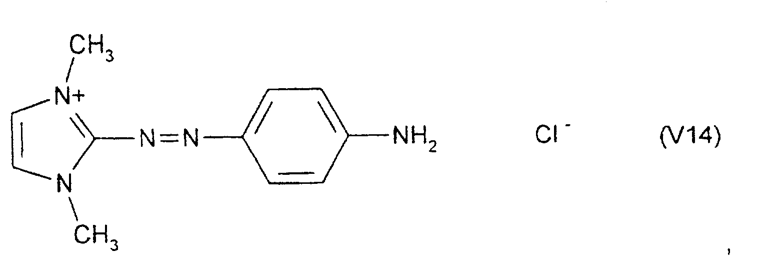 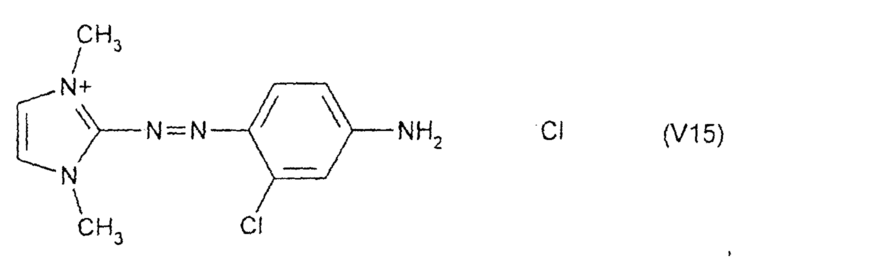 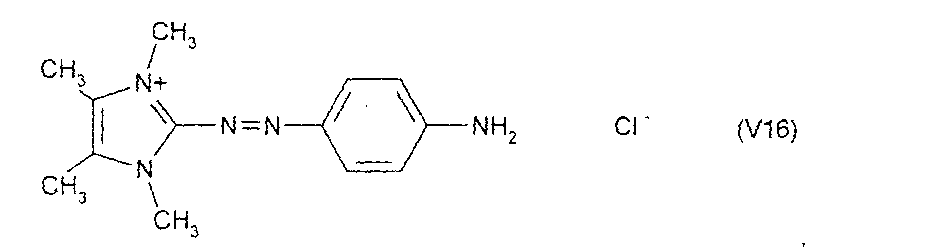 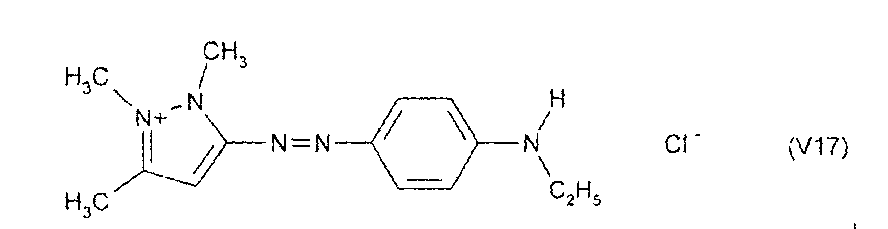 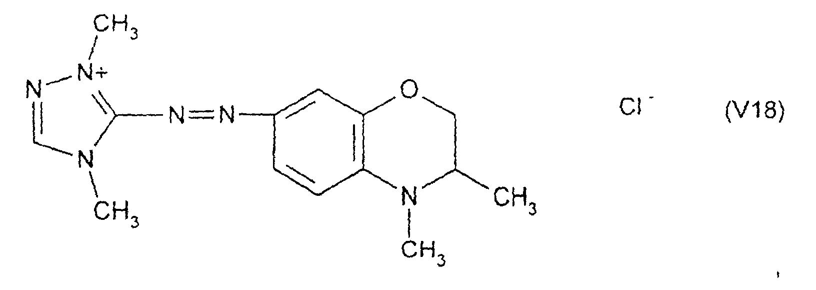 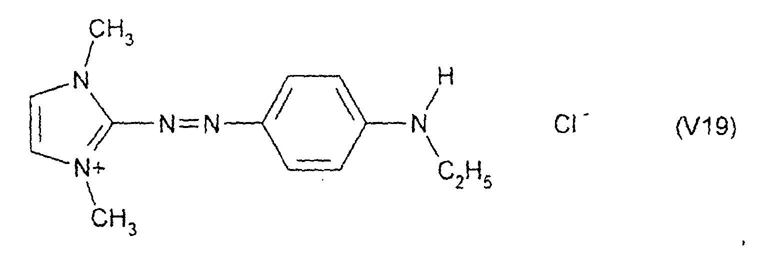 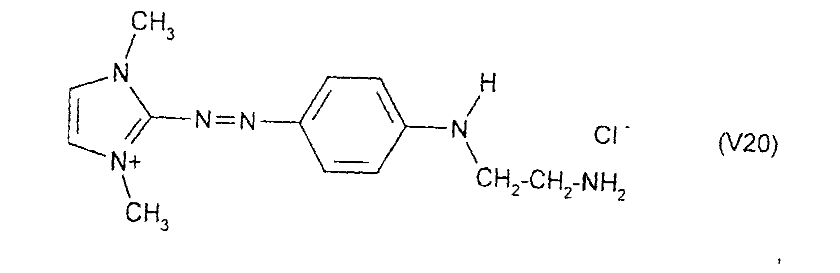 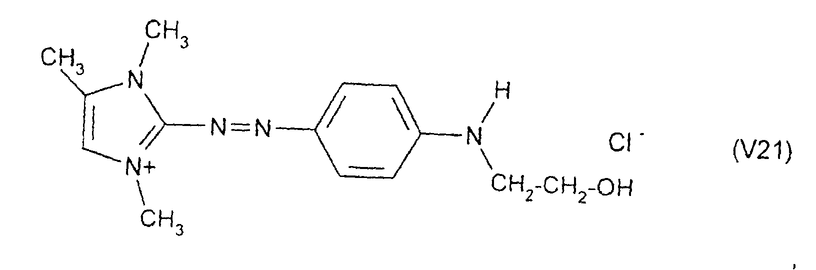 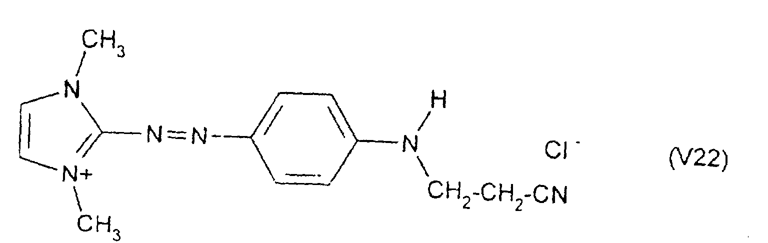 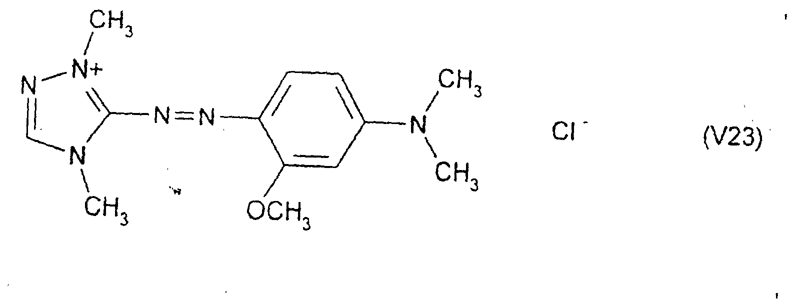 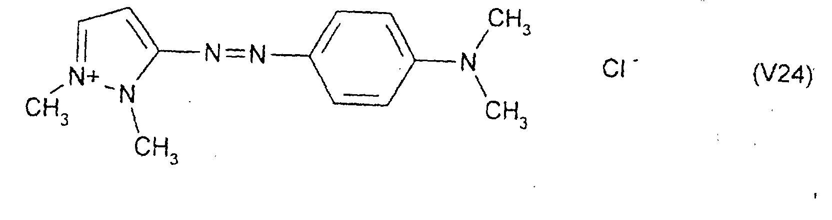 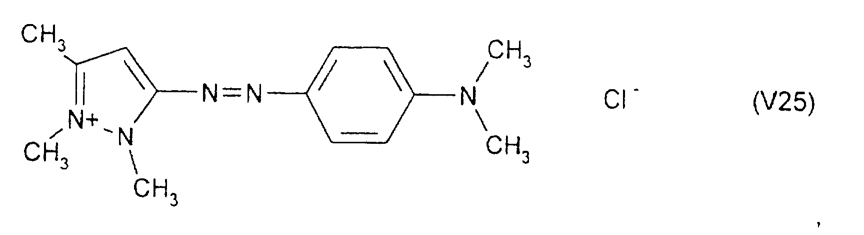 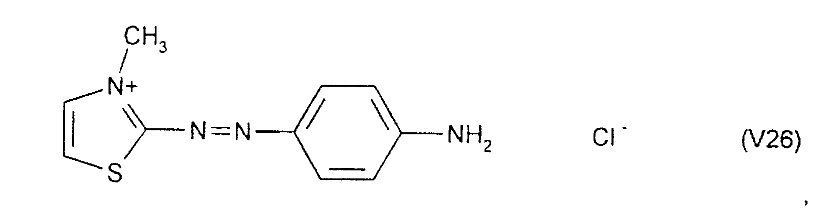 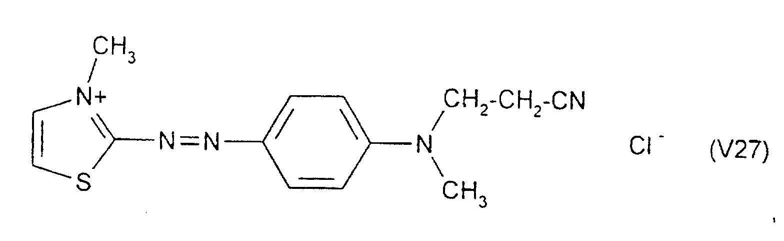 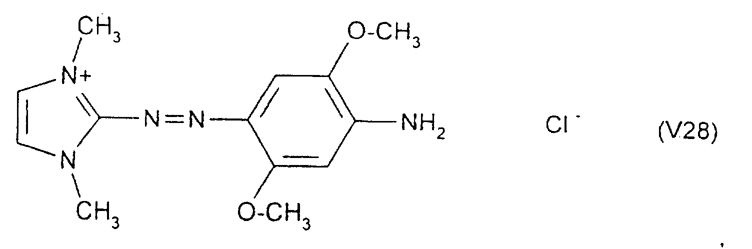 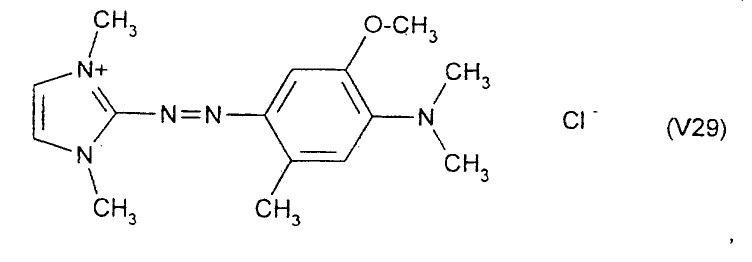 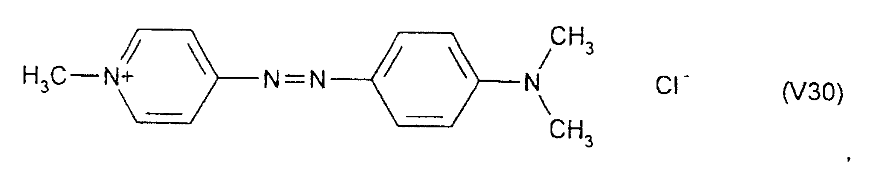 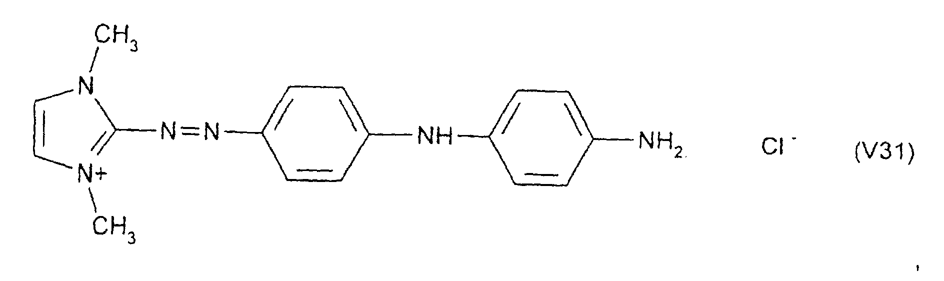 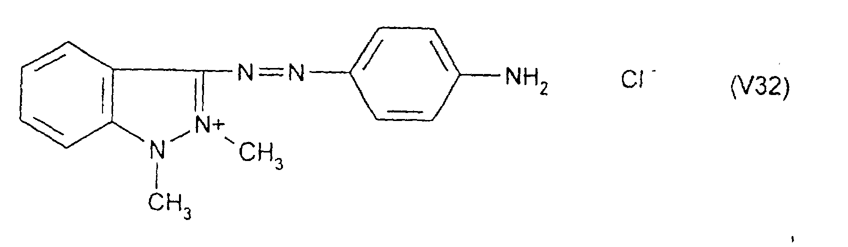 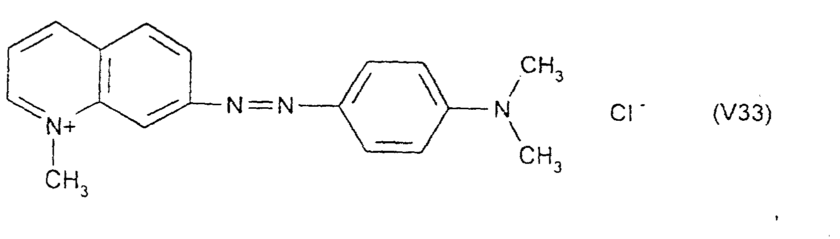 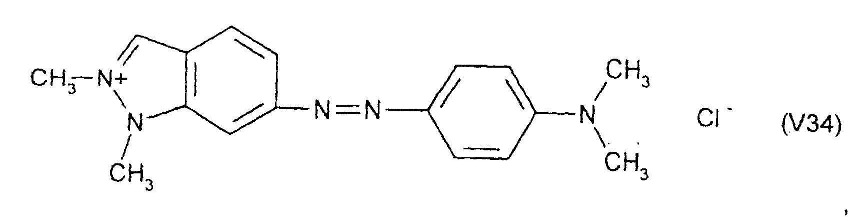 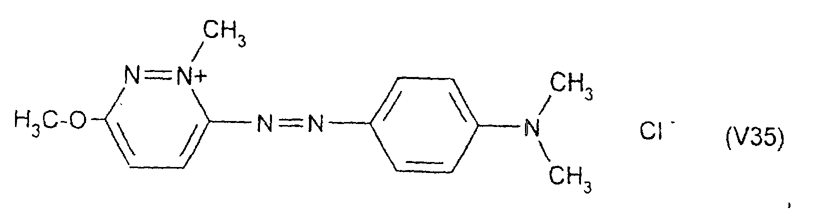 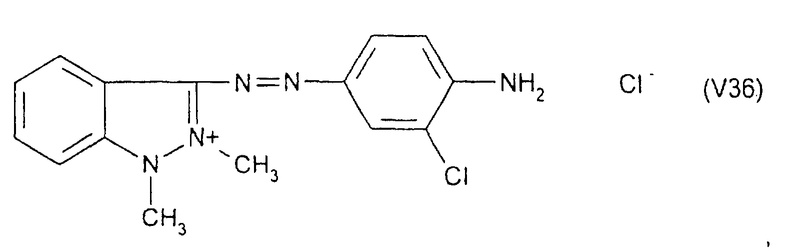 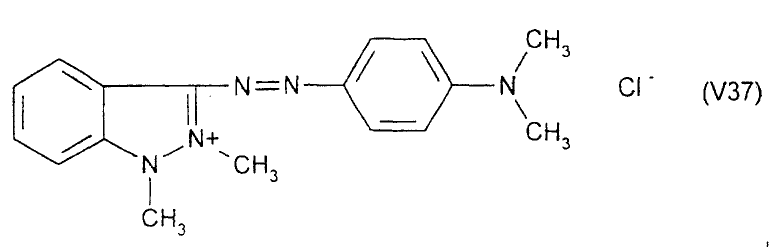 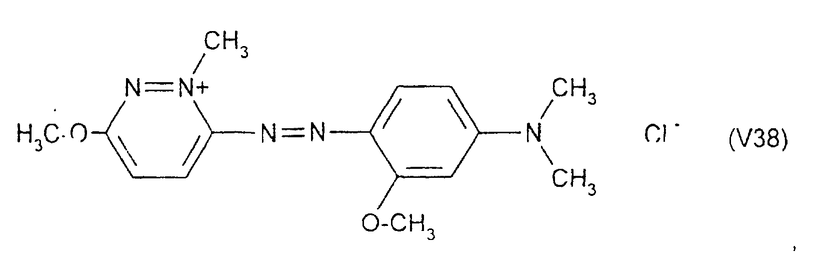 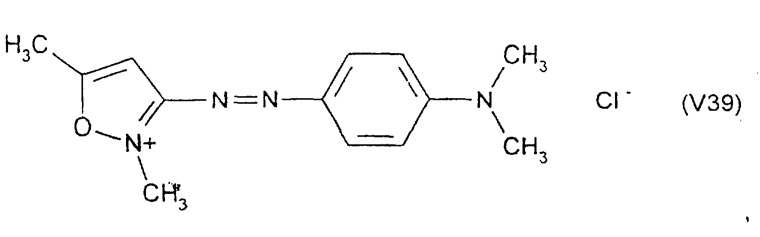 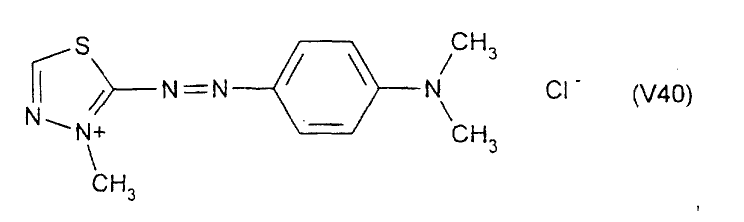 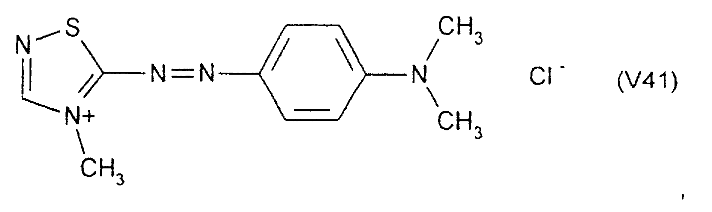 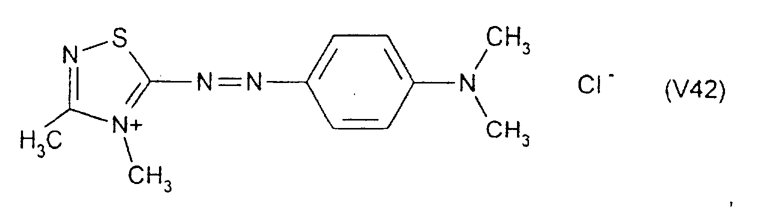 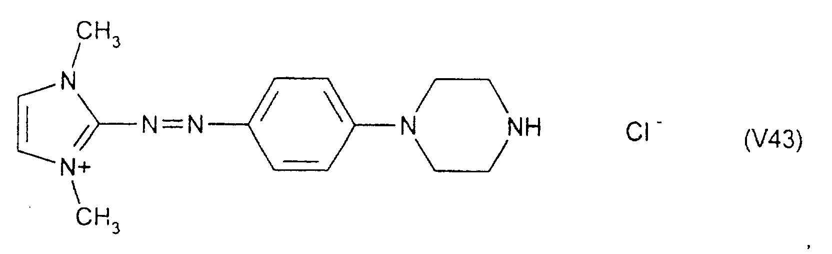 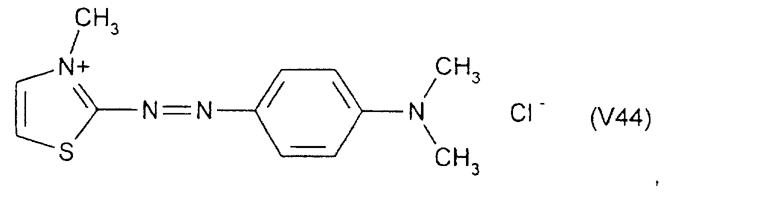 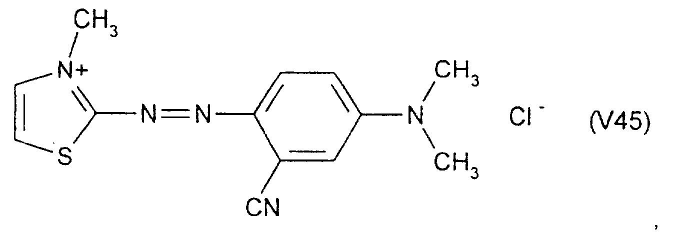 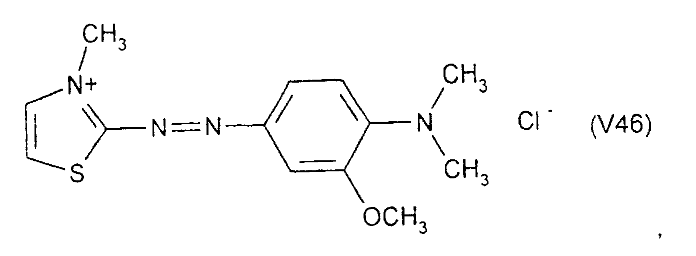 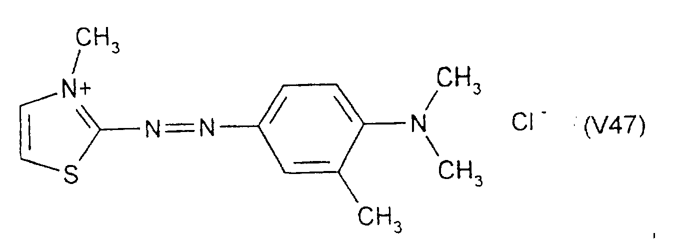 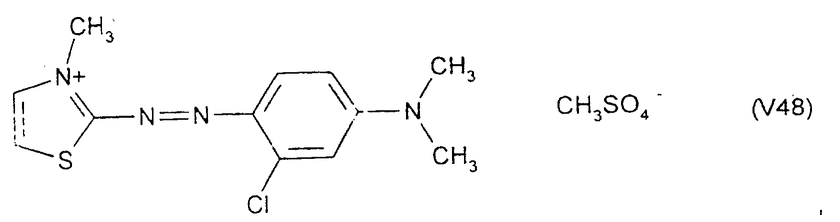 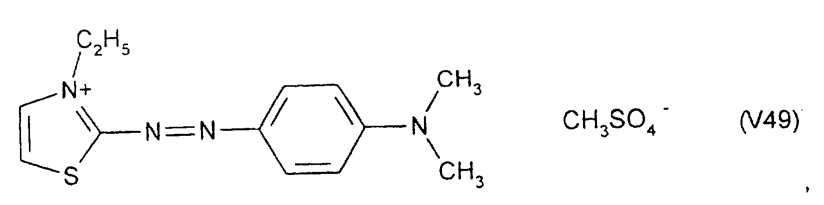 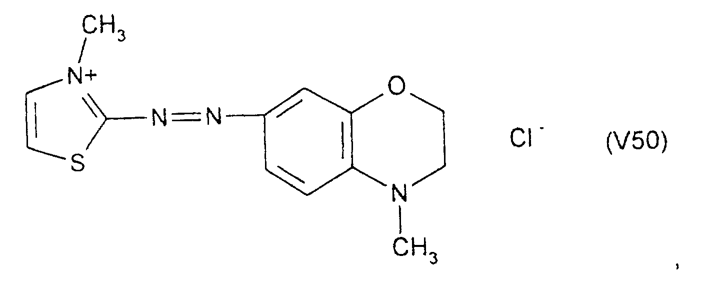 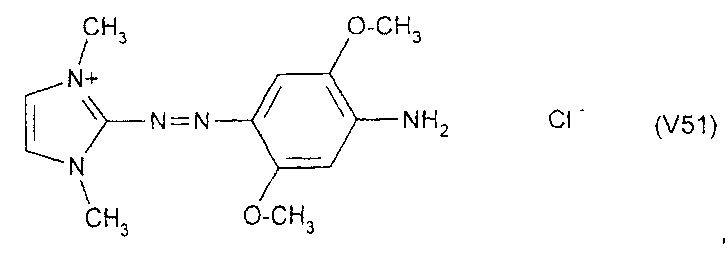 et

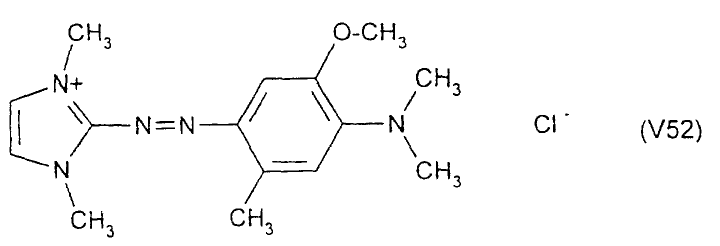

Parmi les composés de structures (V1) à (V52) décrits ci-dessus, on préfère tout particulièrement les composés répondent aux structures (V1), (V2), (V4), (V14) et (V31).

Parmi les colorants directs cationiques de formule (VI) utilisables dans les compositions tinctoriales prêtes à l'emploi conformes à l'invention, on peut plus particulièrement citer les composés répondant aux structures (VI1) à (VI12) suivantes : 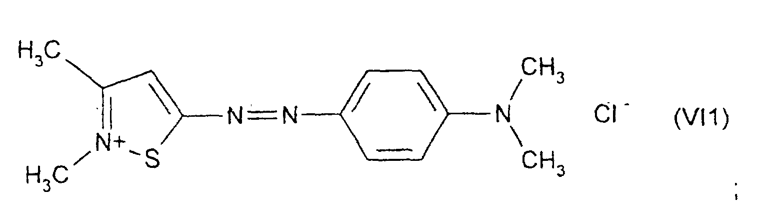 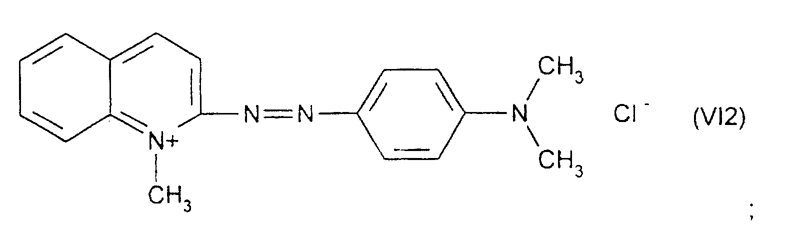 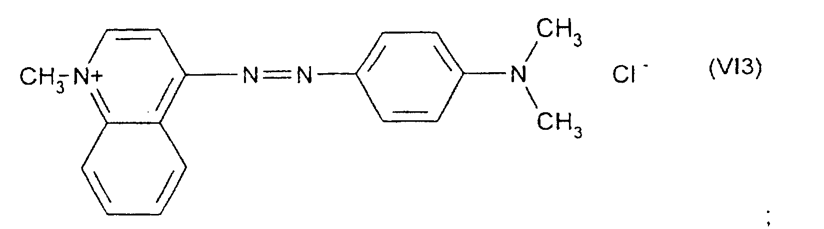 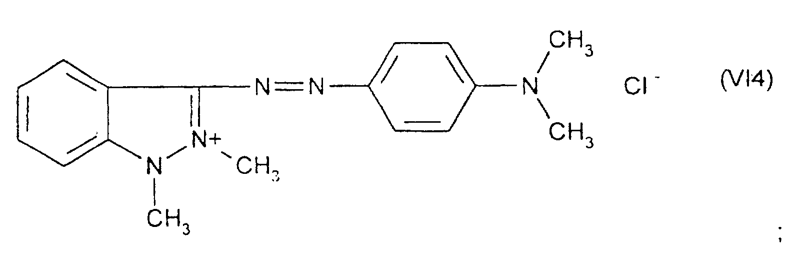 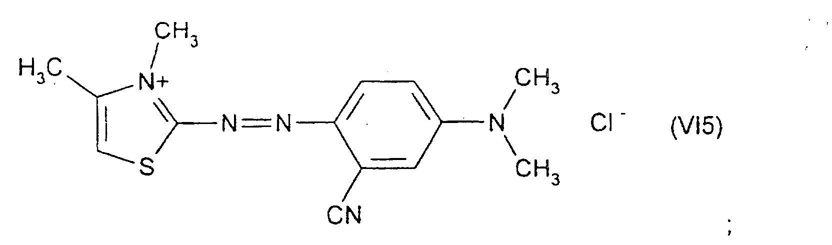 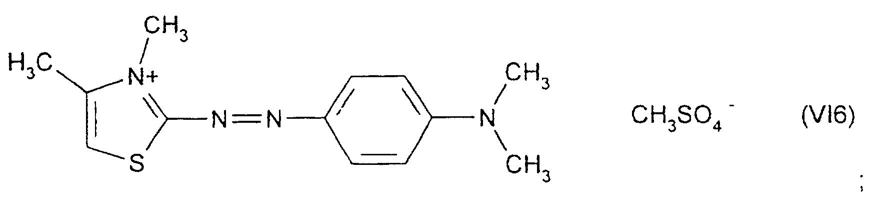 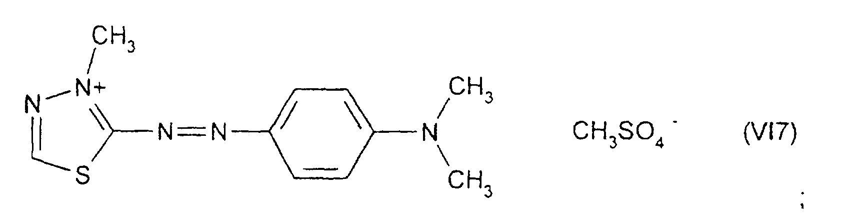 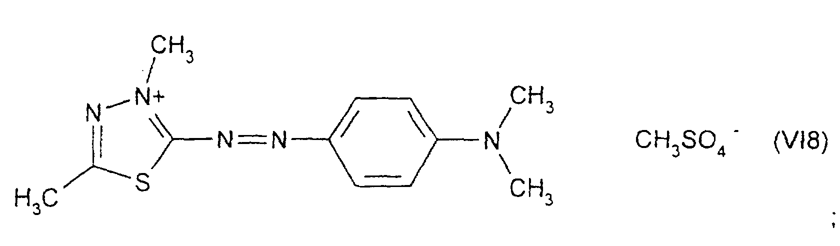 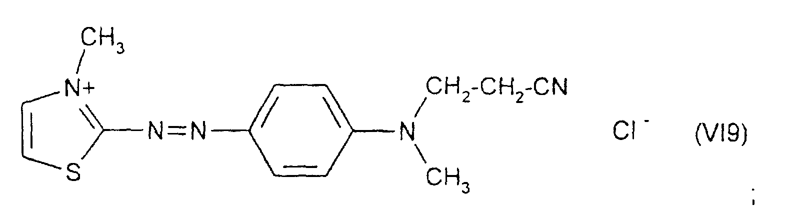 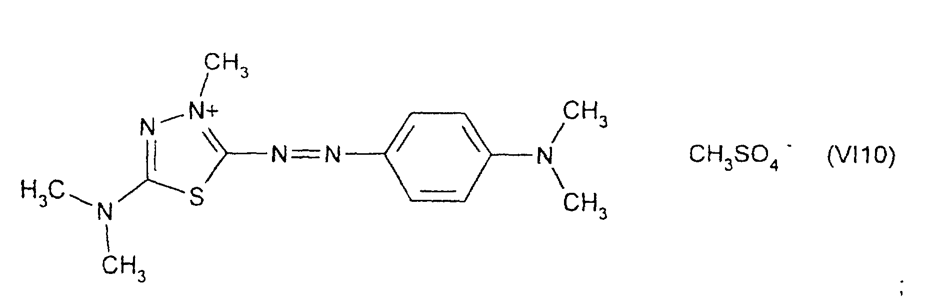 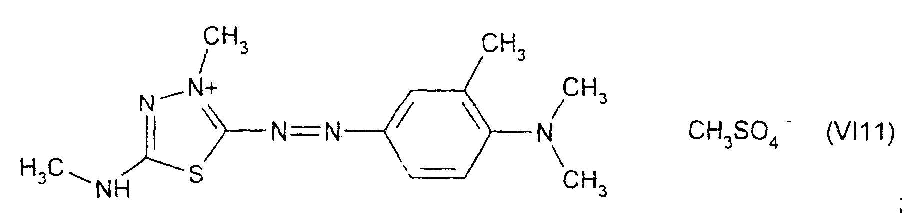 et

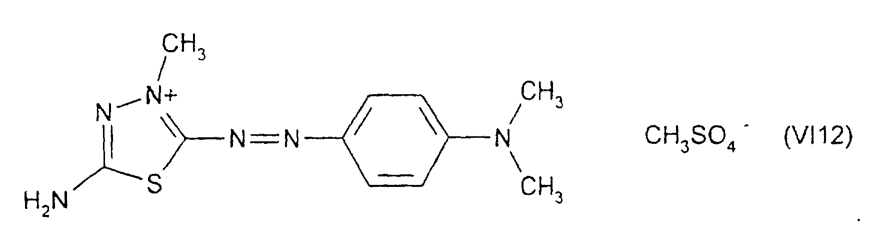

Parmi les colorants directs cationiques de formule (VII), utilisables dans les compositions tinctoriales prêtes à l'emploi conformes à l'invention, on peut plus particulièrement citer les composés répondant aux structures (VII1) à (VII18) suivantes : 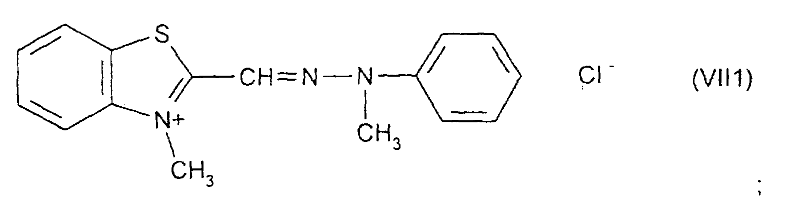 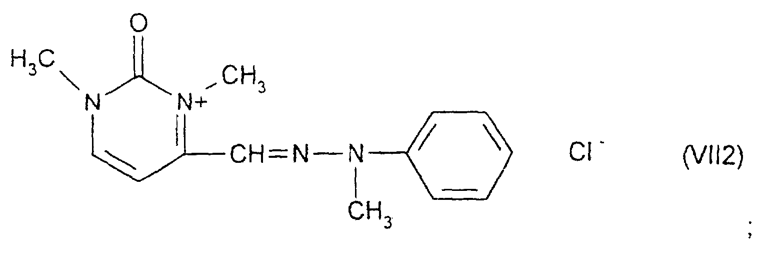 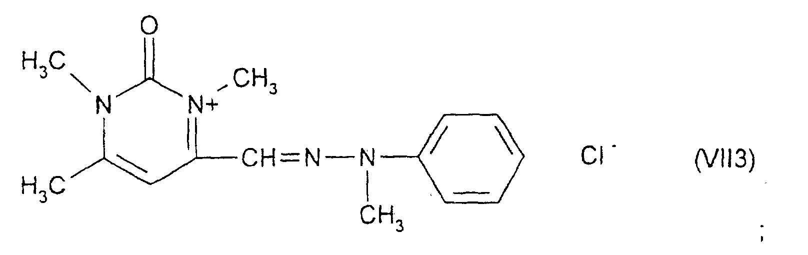 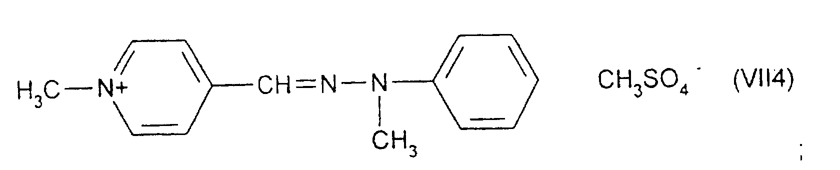 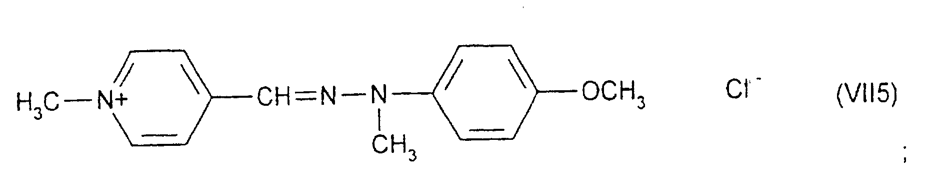 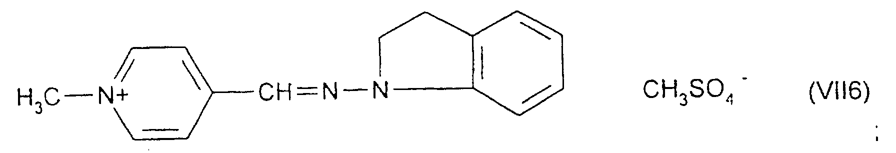 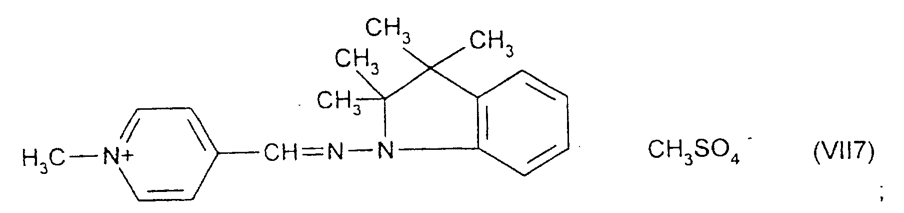 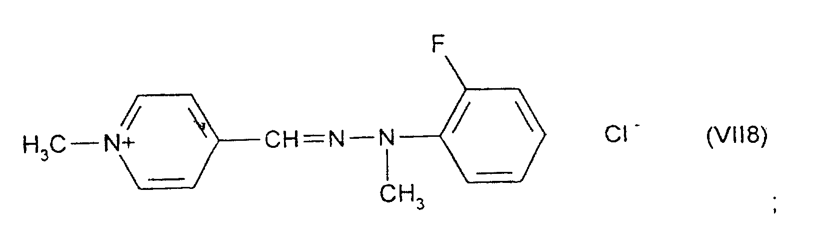 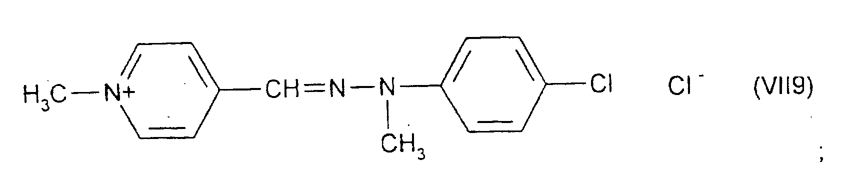 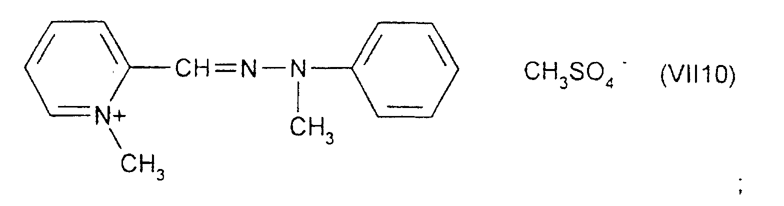 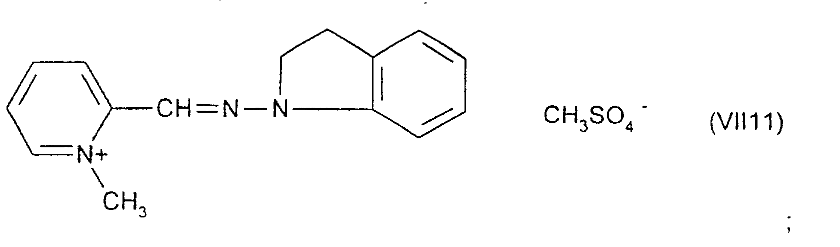 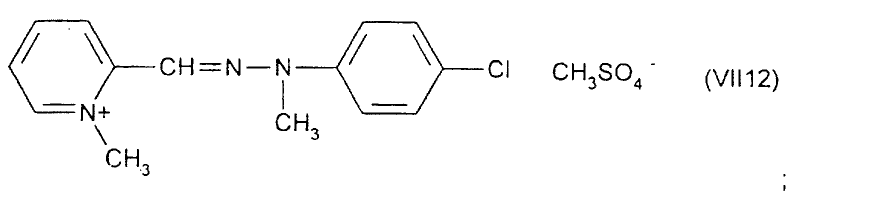 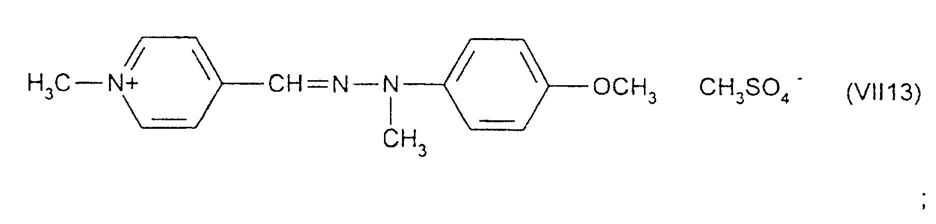 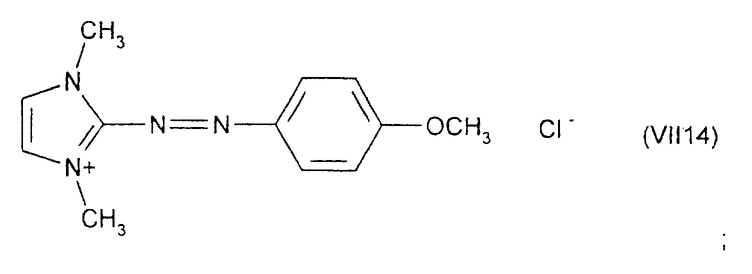 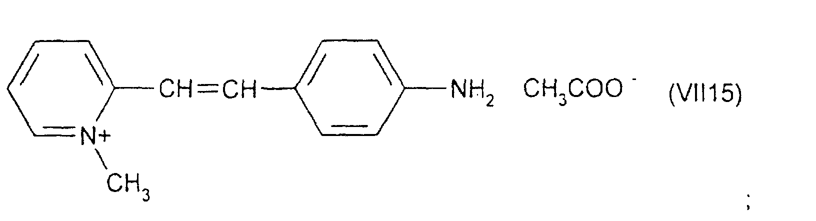 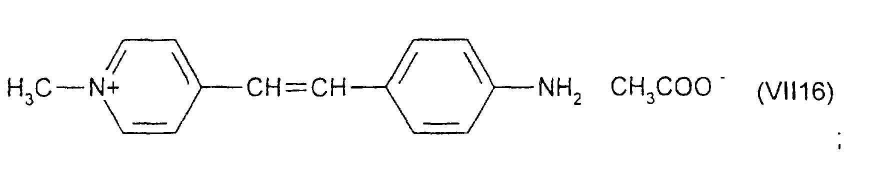 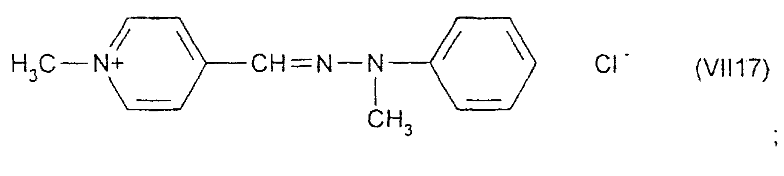 et

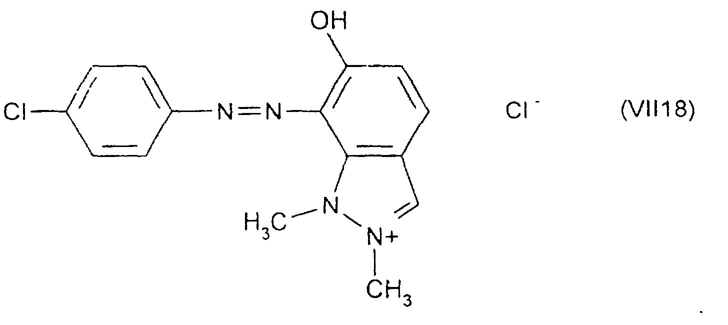

Parmi les composés particuliers de structures (VII1) à (VII18) décrits ci-dessus, on préfère tout particulièrement les composés répondant aux structures (VII4), (VII5) et (VII13).
Parmi les colorants directs cationiques de formule (VII'), utilisables dans les compositions tinctoriales prêtes à l'emploi conformes à l'invention, on peut plus particulièrement citer les composés répondant aux structures (VII'1) à (VII'3) suivantes : 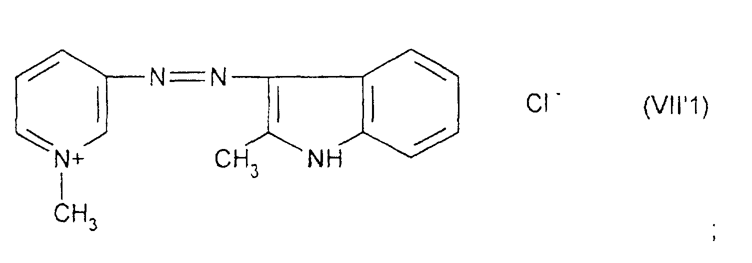 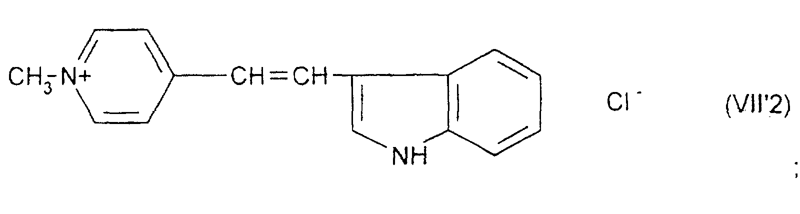 et

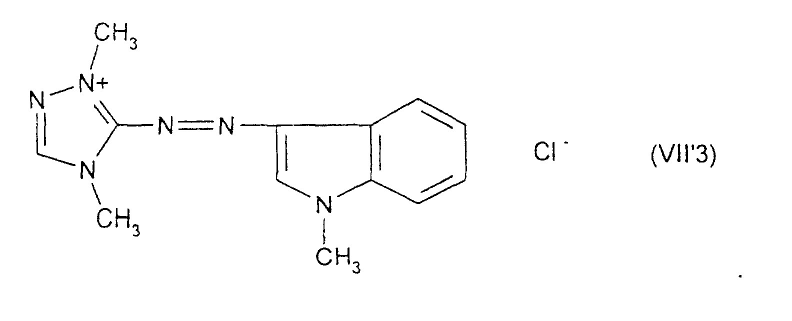

Le ou les colorants directs cationiques utilisés selon l'invention, représentent de préférence de 0,001 à 10 % en poids environ du poids total de la composition tinctoriale prête à l'emploi et encore plus préférentiellement de 0,05 à 5 % en poids environ de ce poids.

La composition tinctoriale conforme à l'invention peut en outre contenir un ou plusieurs coupleurs et/ou des colorants directs non cationiques notamment pour modifier les nuances ou les enrichir en reflets.
Parmi les coupleurs pouvant être présents dans la composition tinctoriale conforme prête à l'emploi conforme à l'invention, on peut notamment citer les méta-phénylènediamines, les méta-aminophénols, les métadiphénols, les coupleurs hétérocycliques, et leurs sels d'addition avec un acide.

Lorsqu'ils sont présents ces coupleurs additionnels représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué' par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition prête à l'emploi conforme à l'invention est choisi de telle manière que l'activité enzymatique de l'oxydo-réductase à 2 électrons soit suffisante. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (VIII) suivante : 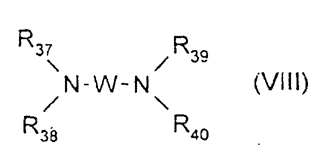 dans laquelle W est un reste propylène éventuellement substitué par un groupement hydroxyle ou un radical alkyle en C₁-C₄ ; R₃₇, R₃₈, R₃₉ et R₄₀, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes des oxydo-réductases à 2 électrons utilisées conformément à l'invention telles que par exemples des peroxydases, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les colorants d'oxydation et la ou les oxydo-réductases à 2 électrons sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins une base d'oxydation, au moins un colorant direct cationique et, d'autre part, une composition (B) renfermant, dans un milieu approprié pour la teinture, au moins une enzyme de type oxydo-réductase à 2 électrons en présence d'au moins un donneur pour ladite enzyme, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement' à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 à 3 DE TEINTURE

On a préparé les compositions tinctoriales prêtes à l'emploi suivantes (teneurs en grammes) :

| **COMPOSITION** | **1** | **2** | **3** |
|---|---|---|---|
| Paraphénylènediamine (base d'oxydation) | 0,7 | - | 0,36 |
| Colorant direct cationique rouge de structure (V1) | 0,6 | - | - |
| Para-aminophénol (Base d'oxydation) | - | 0,187 | - |
| 5-N-(β-hydroxyéthyl)amino 2-méthyl phénol (coupleur) | - | 0,21 | 0,36 |
| Colorant direct cationique orangé de structure (V4) | - | 0,065 | - |
| Colorant direct cationique : Basic Red 76 (Arianor Madder Red) | - | - | 0,12 |
| Uricase d'Arthrobacter globiformis à 20 Unités Internationales (U.I.) / mg, commercialisée par la société Sigma | 1,5 | 1,5 | 1,5 |
| Acide urique | 1,5 | 1,5 | 1,5 |
| Support de teinture commun (*) | (*) | (*) | (*) |
| Eau déminéralisée q.s.p. | 100 g | 100 g | 100 g |

| | | | |
|---|---|---|---|
| (*) : Support de teinture commun : - Ethanol 20,0 g - Hydroxyéthylcellulose vendue sous la dénomination NATROSOL 250 HR ® par la société AQUALON 1,0 g - Alkyl (C₈-C₁₀) polyglucoside en solution aqueuse à 60 % de matière active (M.A.) tamponné par du citrate d'ammonium (0,5%), vendu sous la dénomination ORAMIX CG110 ® par la société SEPPIC 8,0 g - Monoéthanolamine q.s. pH = 9,5 | | | |

Chacune des compositions tinctoriales prêtes à l'emploi décrites ci-dessus a été appliquée sur des mèches de cheveux gris naturels à 90 % de blancs pendant 30 minutes. Les cheveux ont ensuite été rincés, lavés avec un shampooing standard, puis séchés.

Les cheveux ont été teints dans les nuances figurant dans le tableau ci-après :

| **EXEMPLE** | **Nuance obtenue** |
|---|---|
| **1** | Rouge intense |
| **2** | Blond clair cuivré rouge |
| **3** | Blond violine rouge |

## Revendications

1. Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :
- au moins une base d'oxydation,
- au moins colorant direct cationique,
- au moins une enzyme de type oxydo-réductase à 2 électrons,
- et au moins un donneur pour ladite enzyme.

2. Composition selon la revendication 1, **caractérisée par le fait que** l'oxydoréductases à 2 électrons est choisie parmi les pyranose oxydases, les glucose oxydases, les glycérol oxydases, les lactates oxydases, les pyruvate oxydases, et les uricases.

3. Composition selon l'une quelconque des revendications 1 ou 2, **caractérisée par le fait que** l'oxydo-réductases à 2 électrons est choisie parmi les uricases d'origine animale, microbiologique ou biotechnologique.

4. Composition selon l'une quelconque des revendications précédentes, **caractérisée par le fait que** la ou les oxydo-réductases à 2 électrons représentent de 0,01 à 20 % en poids du poids total de la composition tinctoriale prête à l'emploi.

5. Composition selon la revendication 4, **caractérisée par le fait que** la ou les oxydo-réductases à 2 électrons représentent de 0,1 à 5 % en poids du poids total de la composition tinctoriale prête à l'emploi.

6. Composition selon la revendication 3, **caractérisée par le fait que** le donneur (ou substrat) pour ladite oxydo-réductase à 2 électrons est choisi parmi l'acide urique et ses sels.

7. Composition selon l'une quelconque des revendications précédentes, **caractérisée par le fait que** le ou les donneurs représentent de 0,01 à 20 % en poids du poids total de la composition tinctoriale prête à l'emploi.

8. Composition selon la revendication 7, **caractérisée par le fait que** le ou les donneurs représentent de 0,1 à 5 % en poids du poids total de la composition tinctoriale prête à l'emploi.

9. Composition selon l'une quelconque des revendications précédentes, **caractérisée par le fait que** la ou les bases d'oxydation sont choisies parmi les paraphénylènediamines, les bases doubles, les para-aminophénols, les ortho aminophénols et les bases d'oxydation hétérocycliques.

10. Composition selon la revendication 9, **caractérisée par le fait que** les paraphénylènediamines sont choisies parmi les composés de formule (I) suivante et leurs sels d'addition avec un acide : 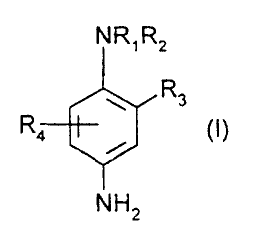 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

11. Composition selon la revendication 10, **caractérisée par le fait que** les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénlènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxy-éthyl) amino 2-chloro aniline, la 2-β-hydroxyéthyl paraphénylènediamine, la 2-fluoro paraphénylènediamine, la 2-isopropyl paraphénylènediamine, la N-(β-hydroxy-propyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl)paraphénylènediamine, et leurs sels d'addition avec un acide.

12. Composition selon la revendication 9, **caractérisée par le fait que** les bases doubles sont choisies parmi les composés de formule (II), et leurs sels d'addition avec un acide : 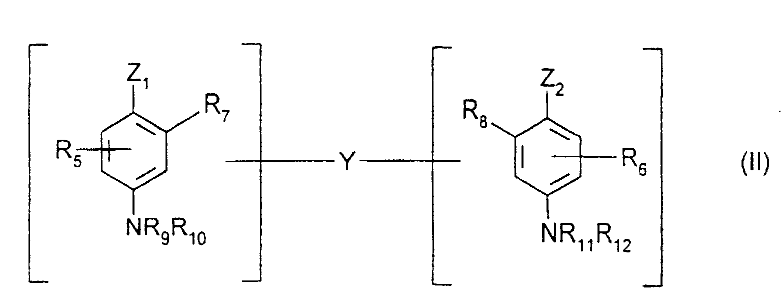 dans laquelle :
- Z₁ et Z₂, identiques ou différents, représentent un radical hydroxyle ou -NH₂ pouvant être substitué par un radical alkyle en C₁-C₄ ou par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

13. Composition selon la revendication 12, **caractérisée par le fait que** les bases doubles de formule (II) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

14. Composition selon la revendication 9, **caractérisée par le fait que** les para-aminophénols sont choisis parmi les composés de formule (III), et leurs sels d'addition avec un acide : 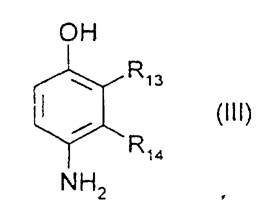 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

15. Composition selon la revendication 14, **caractérisée par le fait que** les para-aminophénols de formule (III) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

16. Composition selon la revendication 9, **caractérisée par le fait que** les orthoaminophénols sont choisis parmi 2-amino phénol, le 2-amino 5-méthyl phénol, le 2-amino 6-méthyl phénol, le 5-acétamido 2-amino phénol, et leurs sels d'addition avec un acide.

17. Composition selon la revendication 9, **caractérisée par le fait que** les bases d'oxydation hétérocycliques sont choisies parmi les dérivés pyridiniques, les dérivés pyrimidiniques, les dérivés pyrazoliques, les dérivés pyrazolo-pyrimidiniques, et leurs sels d'addition avec un acide.

18. Composition selon l'une quelconque des revendications précédentes, **caractérisée par le fait que** la ou les bases d'oxydation représentent de 0,0005 à 12 % en poids du poids total de la composition tinctoriale.

19. Composition selon la revendication 18, **caractérisée par le fait que** la ou les bases d'oxydation représentent de 0,005 à 6 % en poids du poids total de la composition tinctoriale.

20. Composition selon l'une quelconque des revendications précédentes, **caractérisée par le fait que** le ou les colorants directs cationiques sont choisis parmi les amino-anthraquinones cationiques, les mono- ou di-azoïques cationiques, les naphtoquinones cationiques.

21. Composition selon la revendication 20, **caractérisée par le fait que** le ou les colorants directs cationiques sont choisis parmi le chlorure de [8-[(p-aminophényl)azol]-7-hydroxy-2-naphtyl]triméthylammonium, le chlorure de 3-[(4-amino-6-bromo-5,8-dihydro-1-hydroxy-8-imino-5-oxo-2-naphtalényl)-amino]-N,N,N-triméthyl-benzénaminium, le chlorure de 7-hydroxy-8-[(2-méthoxy-phényl)azo]-N,N,N-triméthyl-2-naphtalènaminium, le chlorure de [8-[(4-amino-2-nitrophényl)azo]-7-hydroxy-2-naphtyl]triméthylammonium et le chlorure de 3-[(4,5-dihydro-3-méthyl-5-oxo-1-phényl-1H-pyrazol-4-yl)azo]-N,N,N-triméthyl-benzènaminium.

22. Composition selon l'une quelconque des revendications 1 à 19, **caractérisée par le fait que** le ou les colorants directs cationiques sont choisis parmi :
a) les composés de formule (V) suivante : 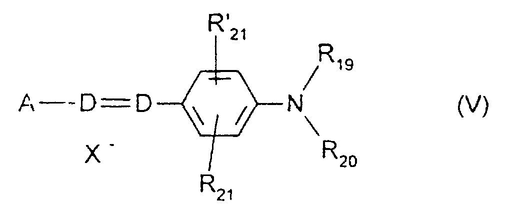 dans laquelle :
D représente un atome d'azote ou le groupement -CH,
R₁₉ et R₂₀, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₂₁ et R'₂₁, identiques ou différents, représentent un atome d'hydrogène ou d'halogène choisi parmi le chlore, le brome, l'iode et le fluor, un radical cyano, alcoxy en C₁-C₄ ou acétyloxy,
X⁻ représente un anion de préférence choisi parmi le chlorure, le méthyl sulfate et l'acétate,
A représente un groupement choisi par les structures A1 à A19 suivantes : 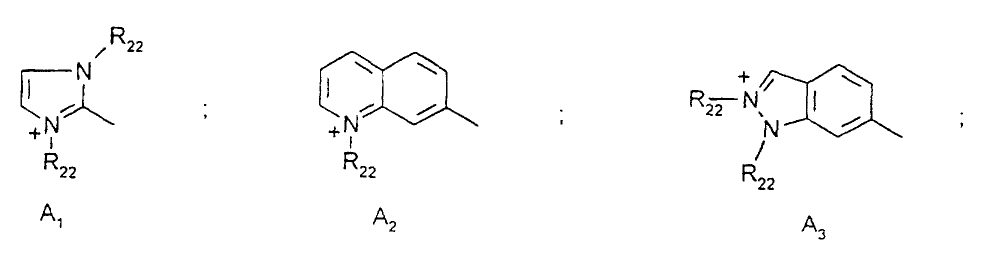 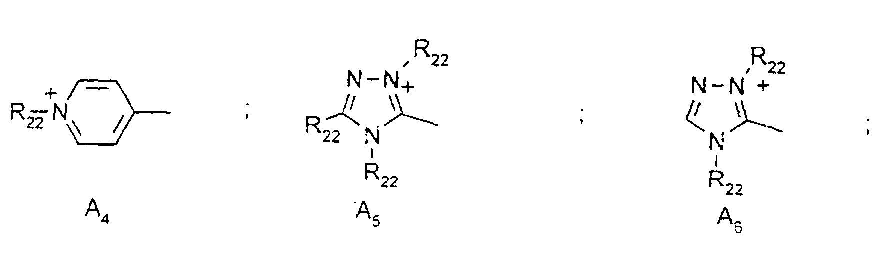 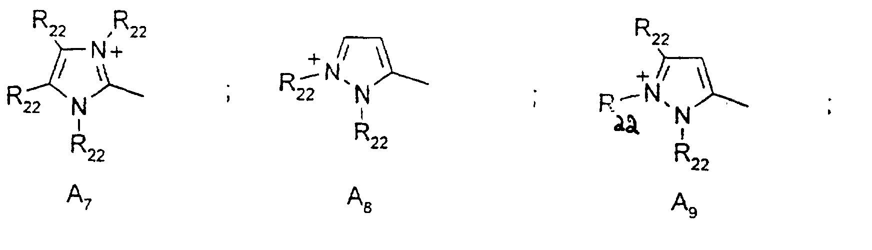 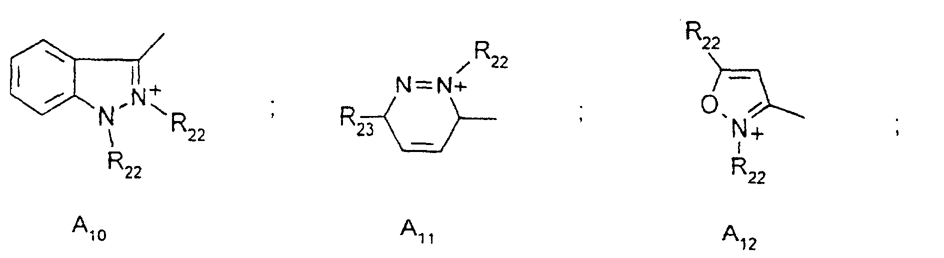 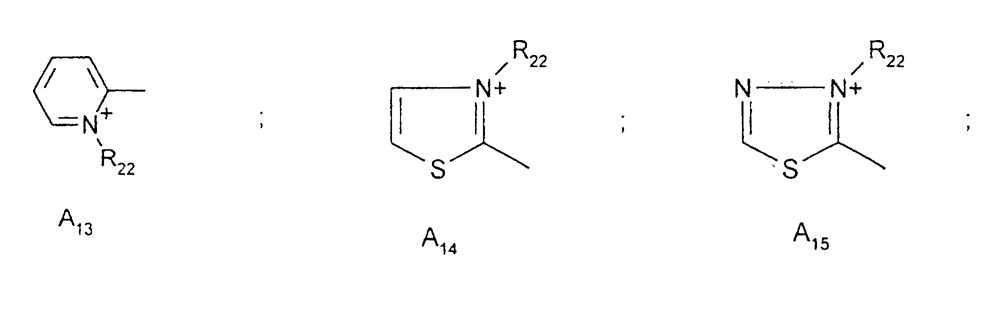 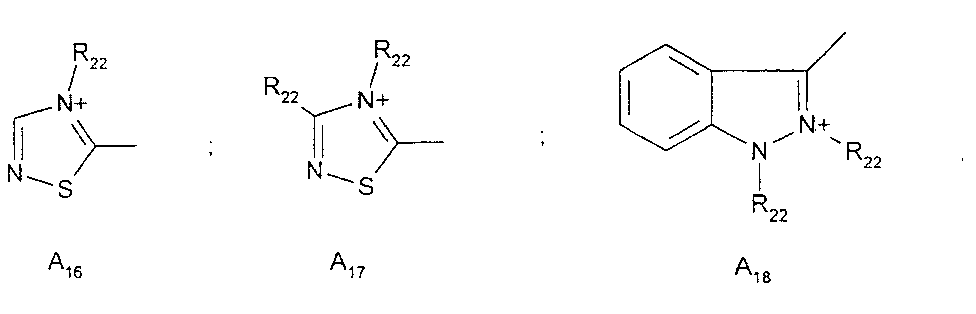 et 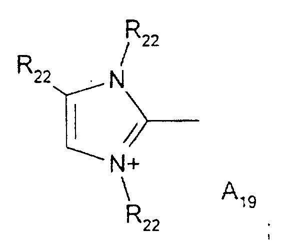
dans lesquelles R₂₂ représente un radical alkyle en C₁-C₄ pouvant être substitué par un radical hydroxyle et R₂₃ représente un radical alcoxy en C₁-C₄ ;
b) les composés de formule (VI) suivante : 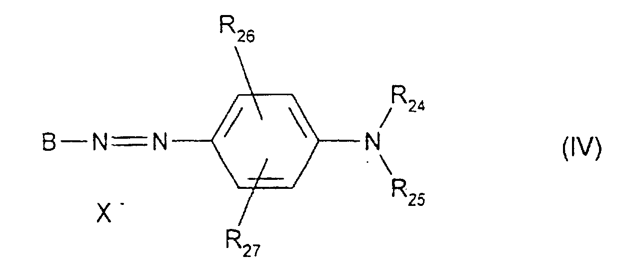 dans laquelle :
R₂₄ représente un atome d'hydrogène ou un radical alkyle en C₁-C₄,
R₂₅ représente un atome d'hydrogène, un radical alkyle pouvant être substitué par un radical -CN ou par un groupement amino, un radical 4'-aminophényle ou forme avec R₂₄ un hétérocycle éventuellement oxygéné et/ou azoté pouvant être substitué par un radical alkyle en C₁-C₄,
R₂₆ et R₂₇, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à B6 suivantes : 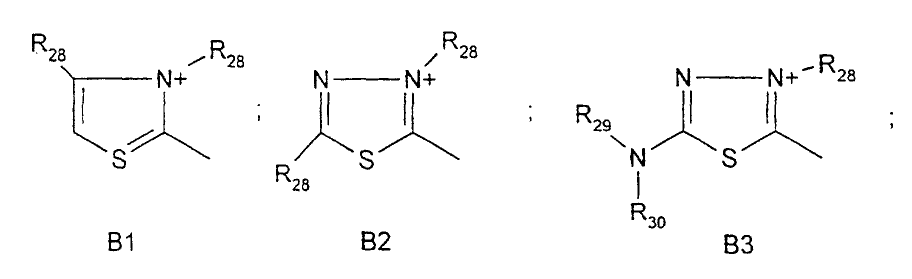 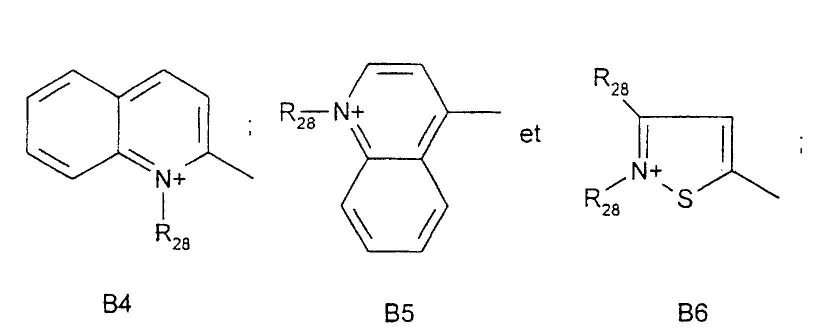
dans lesquelles R₂₈ représente un radical alkyle en C₁-C₄, R₂₉ et R₃₀, identiques ou différents, représentent un atome d'hydrogène ou un radical alkyle en C₁-C₄ ;
c) les composés de formules (VII) et (VII') suivantes : 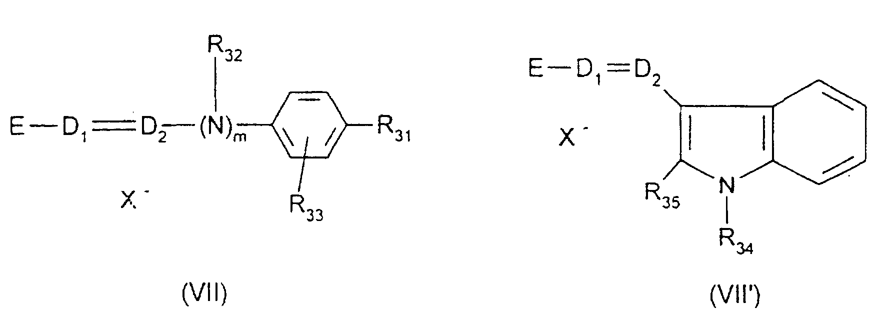 dans lesquelles :
R₃₁ représente un atome d'hydrogène, un radical alcoxy en C₁-C₄, un atome d'halogène tel que le brome, le chlore, l'iode ou le fluor ou un radical amino,
R₃₂ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₃₃ représente un atome d'hydrogène ou d'halogène tel que le brome, le chlore, l'iode ou le fluor,
R₃₄ et R₃₅, identiques ou différents, représentent un atome d'hydrogène ou un radical alkyle en C₁-C₄,
D₁ et D₂, identiques ou différents, représentent un atome d'azote ou le groupement -CH,
m = 0 ou 1,
étant entendu que lorsque R₃₁ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes : 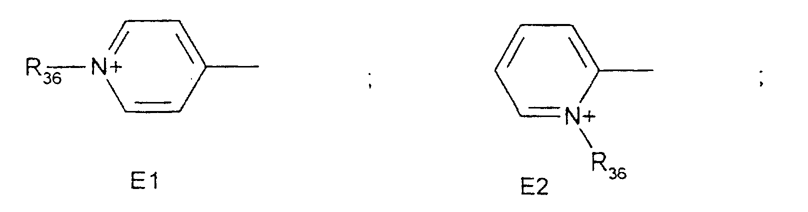 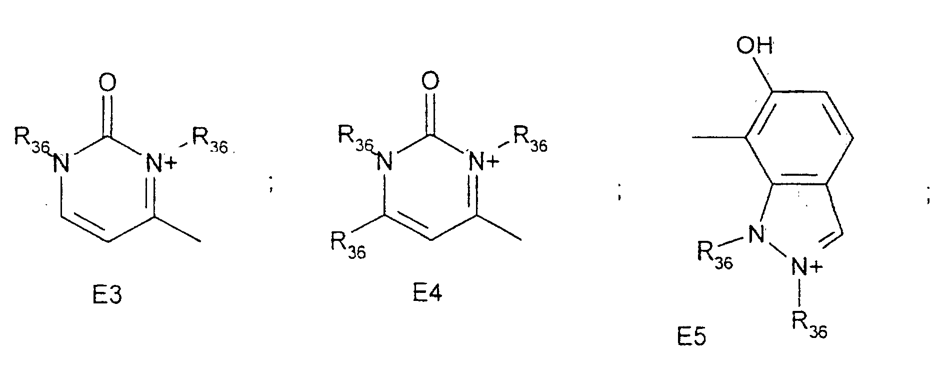 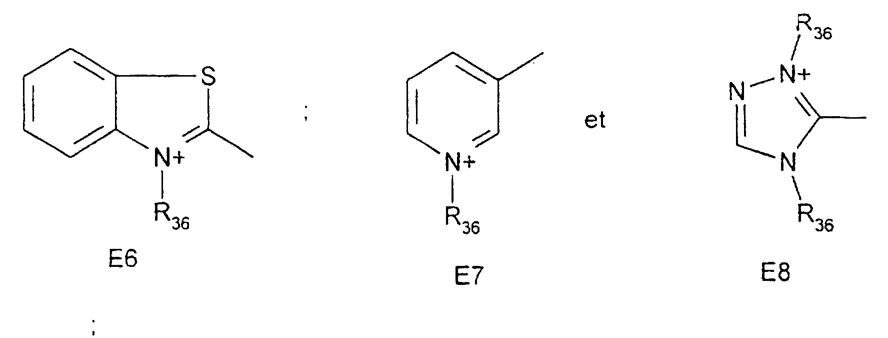
dans lesquelles R₃₆ représente un radical alkyle en C₁-C₄ ;
lorsque m = 0 et que D₁ représente un atome d'azote, alors E peut également désigner un groupement de structure E9 suivante : 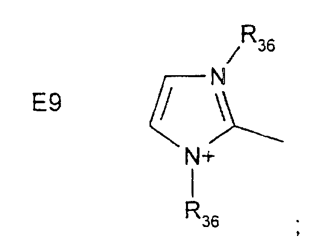 dans laquelle R₃₆ représente un radical alkyle en C₁-C₄.

23. Composition selon la revendication 22, **caractérisée par le fait que** les colorants directs cationiques de formule (V) sont choisis parmi les composés répondant aux structures (V1) à (V52) suivantes : 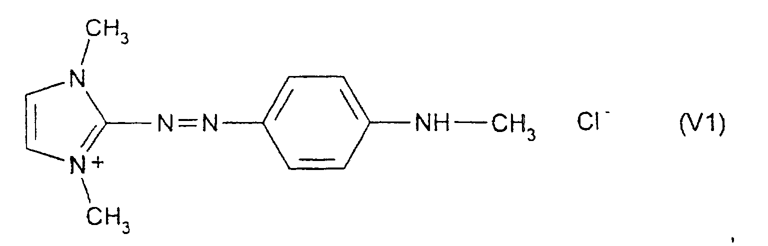 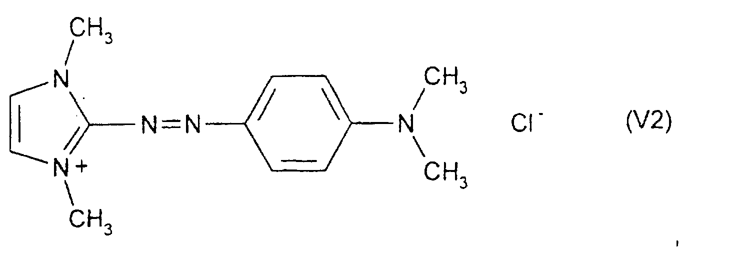 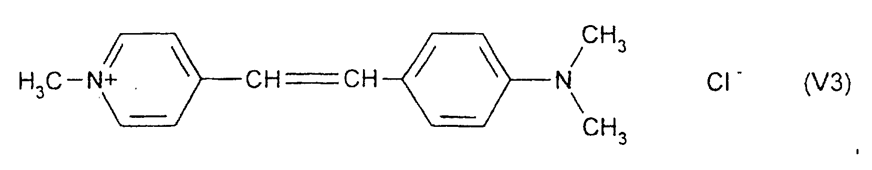 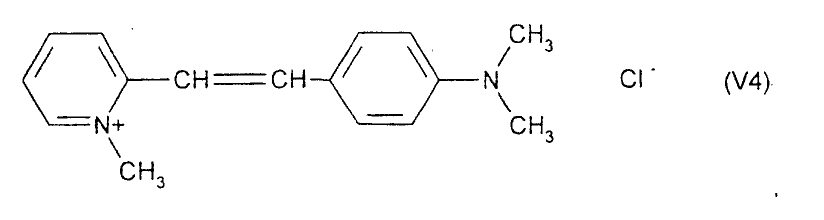 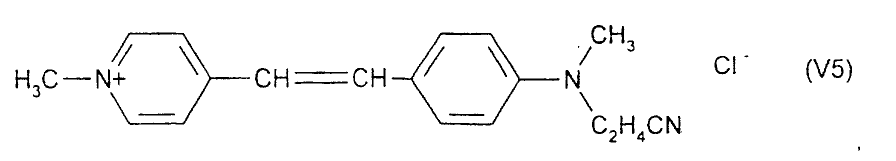 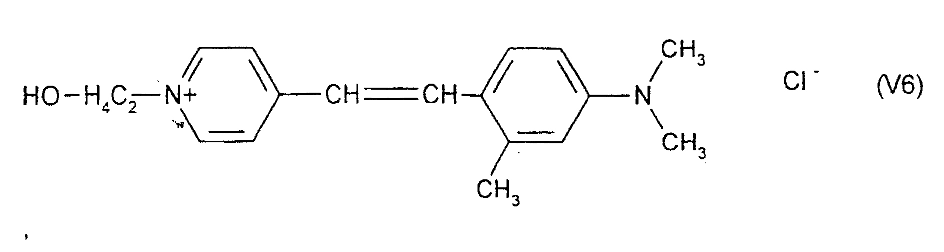 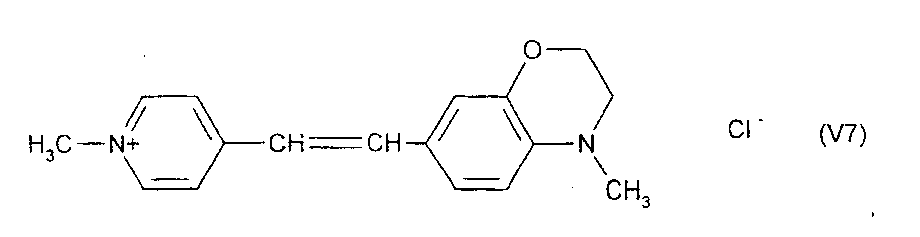 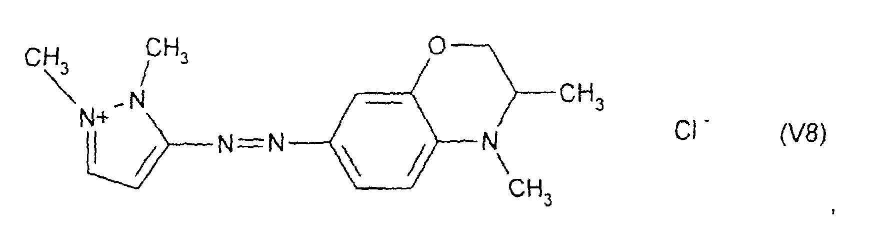 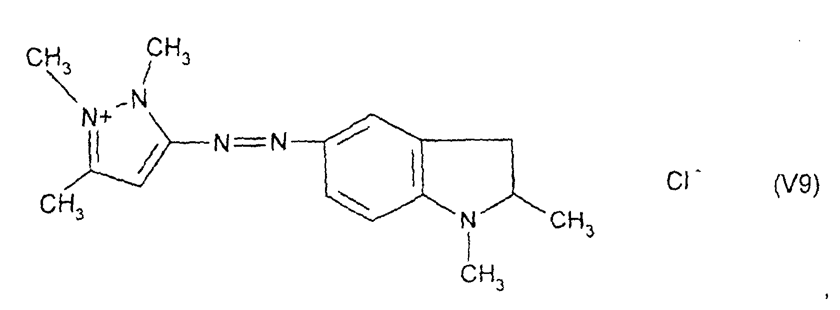 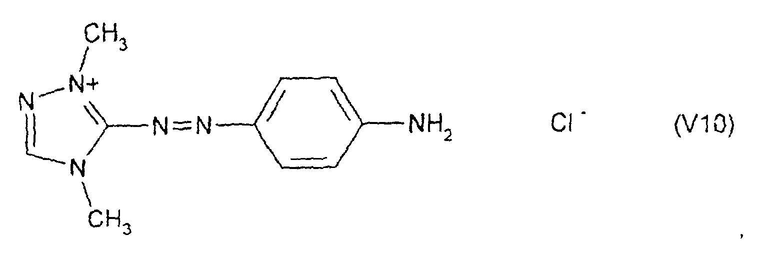 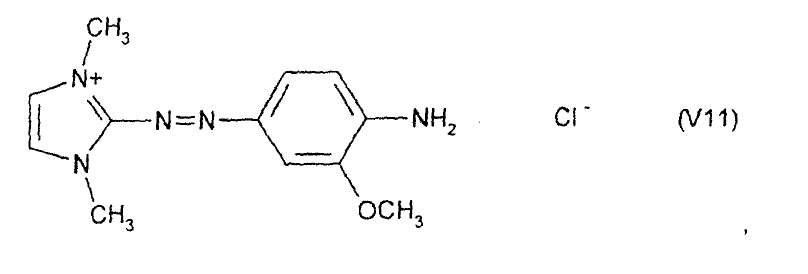 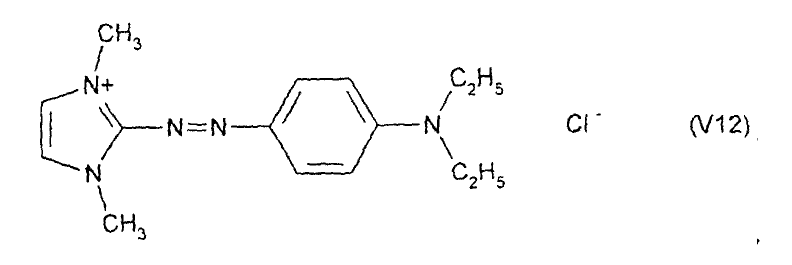 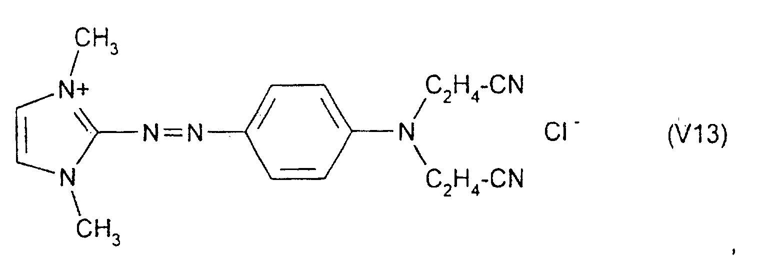 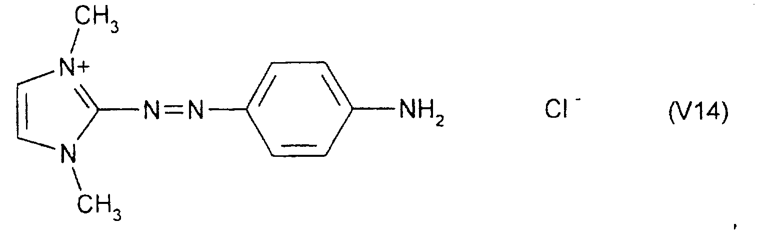 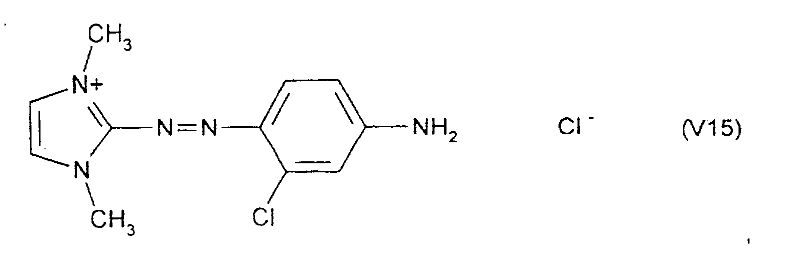 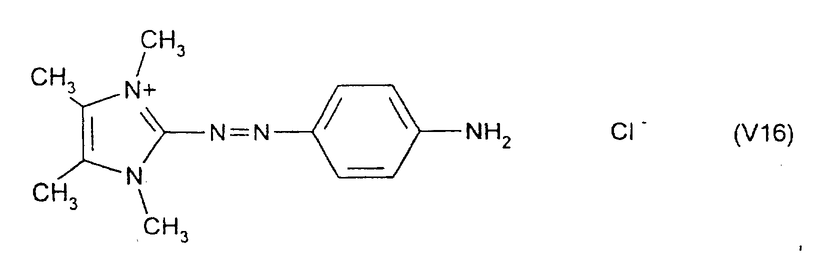 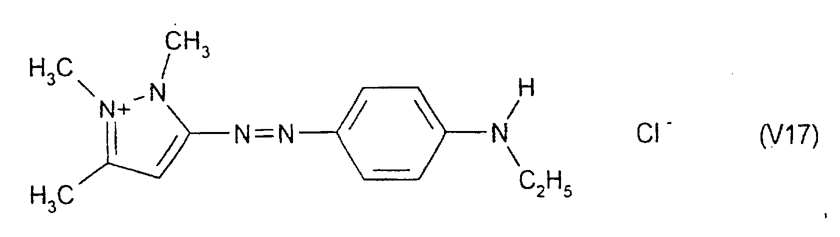 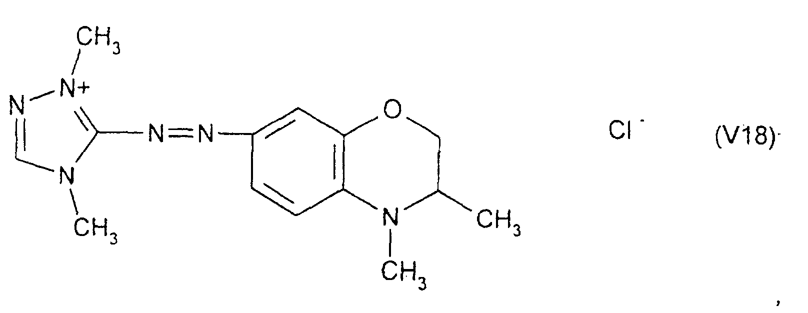 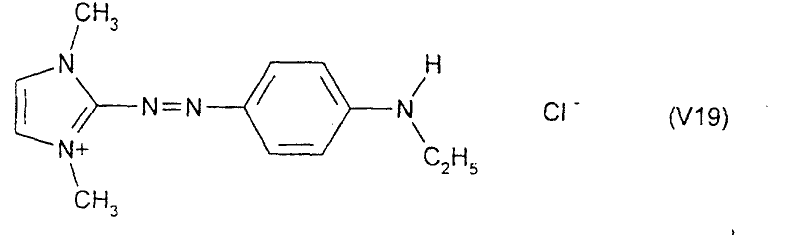 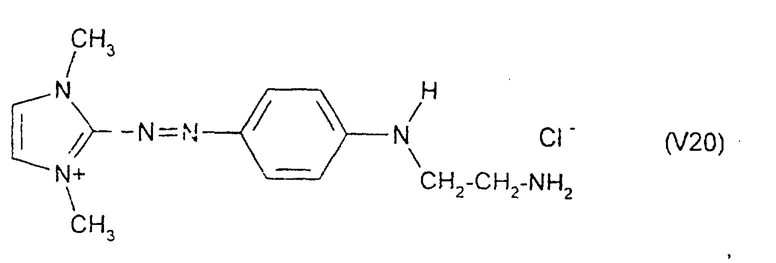 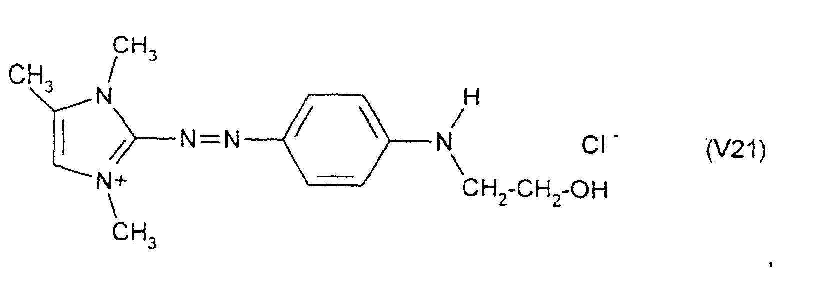 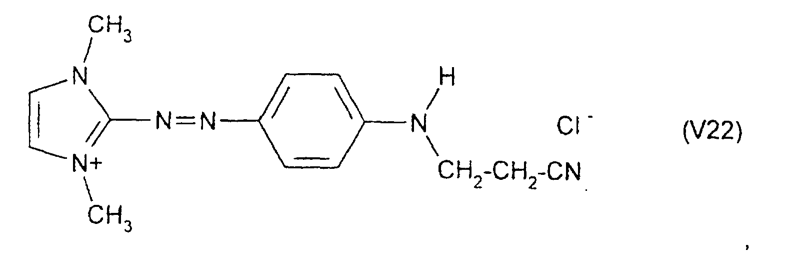 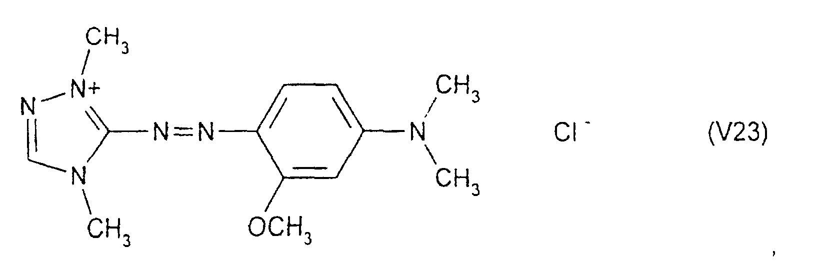 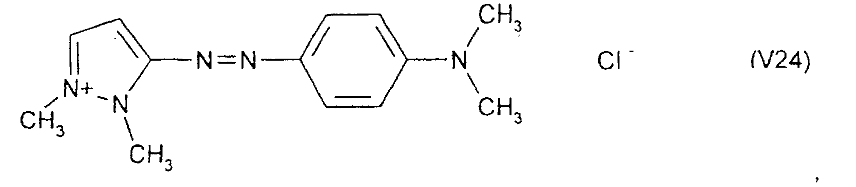 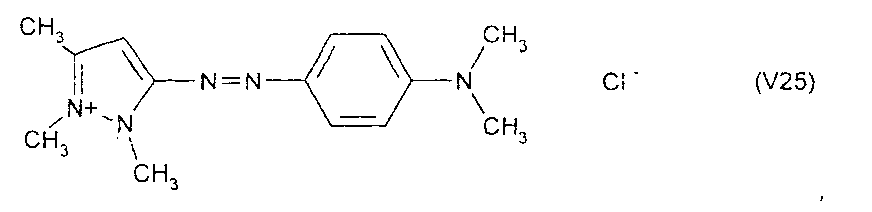 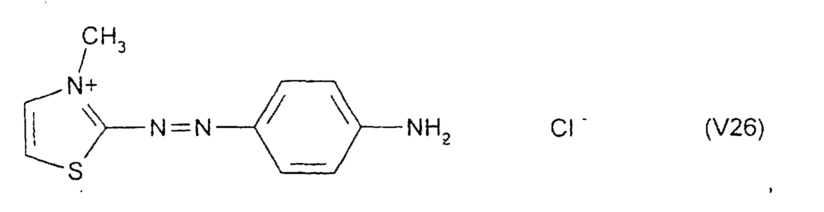 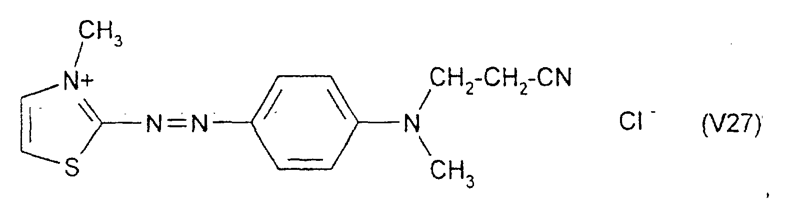 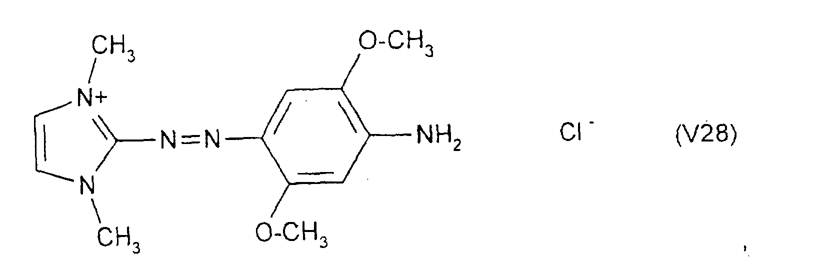 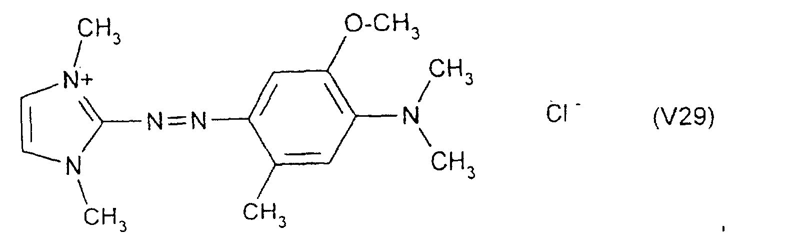 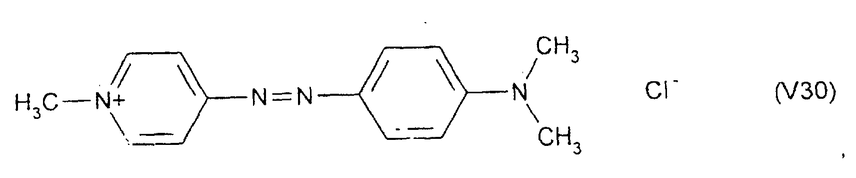 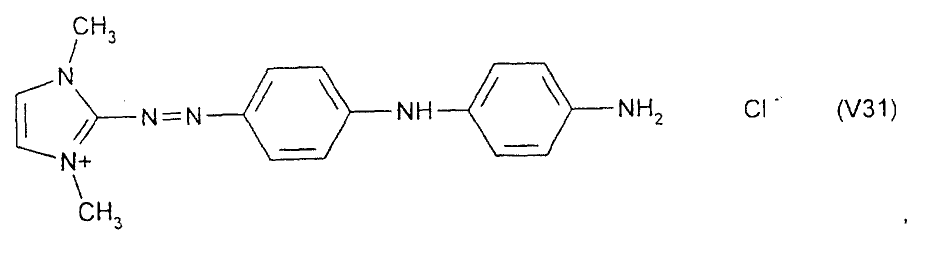 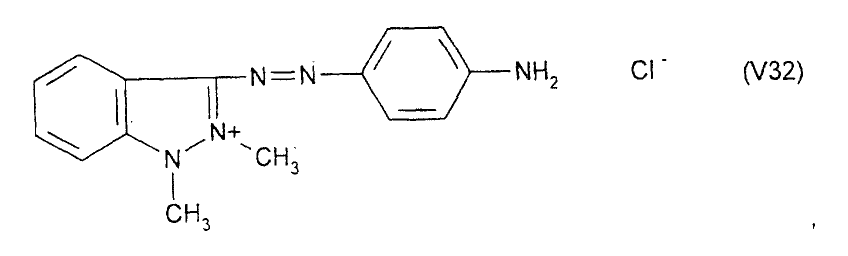 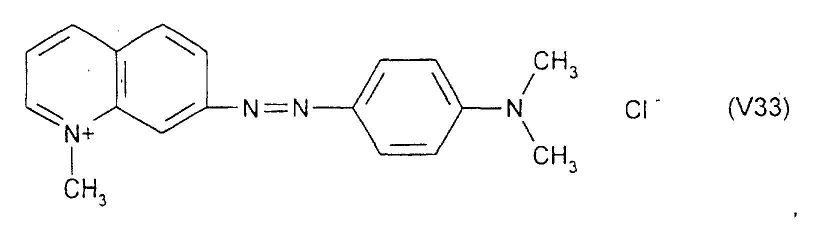 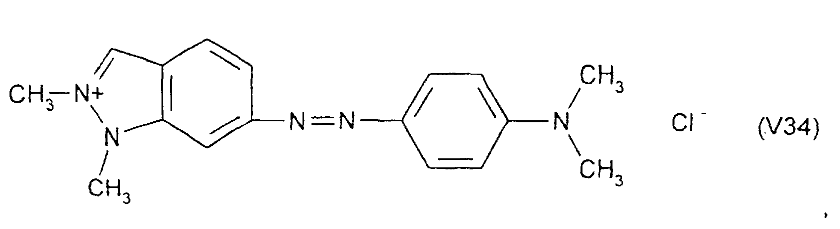 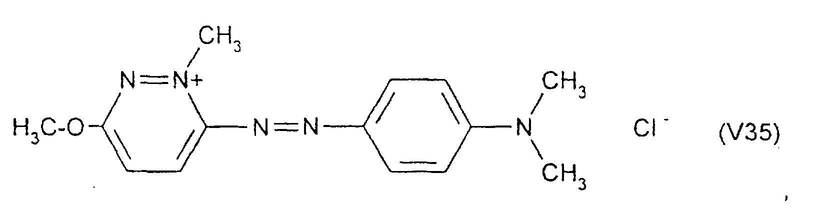 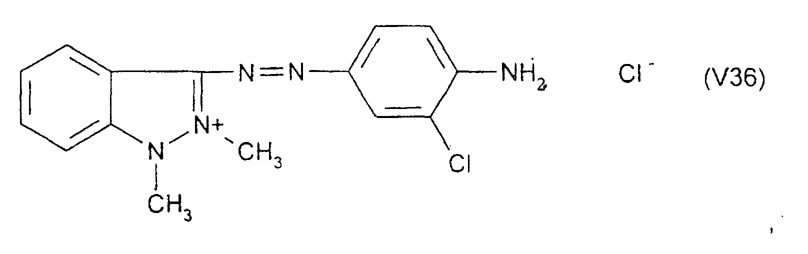 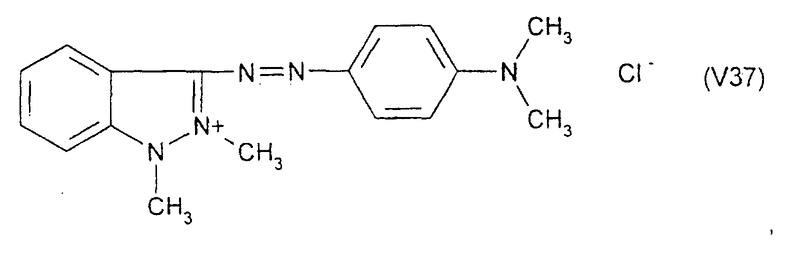 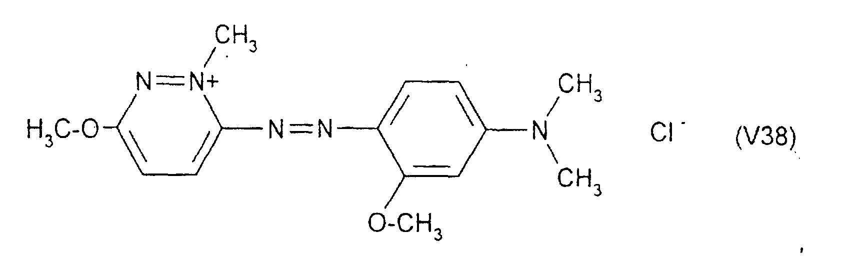 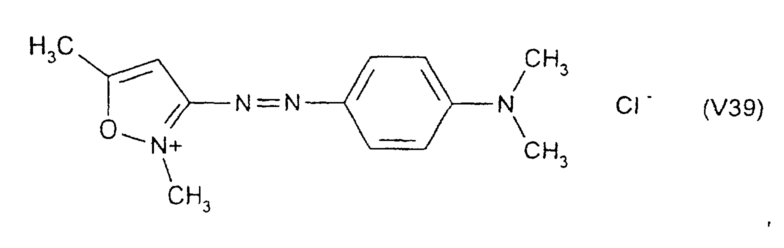 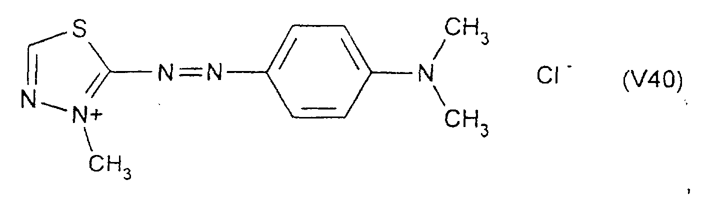 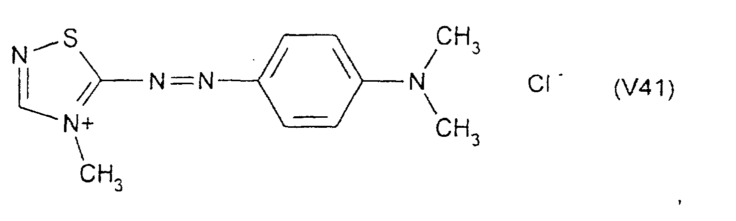 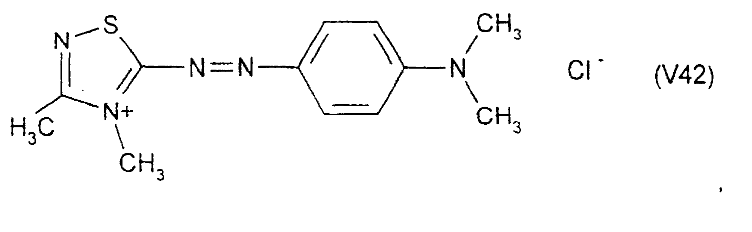 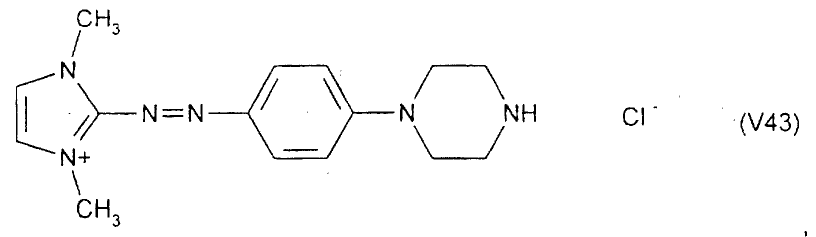 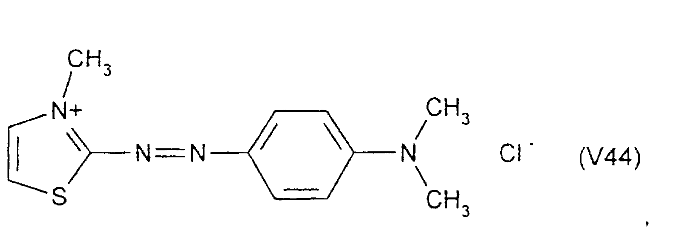 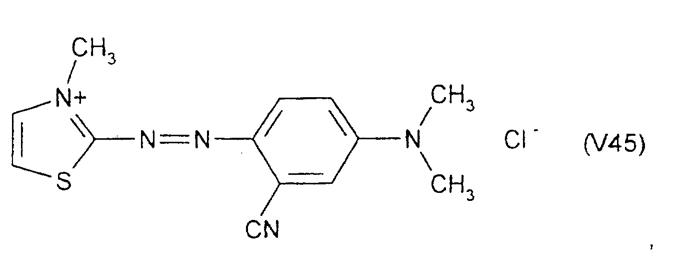 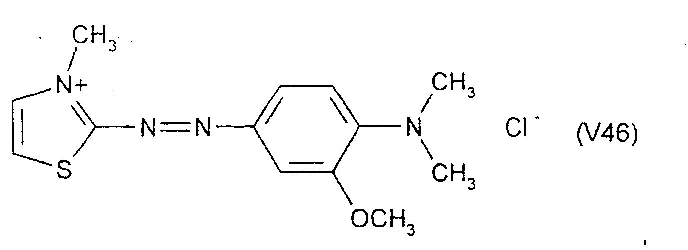 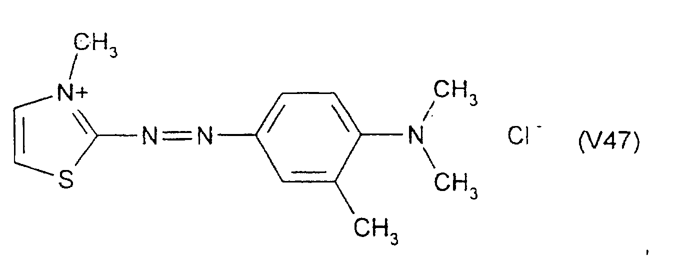 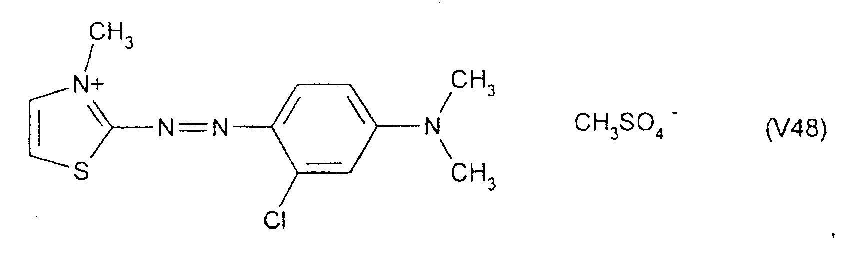 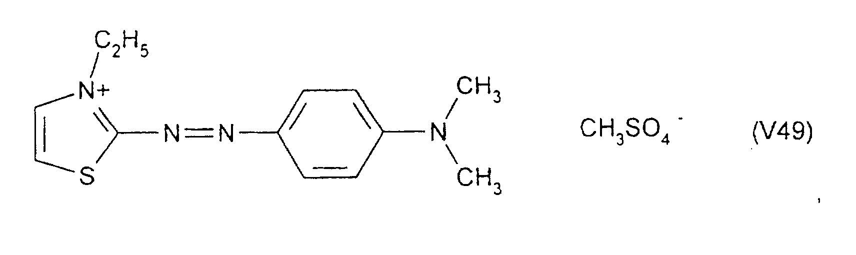 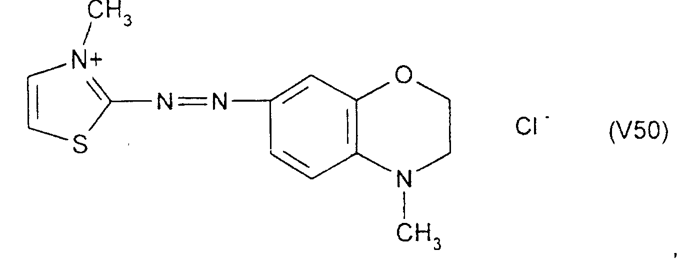 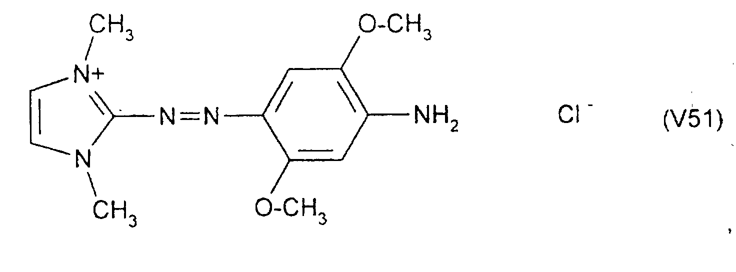 et

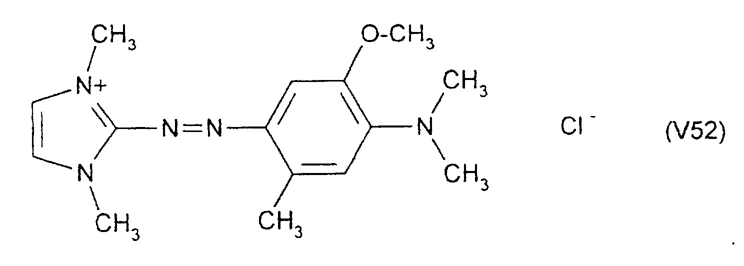

24. Composition selon la revendication 22, **caractérisée par le fait que** les colorants directs cationiques de formule (VI) sont choisis parmi les composés répondant aux structures (VI1) à (VI12) suivantes : 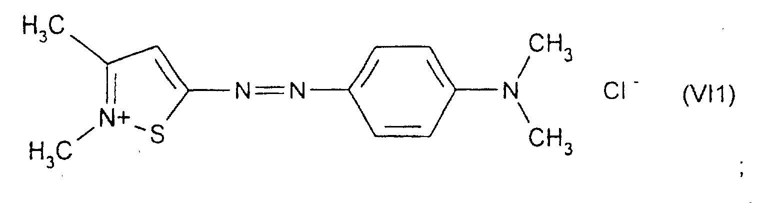 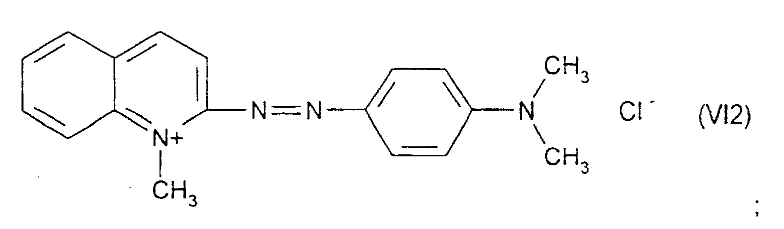 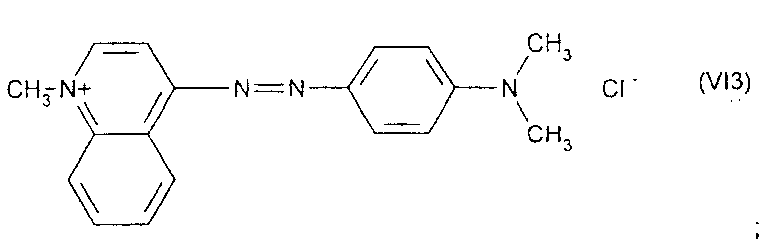 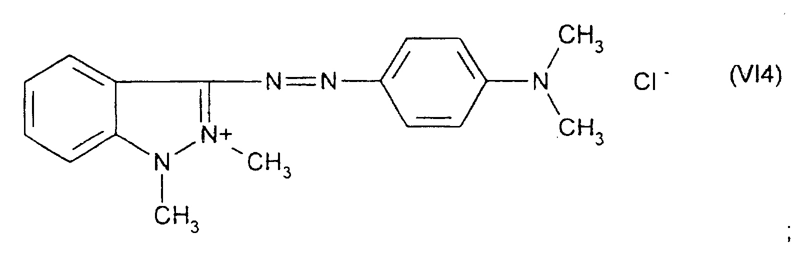 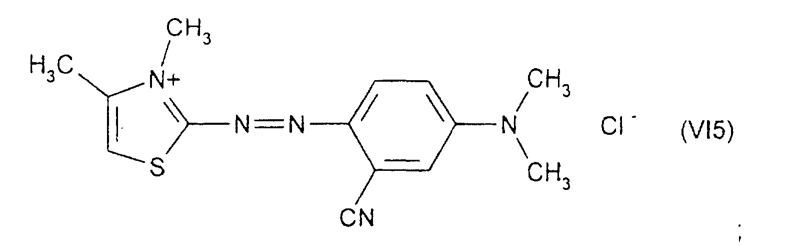 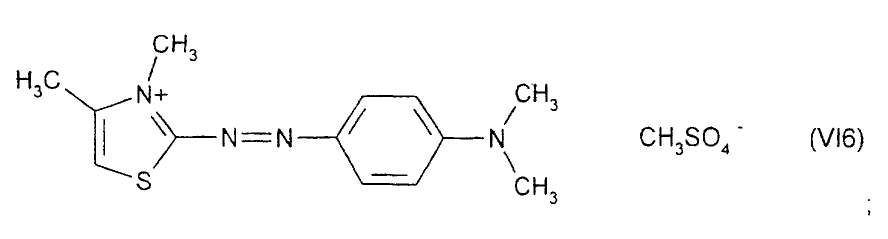 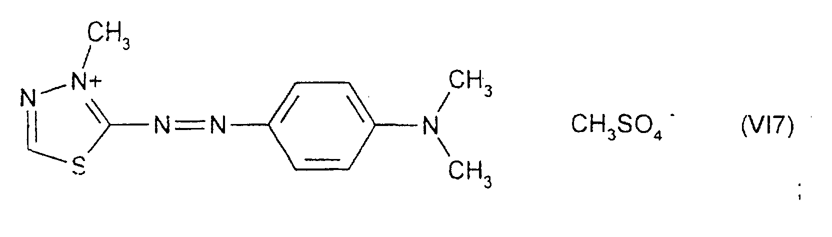 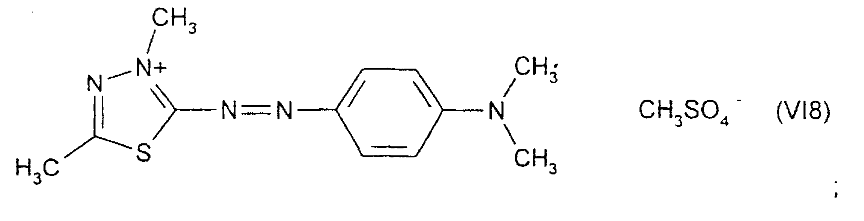 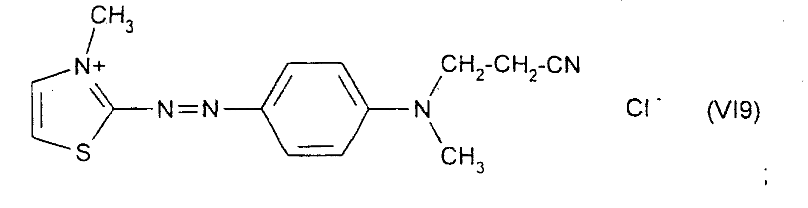 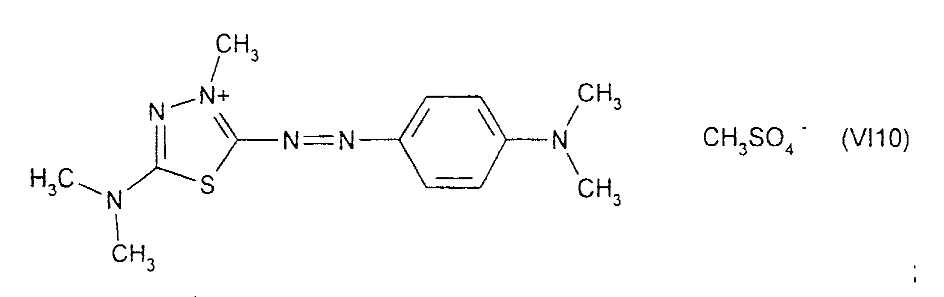 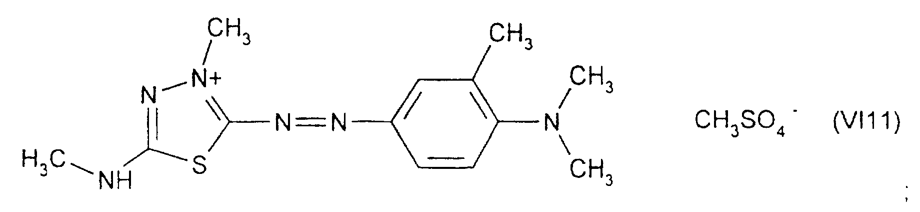 et

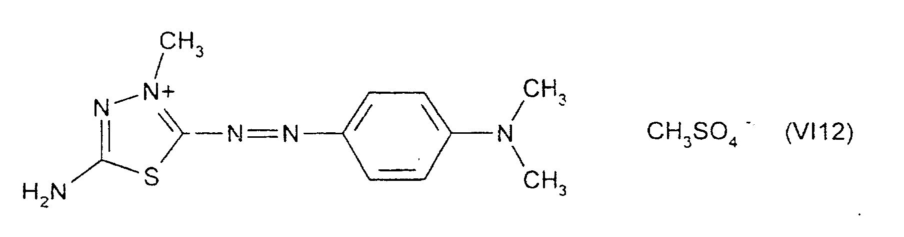

25. Composition selon la revendication 22, **caractérisée par le fait que** les colorants directs cationiques de formule (VII) sont choisis parmi les composés répondant aux structures (VII1) à (VII18) suivantes : 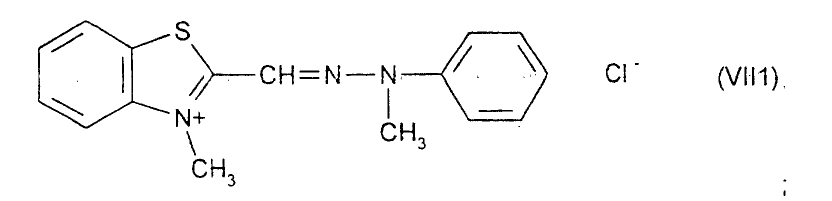 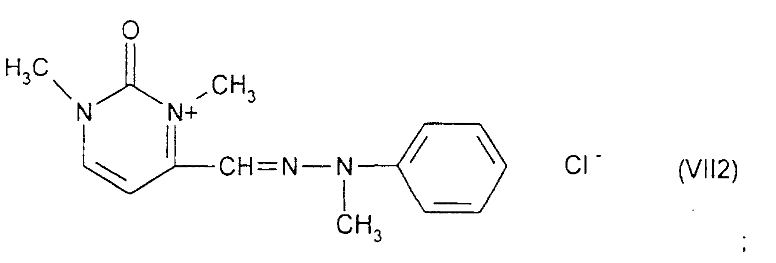 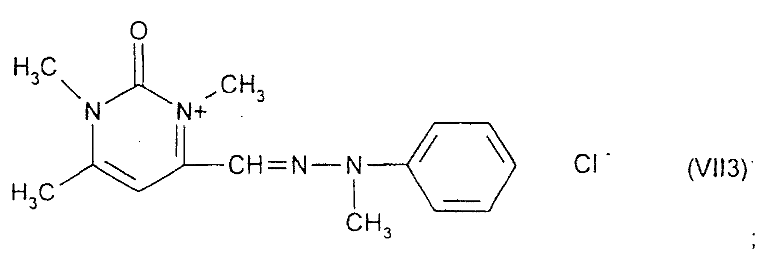 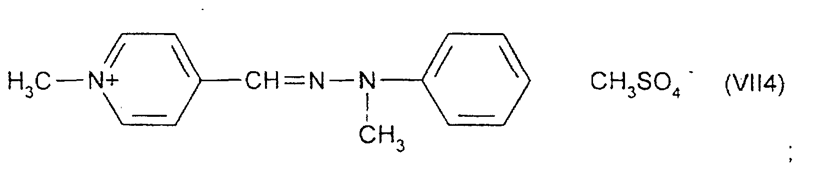 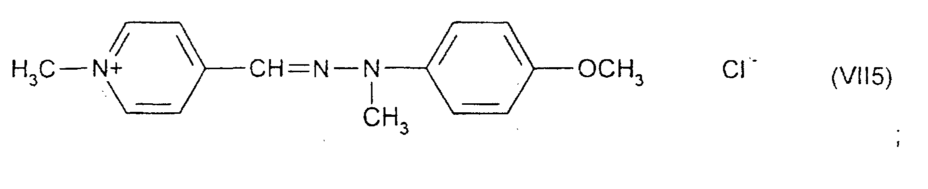 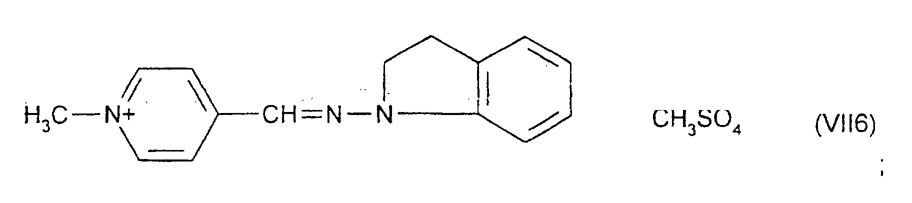 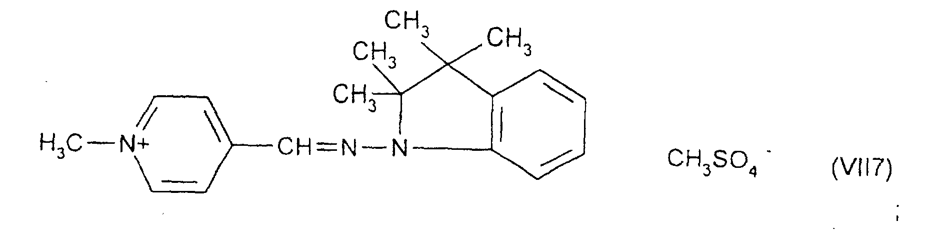 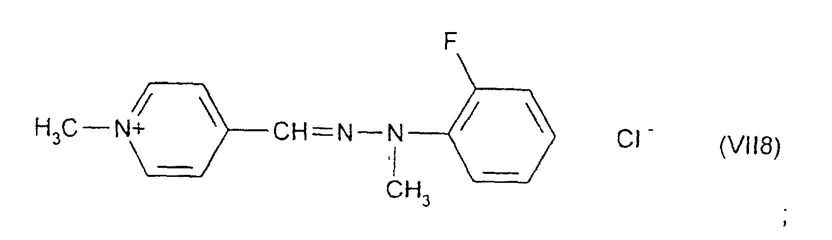 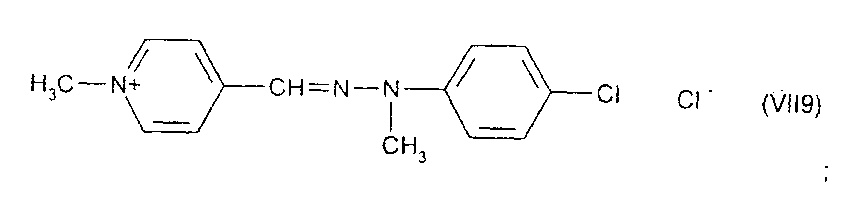 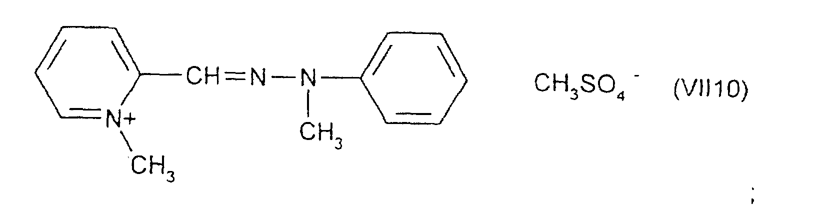 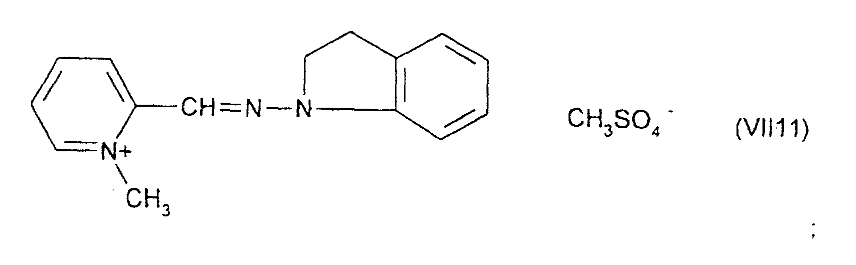 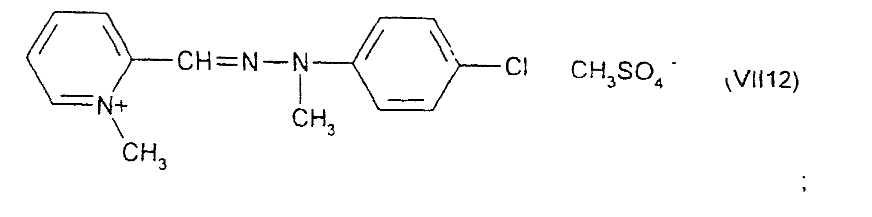 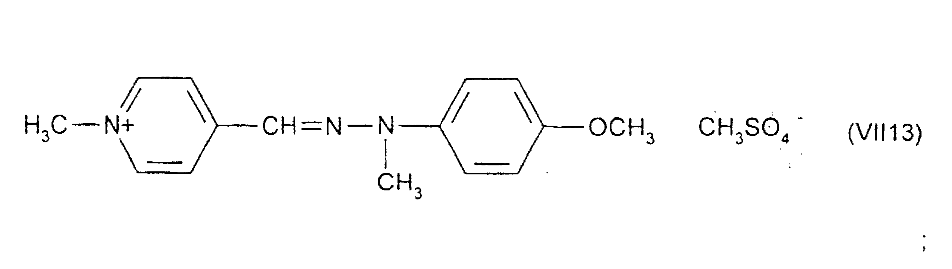 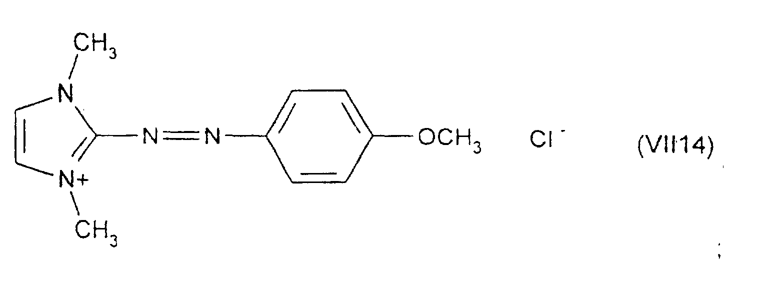 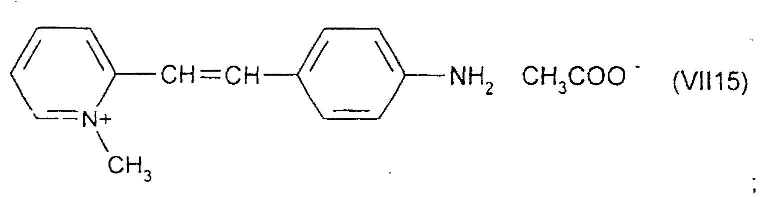 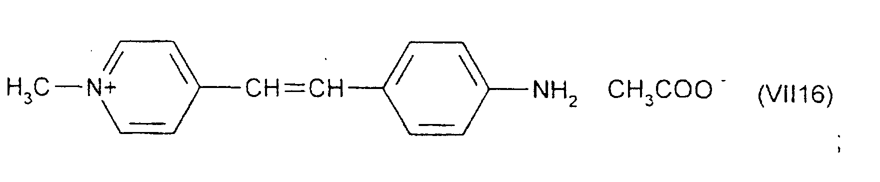 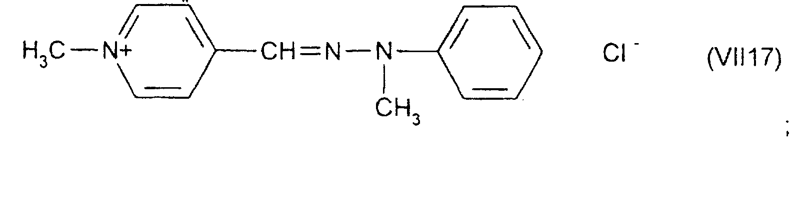 et

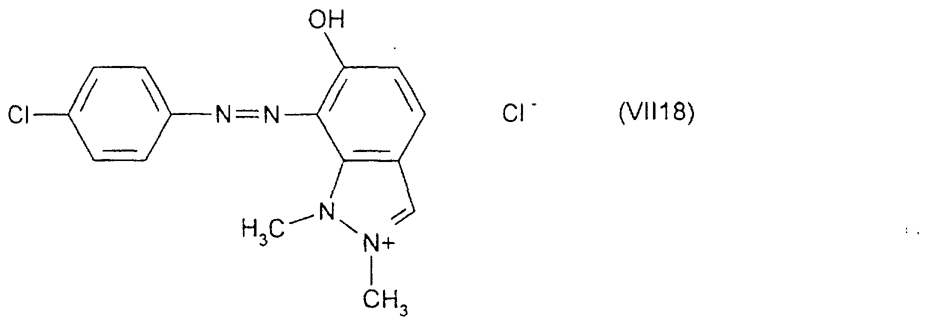

26. Composition selon la revendication 22, **caractérisée par le fait que** les colorants directs cationiques de formule (VII') sont choisis parmi les composés répondant aux structures (VII'1) à (VII'3) suivantes : 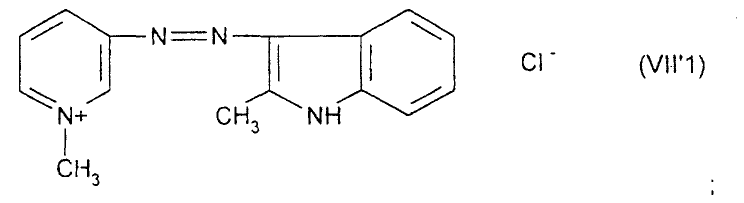 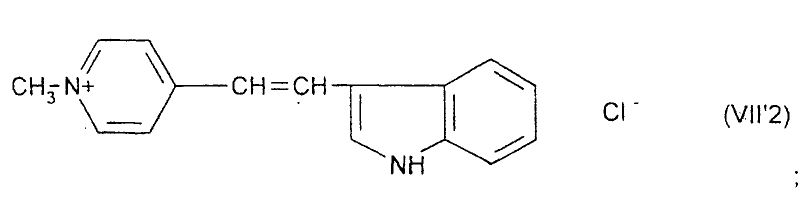 et

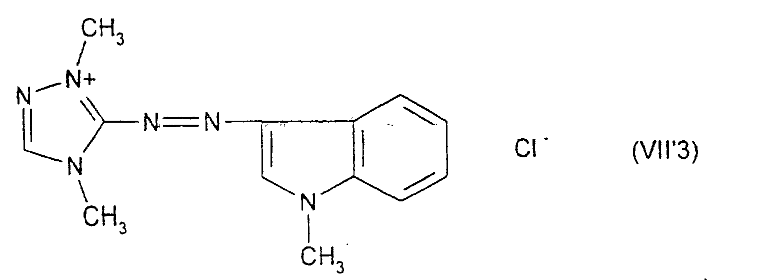

27. Composition selon l'une quelconque des revendications précédentes, **caractérisée par le fait que** le ou les colorants directs cationiques représentent de 0,001 à 10 % en poids du poids total de la composition tinctoriale prête à l'emploi.

28. Composition selon la revendication 27, **caractérisée par le fait que** le ou les colorants directs cationiques représentent de 0,05 à 5 % en poids du poids total de la composition tinctoriale prête à l'emploi.

29. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

30. Composition selon l'une quelconque des revendications précédentes, **caractérisée par le fait que** le milieu approprié pour la teinture est constitué par de l'eau ou par un mélange d'eau et d'au moins un solvant organique.

31. Composition selon l'une quelconque des revendications précédentes, **caractérisée par le fait qu'**elle présente un pH compris 5 et 11.

32. Composition selon l'une quelconque des revendications précédentes, **caractérisée par le fait qu'**elle contient au moins une peroxydase.

33. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

34. Procédé selon la revendication 33, **caractérisé par le fait qu'**il comporte une étape préliminaire consistant à stocker sous forme séparée, d'une part, une composition (A) comprenant, dans un milieu approprié pour la teinture au moins une base d'oxydation, au moins un colorant direct cationique et, d'autre part, une composition (B) renfermant, dans un milieu approprié pour la teinture, au moins une enzyme de type oxydo-réductase à 2 électrons en présence d'au moins un donneur pour ladite enzyme, puis à procéder à leur mélange au moment de l'emploi avant d'appliquer ce mélange sur les fibres kératiniques.

35. Dispositif à plusieurs compartiments ou "kit" de teinture, **caractérisé par le fait qu'**il comporte un premier compartiment renfermant la composition (A) telle que définie dans la revendication 34 et un second compartiment renfermant la composition (B) telle que définie dans la revendication 34.

## Patentansprüche

1. Gebrauchsfertige Zusammensetzung zum oxidativen Färben von Keratinfasern und insbesondere menschlichen Keratinfasern, wie dem Haar, die in einem zum Färben geeigneten Träger enthält:
- mindestens eine Oxidationsbase,
- mindestens einen kationischen Direktfarbstoff,
- mindestens ein Enzym vom Typ der Oxidoreduktasen (2 Elektronen), und
- mindestens einen Donor für das Enzym.

2. Zusammensetzung nach Anspruch 1, **dadurch gekennzeichnet, dass** die Oxidoreduktase(n) (2 Elektronen) unter den Pyranose-Oxidasen, Glucose-Oxidasen, Glycerin-Oxidasen, Lactat-Oxidasen, Pyruvat-Oxidasen und Uricasen ausgewählt sind.

3. Zusammensetzung nach einem der Ansprüche 1 oder 2, **dadurch gekennzeichnet, dass** die Oxidoreduktase(n) (2 Elektronen) unter den Uricasen tierischer, mikrobiologischer oder biotechnologischer Herkunft ausgewählt sind.

4. Zusammensetzung nach einem der vorhergehenden Ansprüche, **dadurch gekennzeichnet, dass** die Oxidoreduktase(n) (2 Elektronen) 0,01 bis 20 Gew.-% des Gesamtgewichts der gebrauchsfertigen Farbmittelzusammensetzung ausmachen.

5. Zusammensetzung nach Anspruch 4, **dadurch gekennzeichnet, dass** die Oxidoreduktase(n) (2 Elektronen) 0,1 bis 5 Gew.-% des Gesamtgewichts der gebrauchsfertigen Farbmittelzusammensetzung ausmachen.

6. Zusammensetzung nach Anspruch 3, **dadurch gekennzeichnet, dass** der Donor (oder das Substrat) für die Oxidoreduktase(n) (2 Elektronen) unter Harnsäure und ihren Salzen ausgewählt ist.

7. Zusammensetzung nach einem der vorhergehenden Ansprüche, **dadurch gekennzeichnet, dass** der Donor oder die Donoren 0,01 bis 20 Gew.-% des Gesamtgewichts der gebrauchsfertigen Farbmittelzusammensetzung ausmachen.

8. Zusammensetzung nach Anspruch 7, **dadurch gekennzeichnet, dass** der Donor oder die Donoren 0,1 bis 5 Gew.-% des Gesamtgewichts der gebrauchsfertigen Farbmittelzusammensetzung ausmachen.

9. Zusammensetzung einem der vorhergehenden Ansprüche, **dadurch gekennzeichnet, dass** die Oxidationsbasen unter den p-Phenylendiaminen, Doppelbasen, p-Aminophenolen, o-Aminophenolen und den heterocyclischen Oxidationsbasen ausgewählt sind.

10. Zusammensetzung nach Anspruch 9, **dadurch gekennzeichnet, dass** die p-Phenylendiamine unter den Verbindungen der folgenden Formel (I) und deren Additionssalzen mit einer Säure ausgewählt sind: 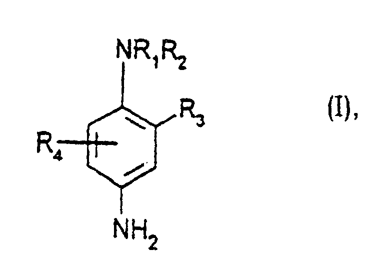 worin bedeuten:
- R₁ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, eine mit einer stickstoffhaltigen Gruppe substituierte C₁₋₄-Alkylgruppe, Phenyl oder 4'-Aminophenyl,
- R₂ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl oder eine mit einer stickstoffhaltigen Gruppe substituierte C₁₋₄-Alkylgruppe,
- R₃ Wasserstoff, Halogen, wie Chlor, Brom, Iod oder Fluor, C₁₋₄-Alkyl, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy, und
- R₄ Wasserstoff, Halogen oder C₁₋₄-Alkyl.

11. Zusammensetzung nach Anspruch 10, **dadurch gekennzeichnet, dass** die p-Phenylendiamine der Formel (I) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-pphenylendiamin, 2,5-Dimethyl-p-phenylendiamin, N,N-Dimethyl-pphenylendiamin, N,N-Diethyl-p-phenylendiamin, N,N-Dipropyl-pphenylendiamin, 4-Amino-N,N-diethyl-3-methyl-anilin, N,N-Bis(β-hydroxyethyl)-p-phenylendiamin, 4-N,N-bis(β-Hydroxyethyl)amino-2-methyl-anilin, 4-N,N-bis(β-Hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-pphenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-pphenylendiamin, N-(β-Methoxyethyl)-p-phenylendiamin und deren Additionssalzen mit einer Säure ausgewählt sind.

12. Zusammensetzung nach Anspruch 9, **dadurch gekennzeichnet, dass** die Doppelbasen unter den Verbindungen der folgenden Formel (II) und deren Additionssalze mit einer Säure ausgewählt sind: 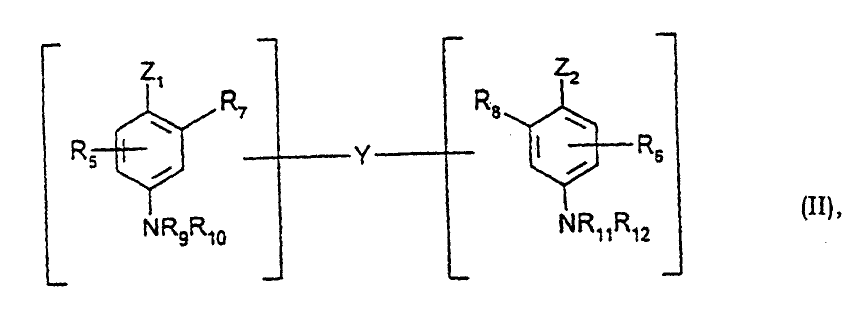 worin bedeuten:
- Z₁ und Z₂, die identisch oder voneinander verschieden sind, Hydroxy oder eine NH₂-Gruppe, die mit C₁₋₄-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder C₁₋₆-Alkoxygruppen substituiert sein kann,
- Rs und R₆ Wasserstoff, Halogen, C₁₋₄-Alkyl, C₁₋₄-Monohydroxyalkyl, C₂₋₄-Polyhydroxyalkyl, C₁₋₄-Aminoalkyl oder eine Verbindungsgruppe Y,
- R₇, R₈, R₉, R₁₀, R₁₁ und R₁₂, die identisch oder voneinander verschieden sind, Wasserstoff, eine Verbindungsgruppe Y oder C₁₋₄-Alkyl,
mit der Maßgabe, dass die Verbindungen der Formel (II) nur eine Verbindungsgruppe Y pro Molekül aufweisen.

13. Zusammensetzung nach Anspruch 12, **dadurch gekennzeichnet, dass** die Doppelbasen der Formel (II) unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'amino, 3'-methylphenyl)-ethylendiamin, 1,8-Bis(2,5-diaminophenoxy)-3,5-dioxaoctan und deren Additionssalzen mit einer Säure ausgewählt sind.

14. Zusammensetzung nach Anspruch 9, **dadurch gekennzeichnet, dass** die p-Aminophenole unter den Verbindungen der folgenden Formel (III) und deren Additionssalze mit einer Säure ausgewählt sind: 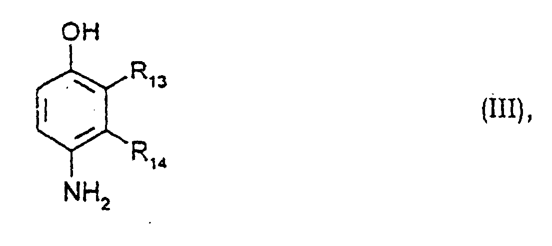 worin bedeuten:
- R₁₃ Wasserstoff, Halogen, C₁₋₄-Alkyl, C₁₋₄-Monohydroxyalkyl, C₁₋₄-Alkoxy-C₁₋₄-alkyl, C₁₋₄-Aminoalkyl oder C₁₋₄-Hydroxyalkyl-C₁₋₄aminoalkyl,
- R₁₄ Wasserstoff, Halogen, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
mit der Maßgabe, dass mindestens eine der Gruppen R₁₃ oder R₁₄ Wasserstoff bedeutet.

15. Zusammensetzung nach Anspruch 14, **dadurch gekennzeichnet, dass** die p-Aminophenole der Formel (III) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

16. Zusammensetzung nach Anspruch 9, **dadurch gekennzeichnet, dass** die o-Aminophenole unter 2-Aminophenol, 2-Amino-5-methylphenol, 2-Amino-6-methyl-phenol, 5-Acetamido-2-amino-phenol und deren Additionssalzen mit einer Säure ausgewählt sind.

17. Zusammensetzung nach Anspruch 9, **dadurch gekennzeichnet, dass** die heterocyclischen Oxidationsbasen unter den Pyridinderivaten, Pyrimidinderivaten, Pyrazolderivaten, Pyrazolopyrimidinderivaten und deren Additionssalzen mit einer Säure ausgewählt sind.

18. Zusammensetzung nach einem der vorhergehenden Ansprüche, **dadurch gekennzeichnet, dass** die Oxidationsbase(n) 0,0005 bis 12 Gew.-% des Gesamtgewichts der Farbmittelzusammensetzung ausmachen.

19. Zusammensetzung nach Anspruch 18, **dadurch gekennzeichnet, dass** die Oxidationsbase(n) 0,005 bis 6 Gew.-% des Gesamtgewichts der Farbmittelzusammensetzung ausmachen.

20. Zusammensetzung nach einem der vorhergehenden Ansprüche, **dadurch gekennzeichnet, dass** der oder die kationischen Direktfarbstoffe unter den kationischen Aminoanthrachinonen, den kationischen Mono- oder Bis-Azo-Farbstoffen und den kationischen Naphthochinonen ausgewählt sind.

21. Zusammensetzung nach Anspruch 20, **dadurch gekennzeichnet, dass** die kationischen Direktfarbstoffe unter [8-[(p-Aminophenyl)-azol]-7-hydroxy-2-naphthyl]-trimethylammoniumchlorid, 3-[(4-Amino-6-brom-5,8-dihydro-1-hydroxy-8-imino-5-oxo-2-naphthalinyl)-amino]-N,N,N-trimethyl-benzolaminiumchlorid, 7-Hydroxy-8-[(2-methoxyphenyl]azo]-N,N,N-trimethyl-2-naphthalinaminiumchlorid, [8-[(4-Amino-2-nitrophenyl)azo]-7-hydroxy-2-naphthyl]-trimethylammoniumchlorid und 3-[(4,5-Dihydro-3-methyl-5-oxo-1-phenyl-1H-pyrazol-4-yl)azo]-N,N,N-trimethylbenzolaminiumchlorid ausgewählt sind.

22. Zusammensetzung nach einem der Ansprüche 1 bis 19, **dadurch gekennzeichnet, dass** der oder die kationischen Direktfarbstoffe ausgewählt sind unter:
(a) den Verbindungen der folgenden Formel (V): 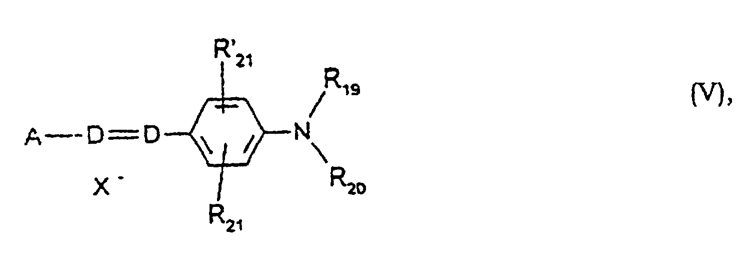 worin bedeuten:
- D ein Stickstoffatom oder die Gruppe -CH,
- die Gruppen R₁₉ und R₂₀, die identisch oder voneinander verschieden sind, Wasserstoff; eine C₁₋₄-Alkylgruppe, die mit -CN, -OH oder -NH₂ substituiert sein kann oder mit einem Kohlenstoffatom des Benzolrings einen gegebenenfalls sauerstoff- oder stickstoffhaltigen Heterocyclus bildet, der mit einer oder mehre-, ren C₁₋₄-Alkylgruppen substituiert sein kann; 4'-Aminophenyl,
- die Gruppen R₂₁ und R'₂₁, die identisch oder voneinander verschieden sind, Wasserstoff oder ein Halogenatom, das unter Chlor, Brom, Iod und Fluor ausgewählt ist, Cyano, C₁₋₄-Alkoxy oder Acetyloxy,
- X⁻ ein Anion, das vorzugsweise unter Chlorid, Methylsulfat und Acetat ausgewählt ist,
- A eine Gruppe, die unter den folgenden Strukturen A1 bis A19 ausgewählt ist: 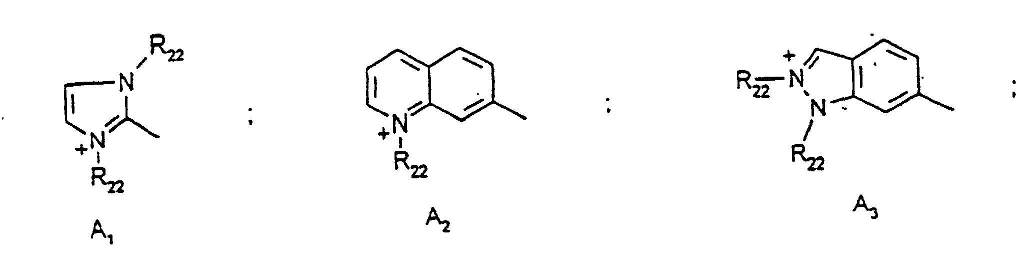 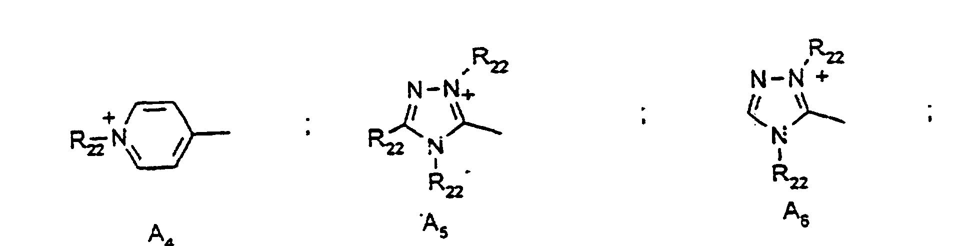 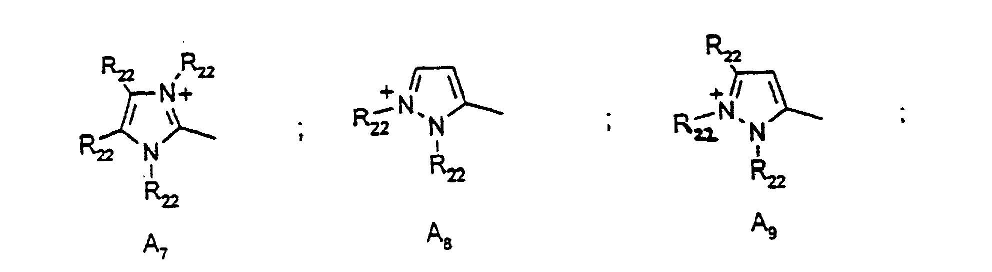 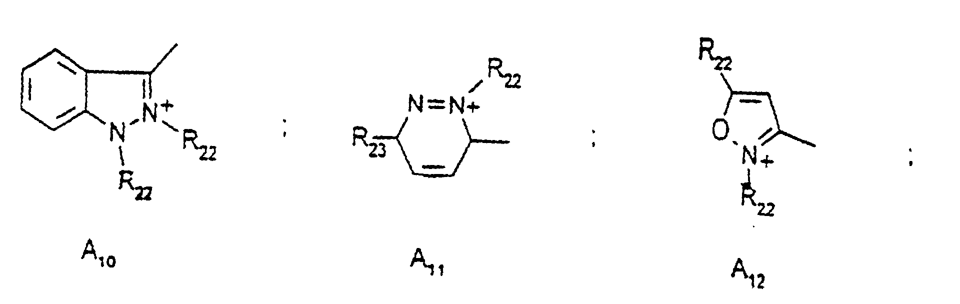 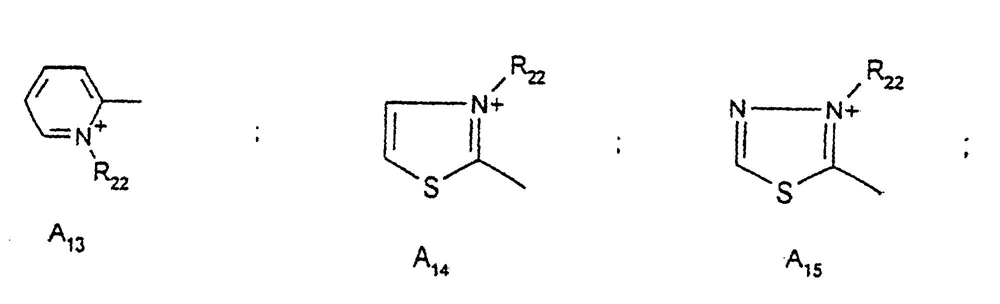 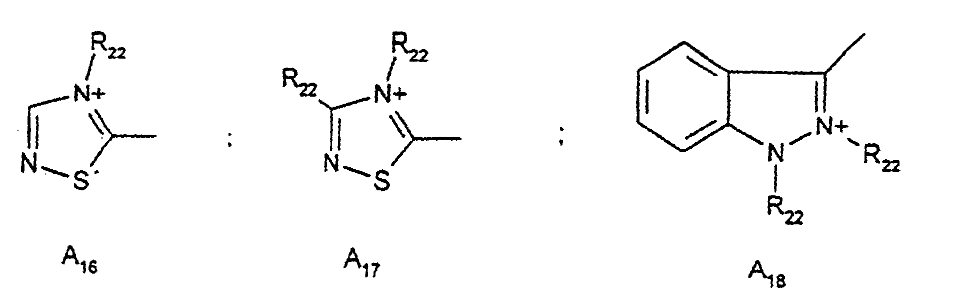 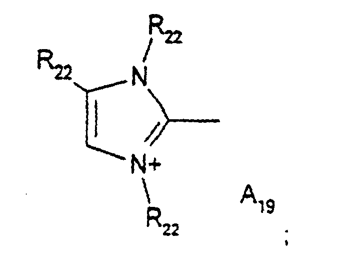
worin R₂₂ eine C₁₋₄-Alkylgruppe, die mit Hydroxy substituiert sein kann, und R₂₃ eine C₁₋₄-Alkoxygruppe bedeutet;
(b) den Verbindungen der folgenden Formel (VI): 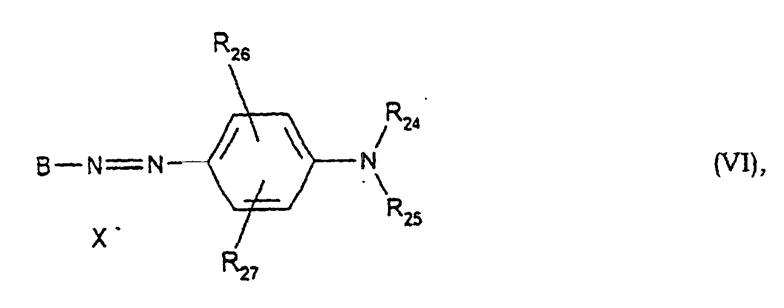 worin bedeuten:
- R₂₄ Wasserstoff oder eine C₁₋₄-Alkylgruppe,
- R₂₅ Wasserstoff, eine Alkylgruppe, die mit -CN oder einer Aminogruppe substituiert sein kann, 4'-Aminophenyl, oder R₂₅ bildet mit R₂₄ einen gegebenenfalls Sauerstoff- und/oder stickstoffhaltigen Heterocyclus, der mit C₁₋₄-Alkyl substituiert sein kann,
- die Gruppen R₂₆ und R₂₇, die identisch oder voneinander verschieden sind, Wasserstoff oder ein Halogenatom, wie Brom, Chlor, Iod und Fluor, C₁₋₄-Alkyl, C₁₋₄-Alkoxy oder -CN,
- X⁻ ein Anion, das vorzugsweise unter Chlorid, Methylsulfat und Acetat ausgewählt ist,
- B eine Gruppe, die unter den folgenden Strukturen B1 bis B6 ausgewählt ist: 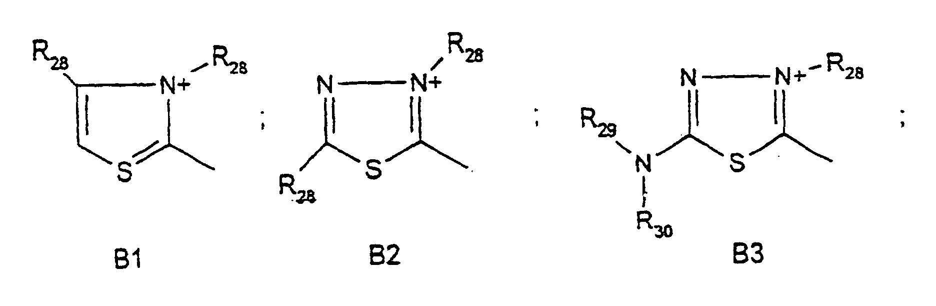 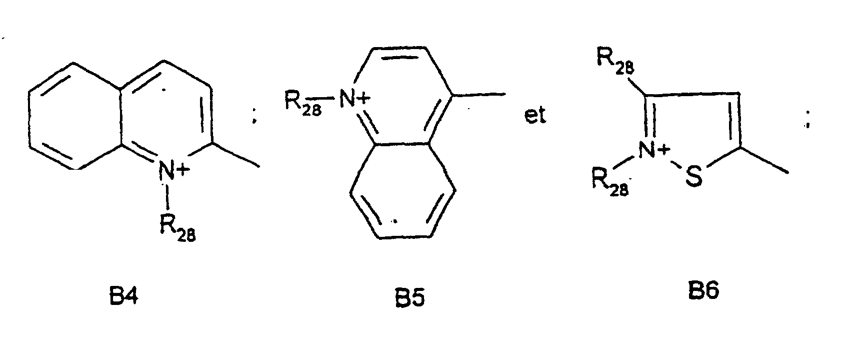
worin R₂₈ eine C₁₋₄-Alkylgruppe und R₂₉ und R₃₀, die identisch oder voneinander verschieden sind, Wasserstoff oder eine C₁₋₄-Alkylgruppe bedeuten;
(c) den Verbindungen der folgenden Formel (VII) und (VII'): 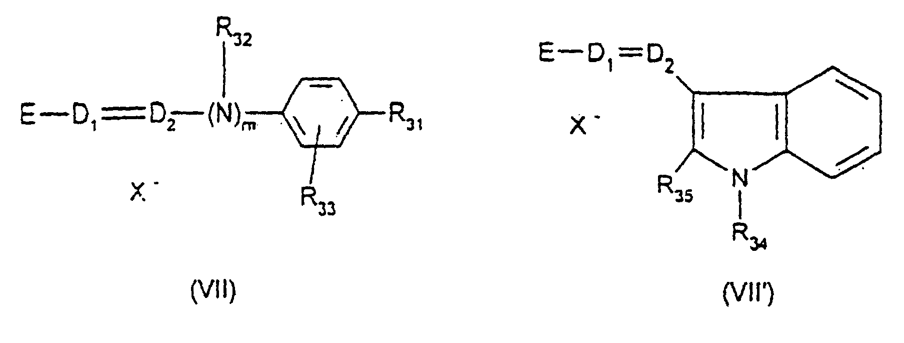
worin bedeuten:
- R₃₁ Wasserstoff, C₁₋₄-Alkoxy, ein Halogenatom, wie Brom, Chlor, Iod und Fluor, oder Amino,
- R₃₂ Wasserstoff oder C₁₋₄-Alkyl oder R₃₂ bildet mit einem Kohlenstoffatom des Benzolrings einen gegebenenfalls sauerstoffhaltigen und/oder mit einer oder mehreren C₁₋₄-Alkylgruppen substituierten Heterocyclus,
- R₃₃ Wasserstoff oder ein Halogenatom, wie Brom, Chlor, Iod und Fluor,
- die Gruppen R₃₄ und R₃₅, die identisch oder voneinander verschieden sind, Wasserstoff oder C₁₋₄-Alkyl,
- D₁ und D₂, die identisch oder voneinander verschieden sind, Stickstoff oder -CH,
- m = 0 oder 1,
- mit der Maßgabe, dass D₁ und D₂ gleichzeitig -CH bedeuten und m = 0, wenn R₃₁ eine unsubstituierte Aminogruppe ist,
- X⁻ ein Anion, das vorzugsweise unter Chlorid, Methylsulfat und Acetat ausgewählt ist,
- E eine Gruppe, die unter den folgenden Strukturen E1 bis E8 ausgewählt ist: 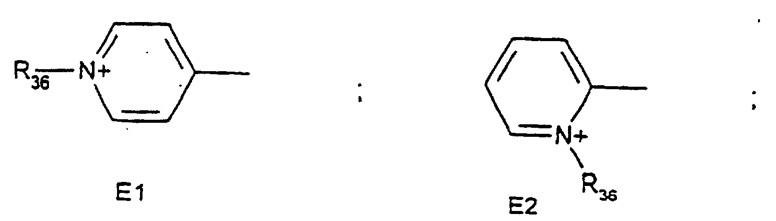 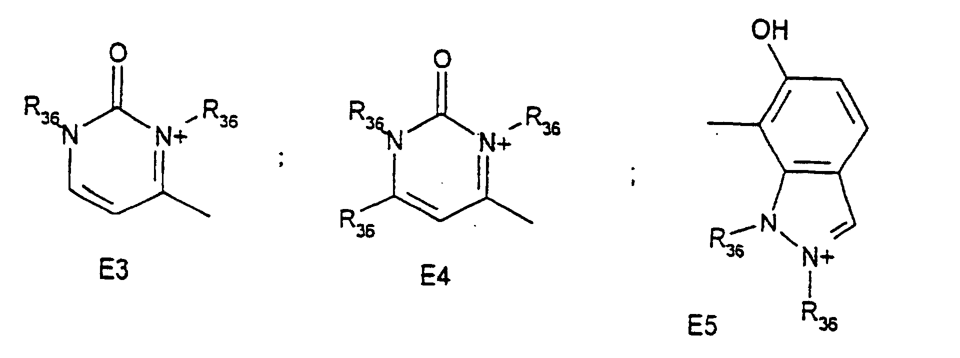 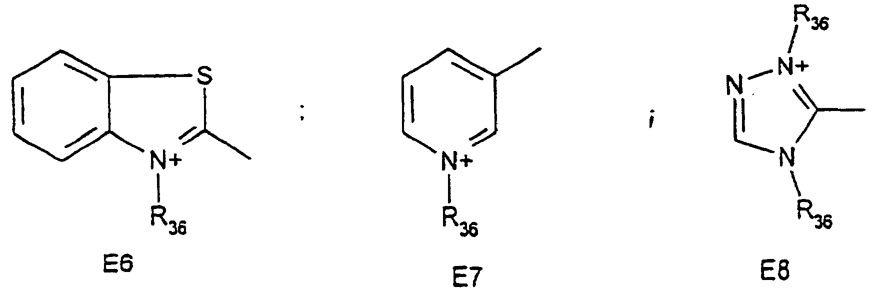
worin R₃₆ eine C₁₋₄-Alkylgruppe bedeutet,
wenn m Null ist und D₁ Stickstoff bedeutet, kann E auch eine Gruppe der folgenden Struktur E9 sein: 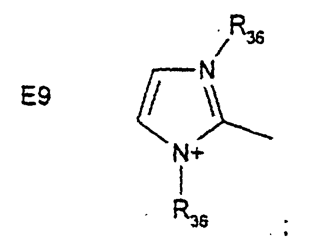 worin R₃₆ eine C₁₋₄-Alkylgruppe bedeutet.

23. Zusammensetzung nach Anspruch 22, **dadurch gekennzeichnet, dass** die kationischen Direktfarbstoffe der Formel (V) unter den Verbindungen der folgenden Strukturen (V1) bis (V52) ausgewählt sind:

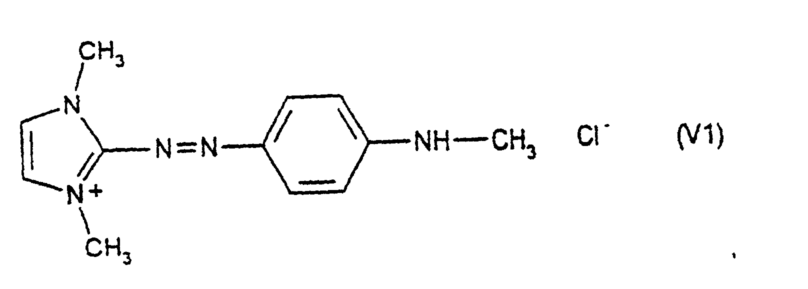

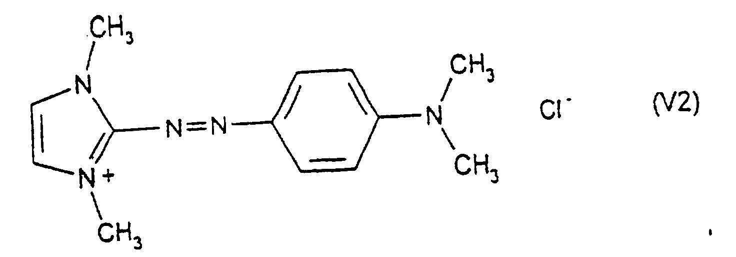

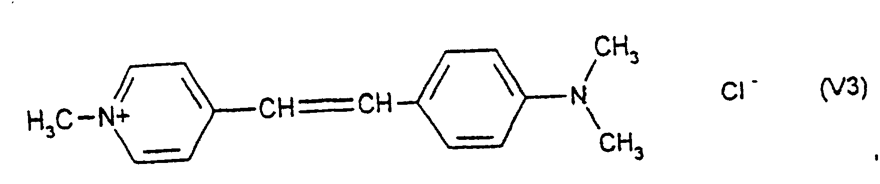

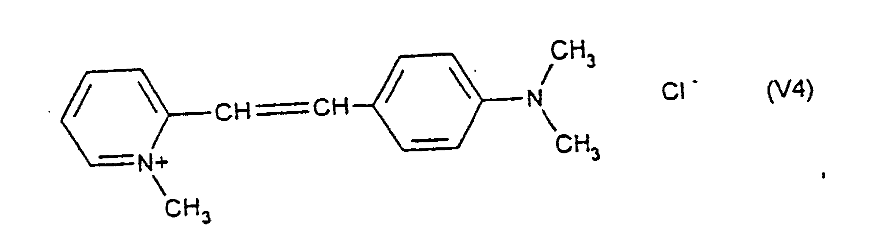

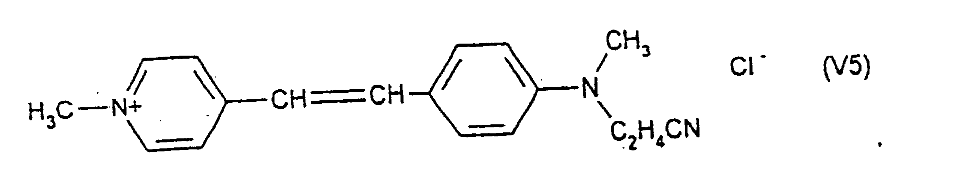

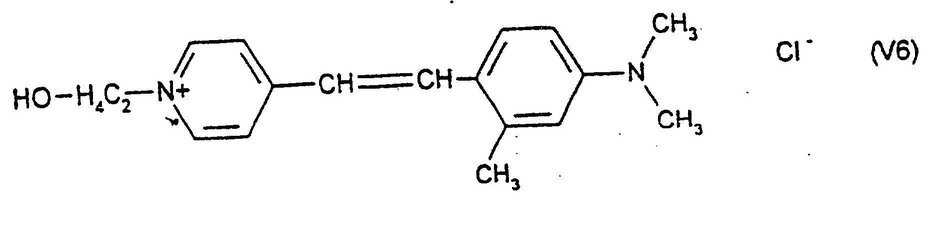

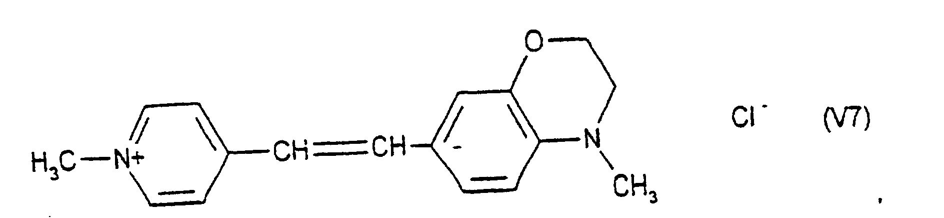

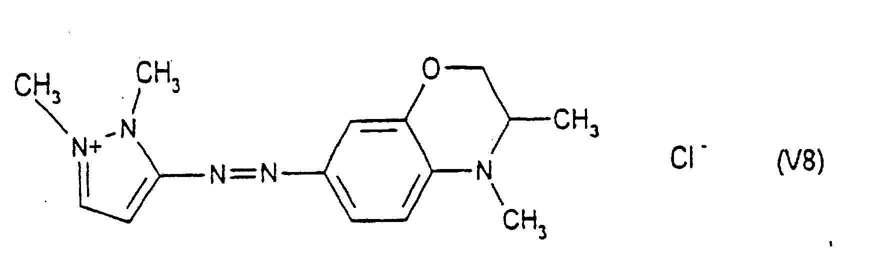

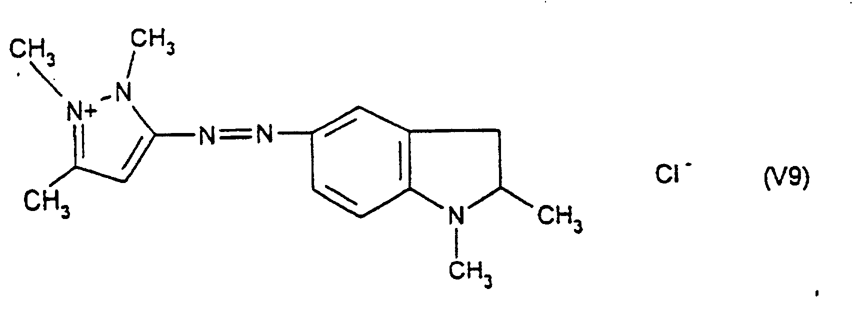

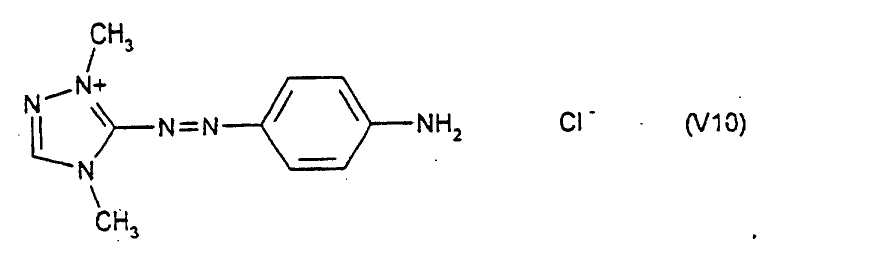

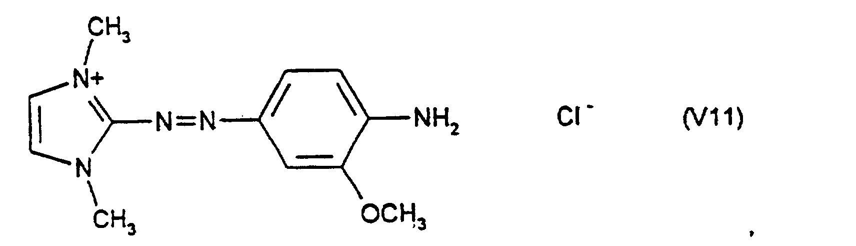

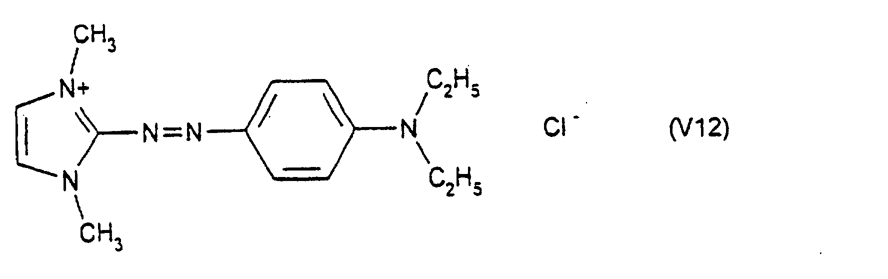

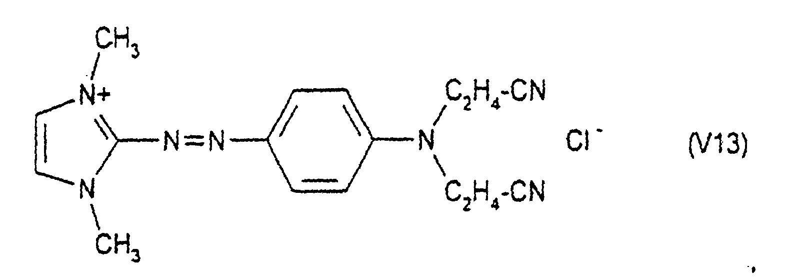

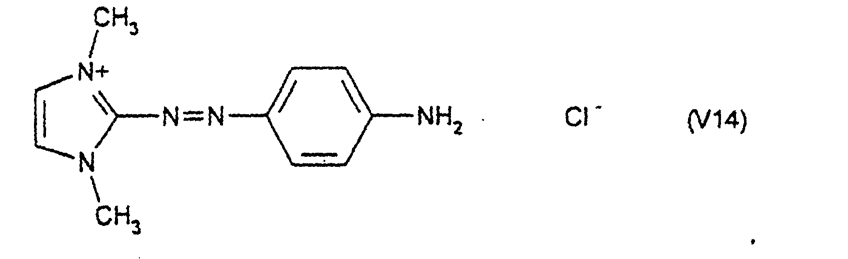

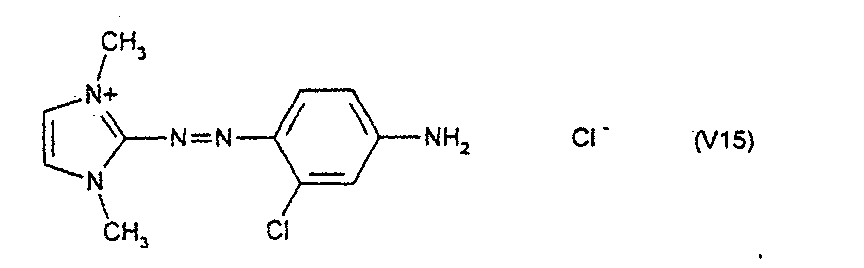

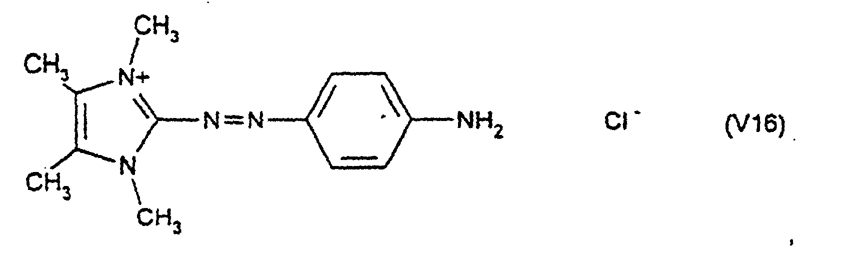

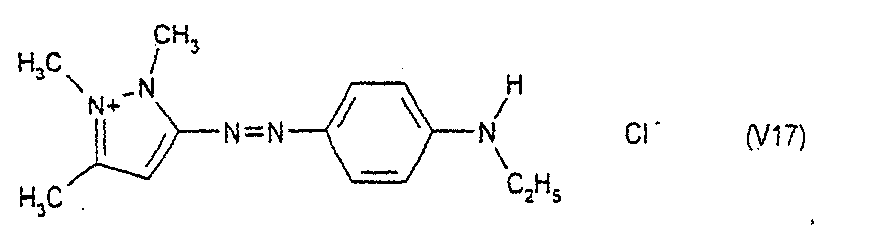

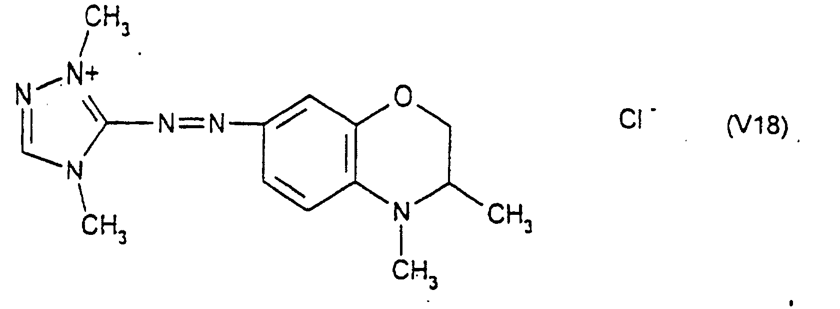

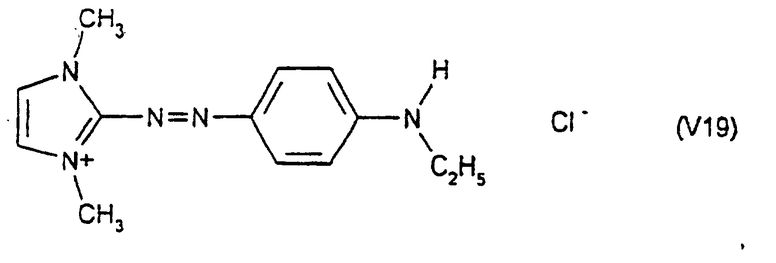

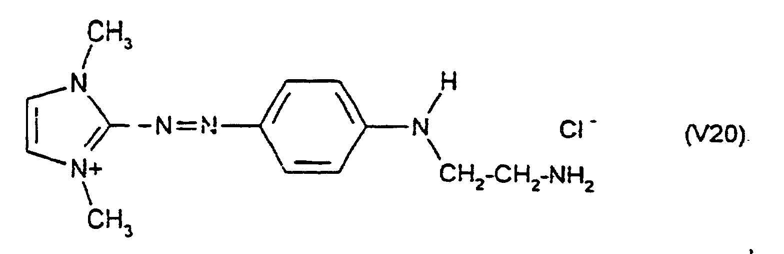

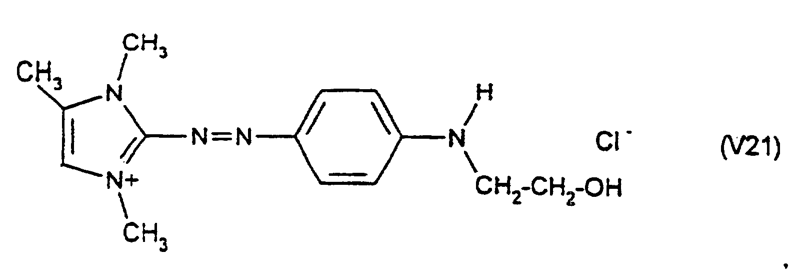

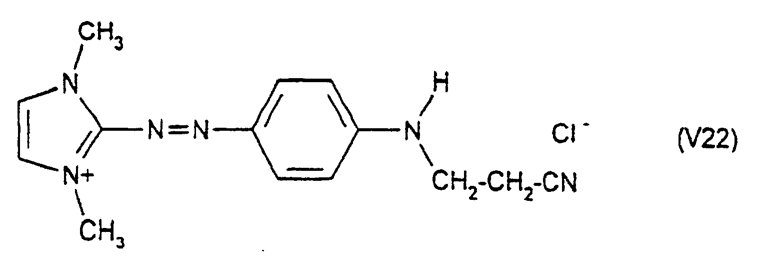

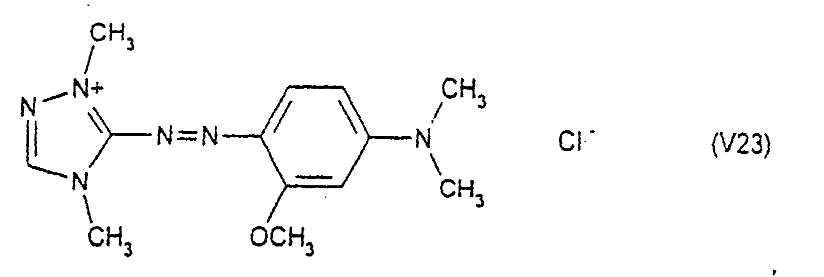

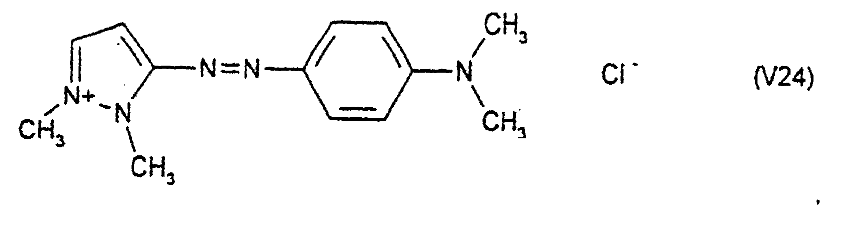

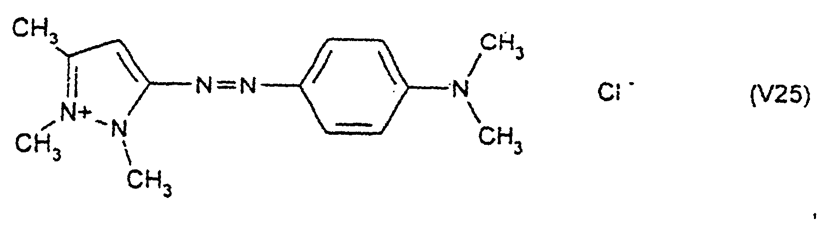

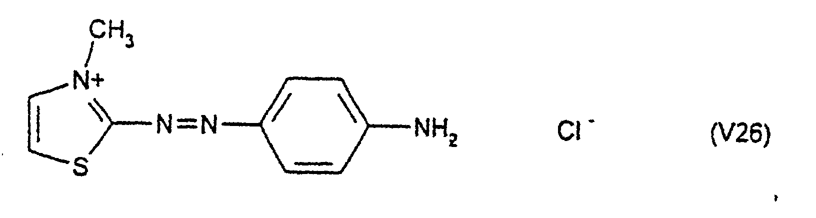

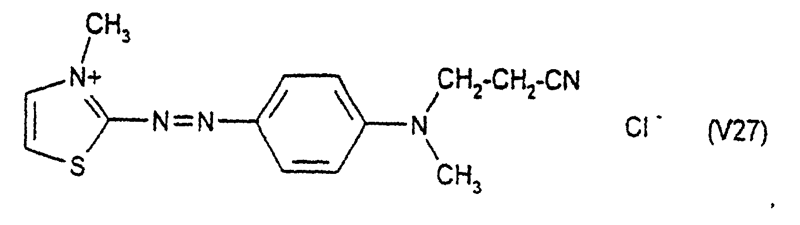

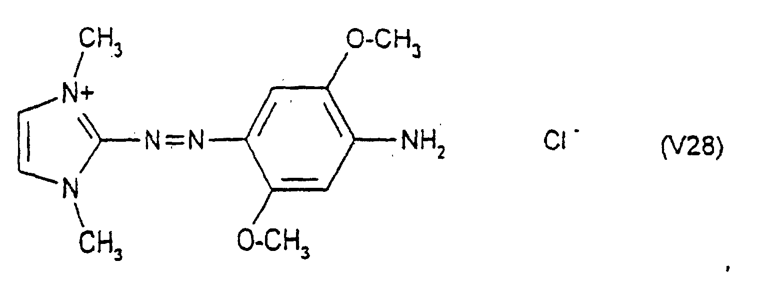

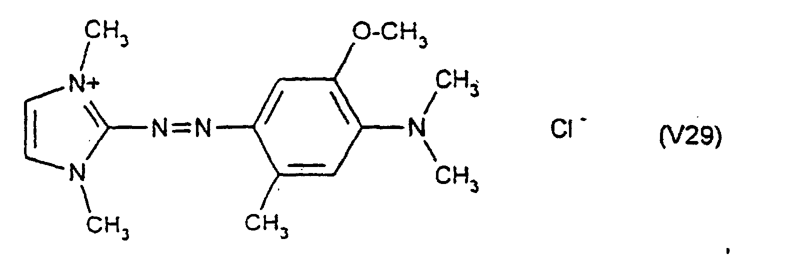

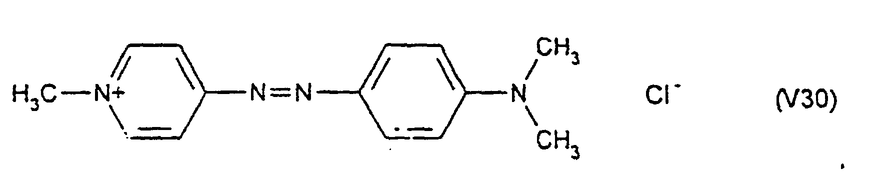

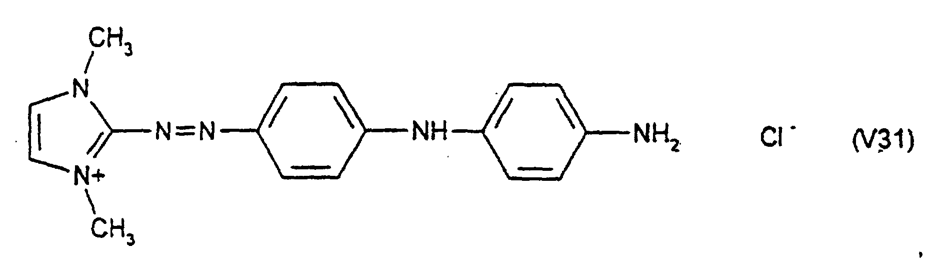

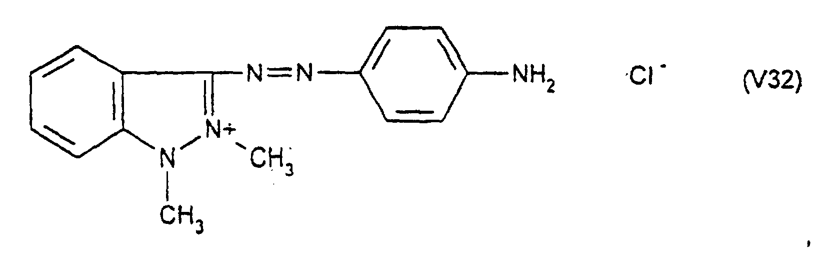

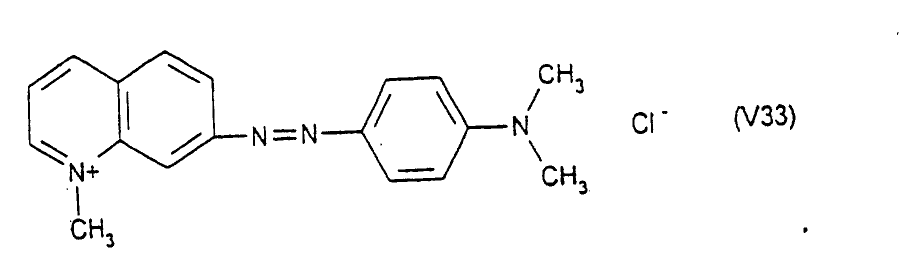

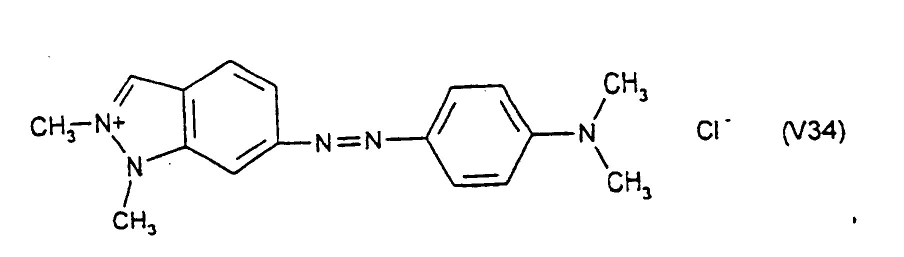

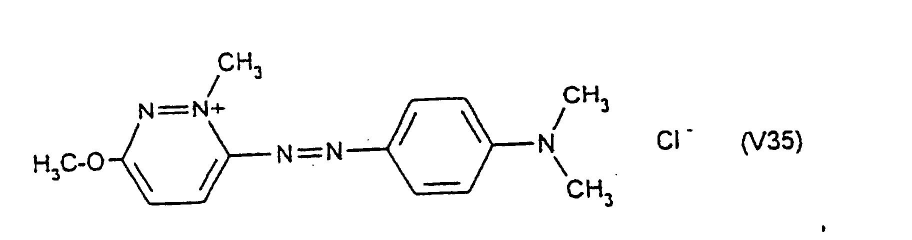

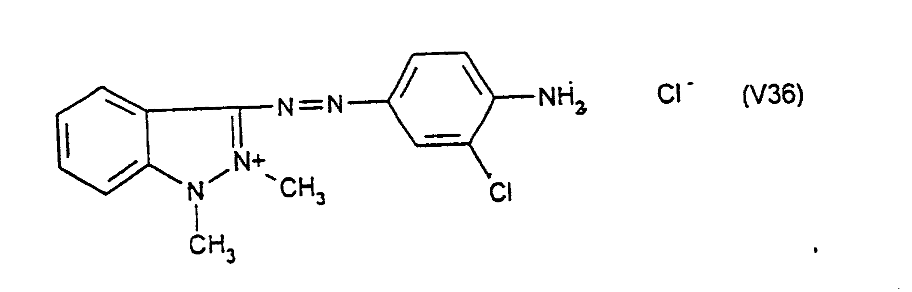

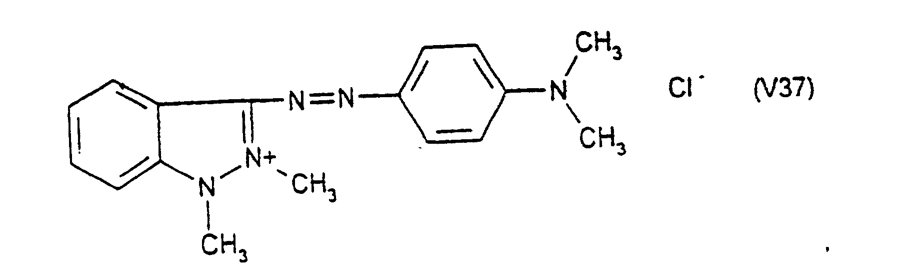

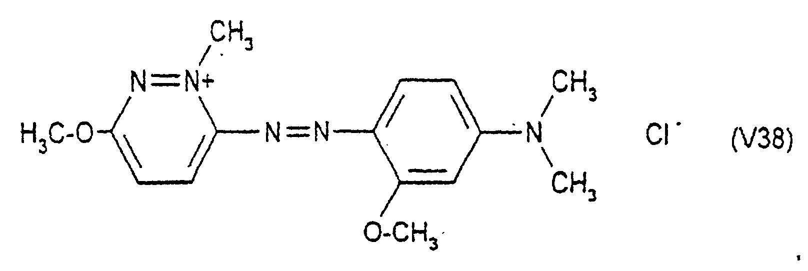

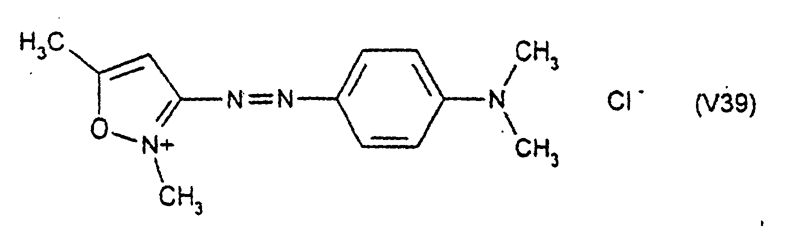

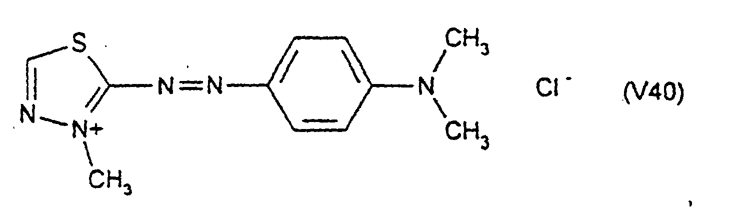

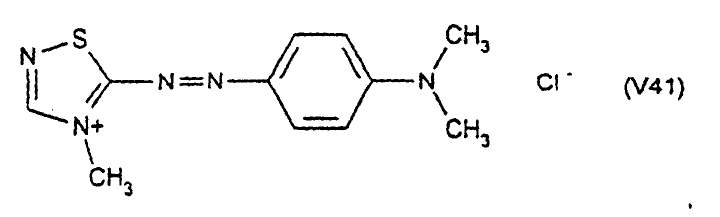

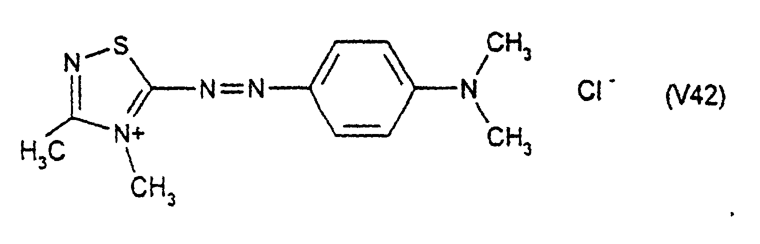

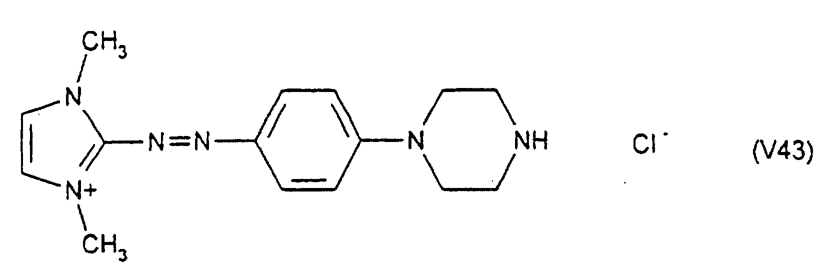

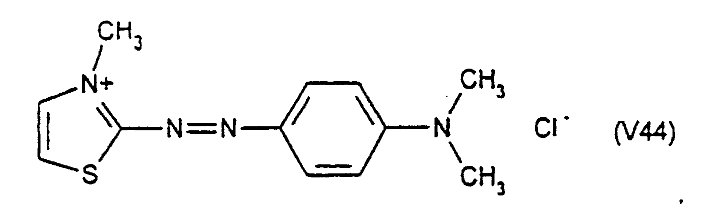

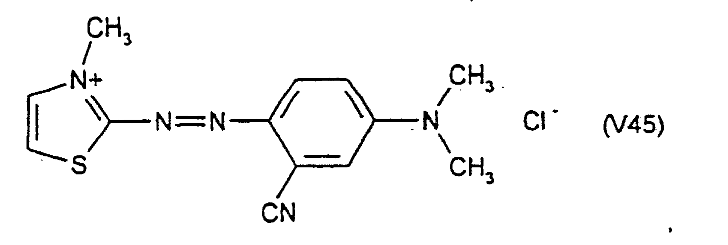

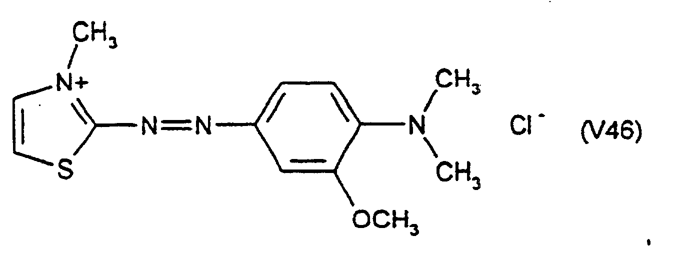

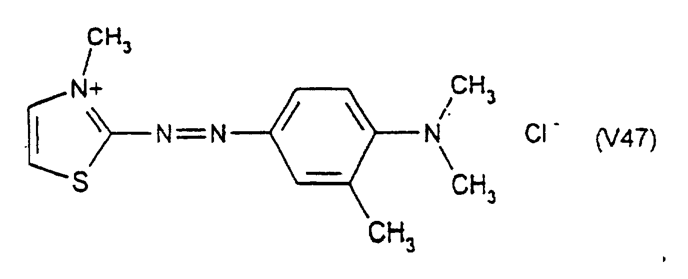

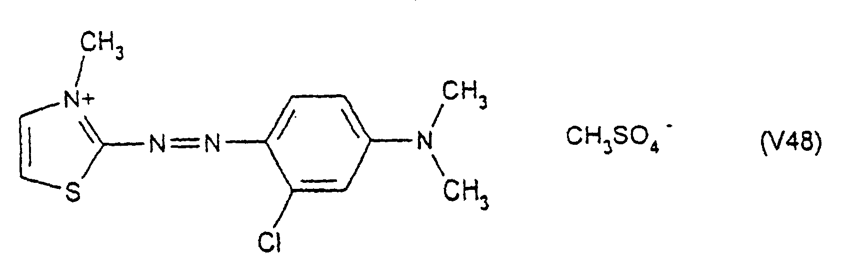

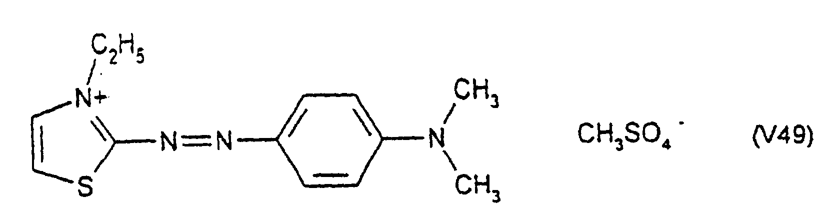

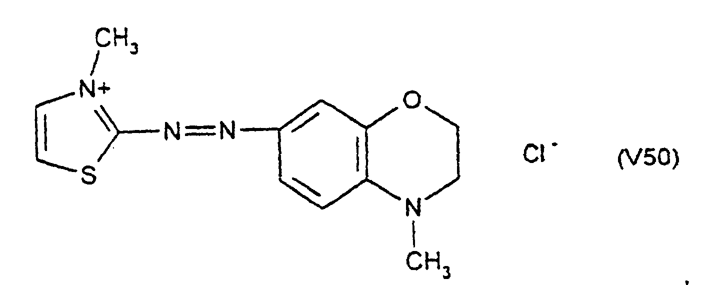

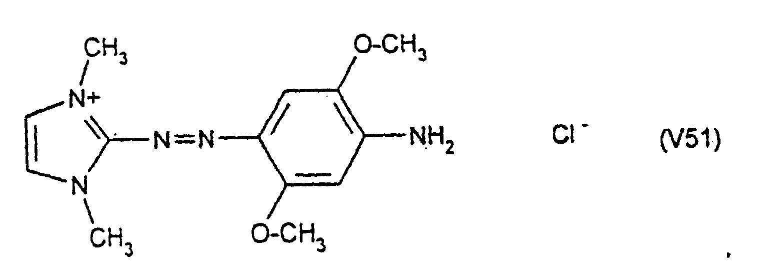

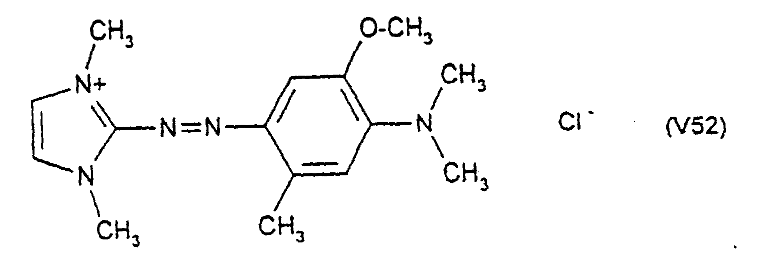

24. Zusammensetzung nach Anspruch 22, **dadurch gekennzeichnet, dass** die kationischen Direktfarbstoffe der Formel (VI) unter den Verbindungen der folgenden Strukturen (VI1) bis (VI12) ausgewählt sind:

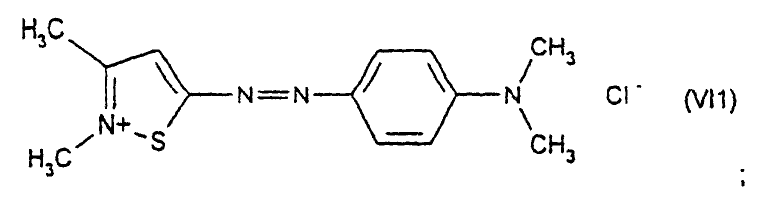

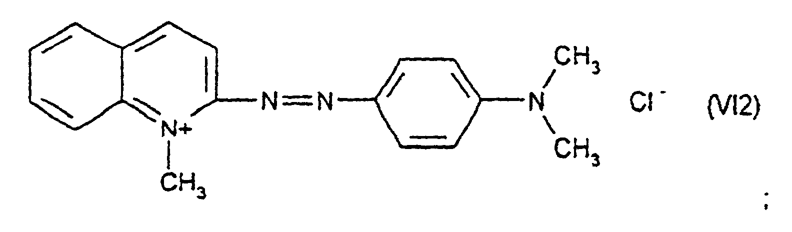

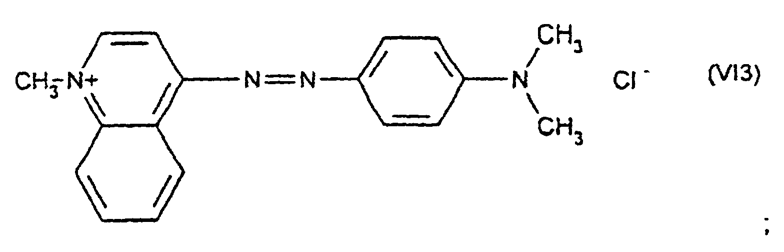

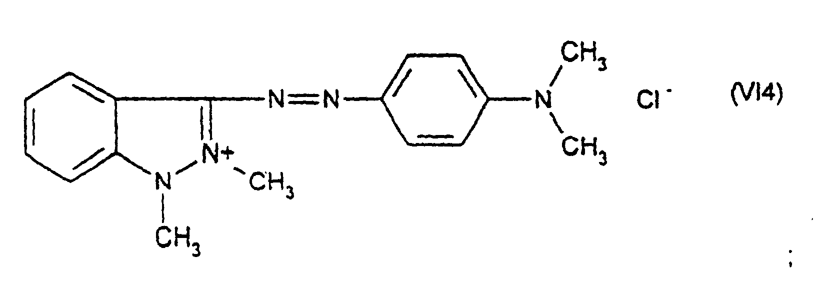

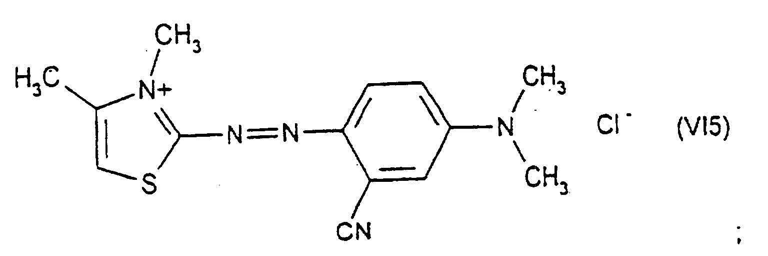

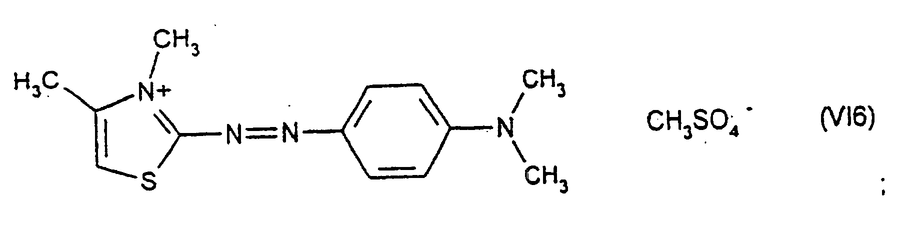

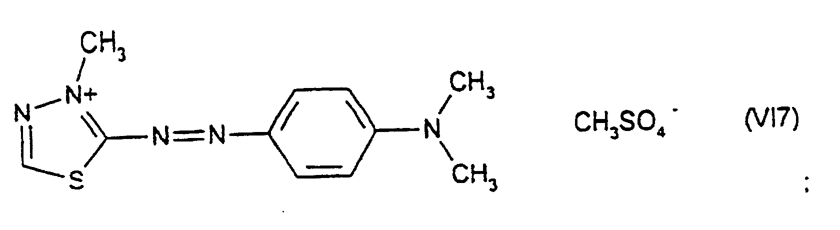

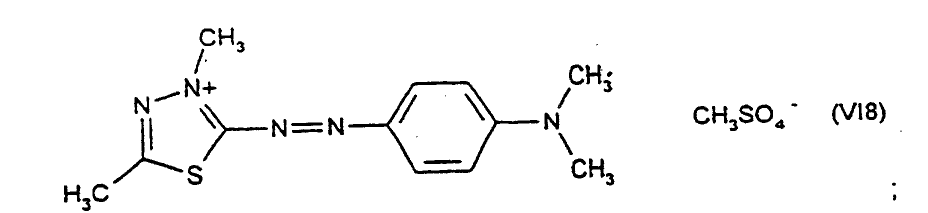

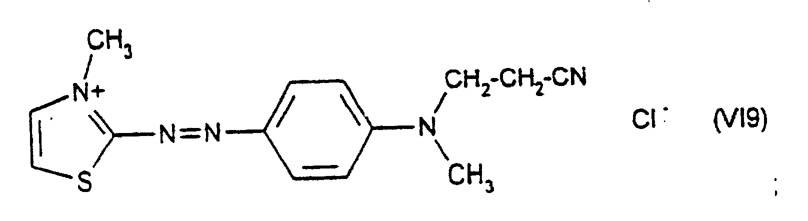

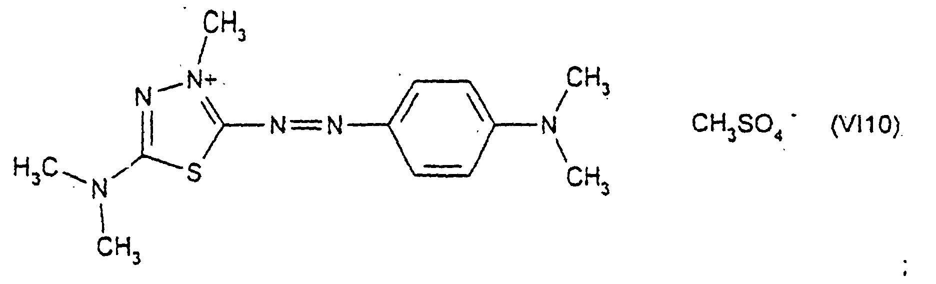

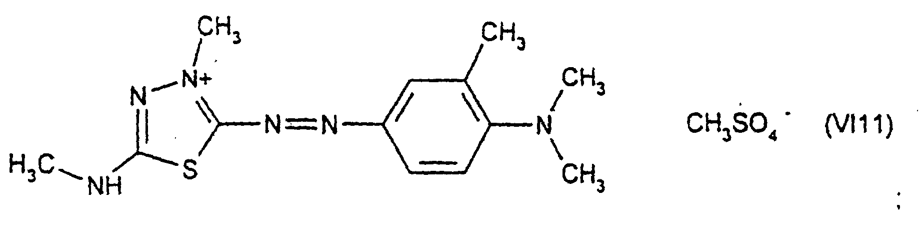

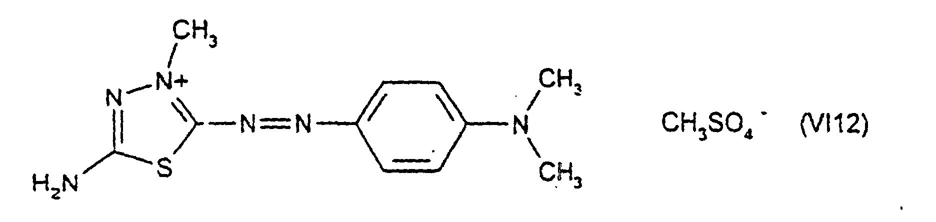

25. Zusammensetzung nach Anspruch 22, **dadurch gekennzeichnet, dass** die kationischen Direktfarbstoffe der Formel (VII) unter den Verbindungen der folgenden Strukturen (VII1) bis (VII18) ausgewählt sind:

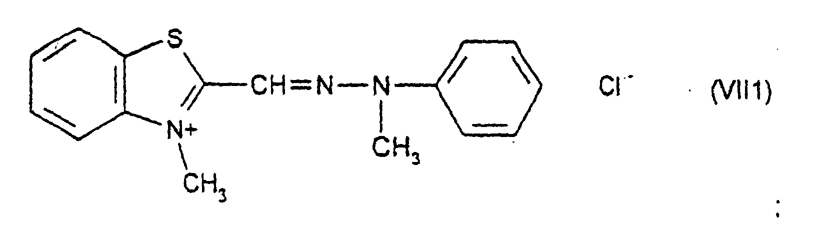

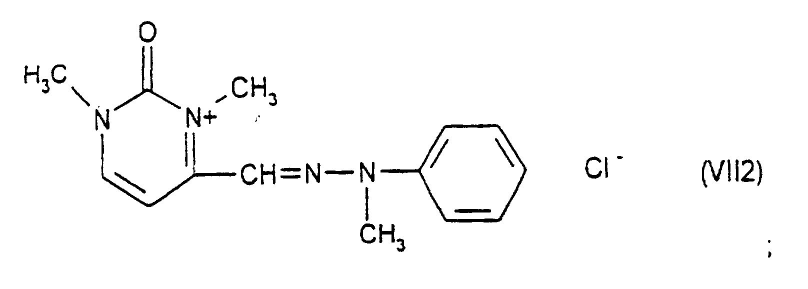

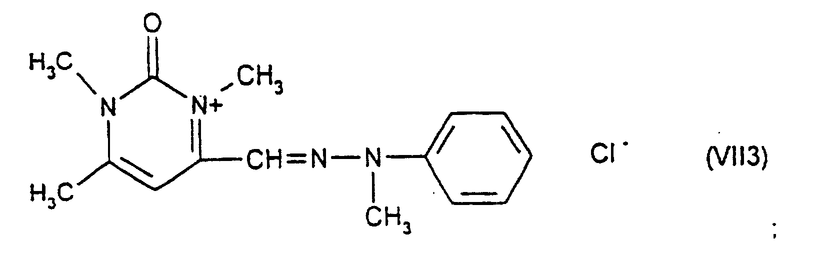

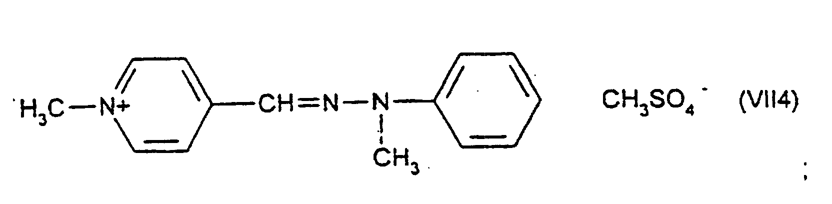

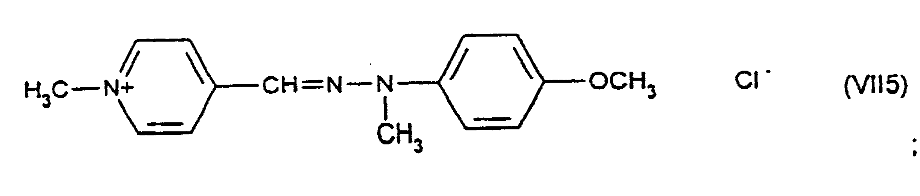

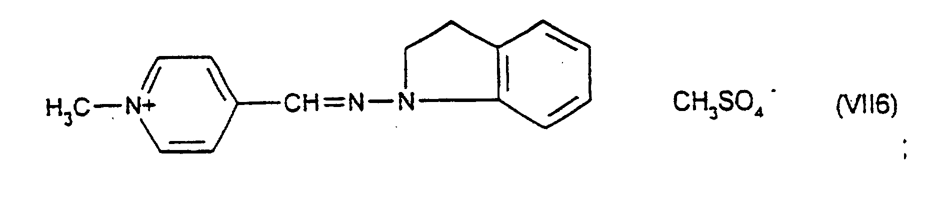

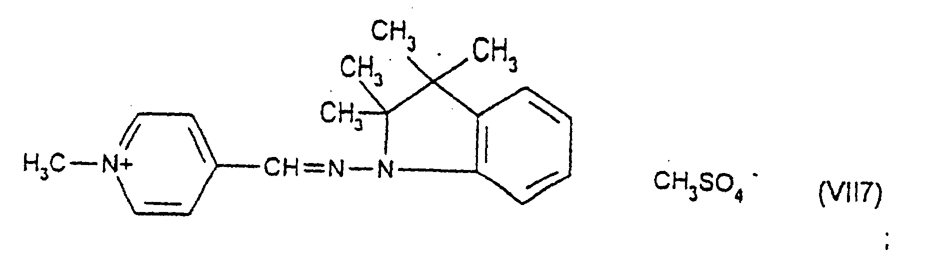

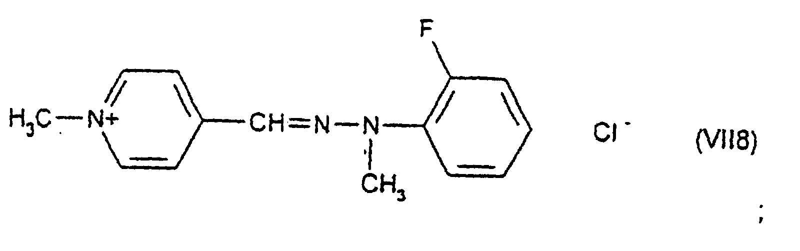

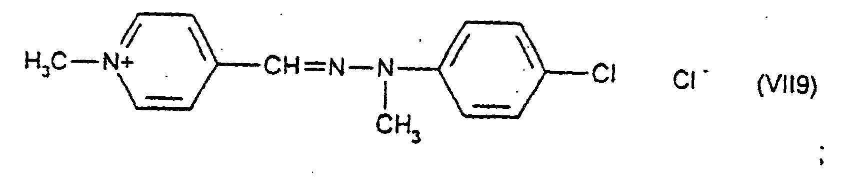

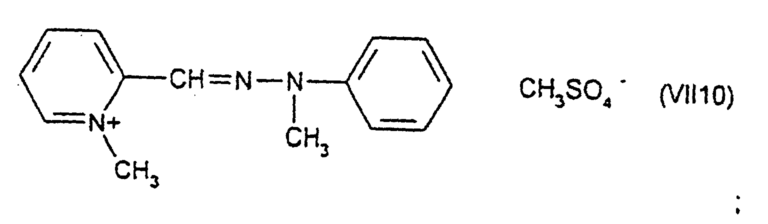

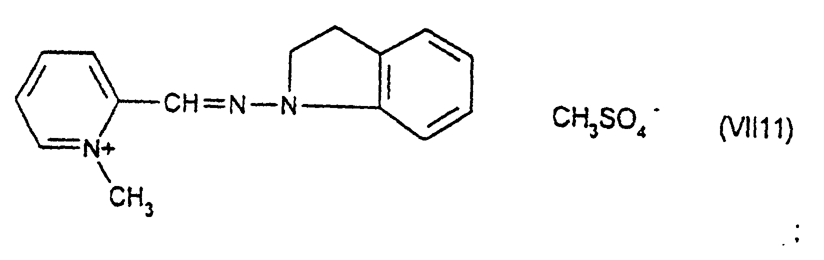

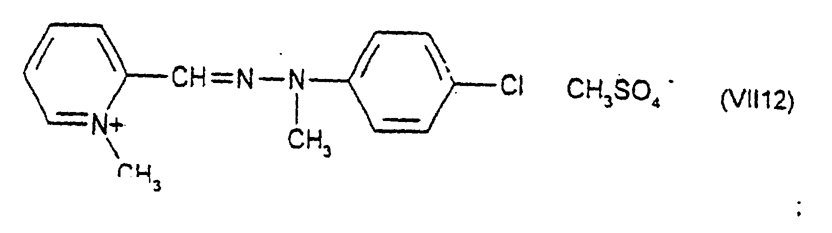

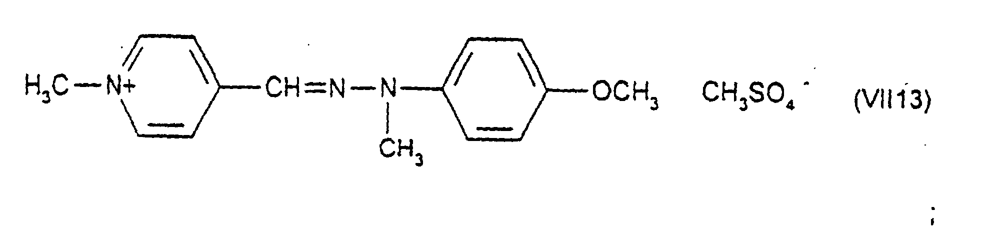

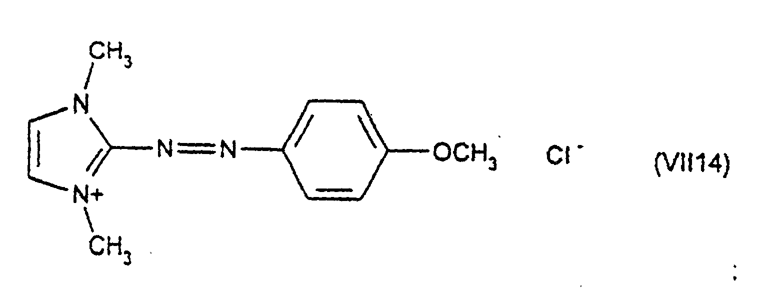

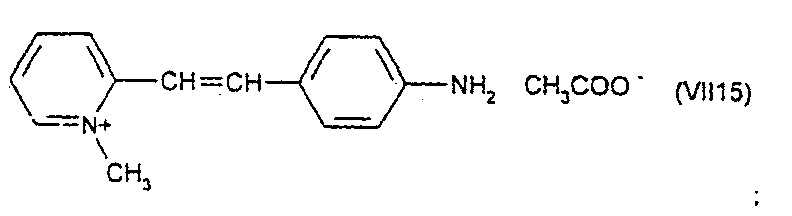

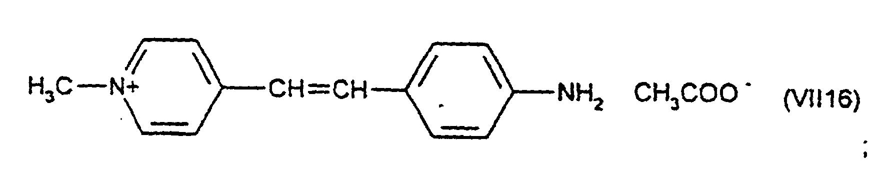

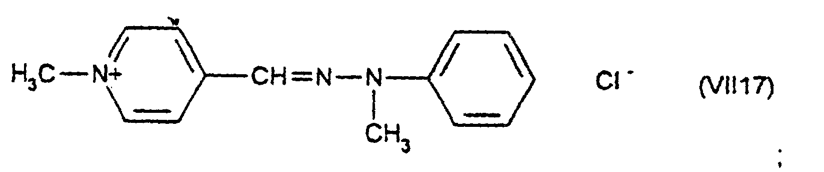

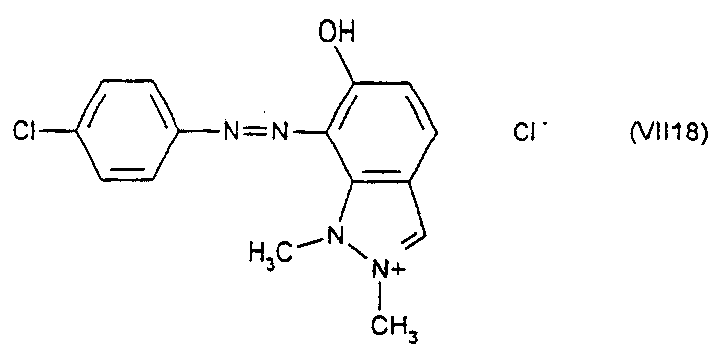

26. Zusammensetzung nach Anspruch 22, **dadurch gekennzeichnet, dass** die kationischen Direktfarbstoffe der Formel (VII') unter den Verbindungen der folgenden Strukturen (VII'1) bis (VII'3) ausgewählt sind:

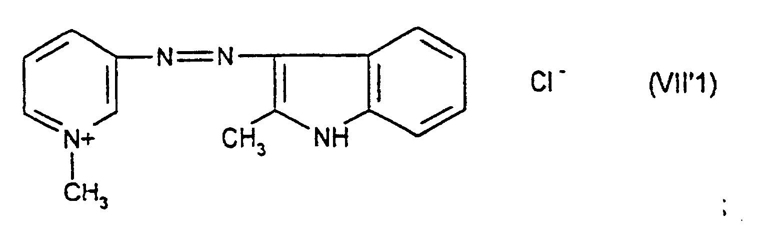

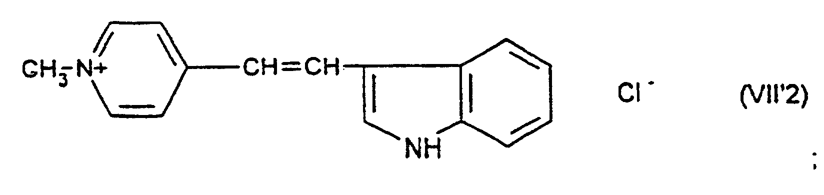

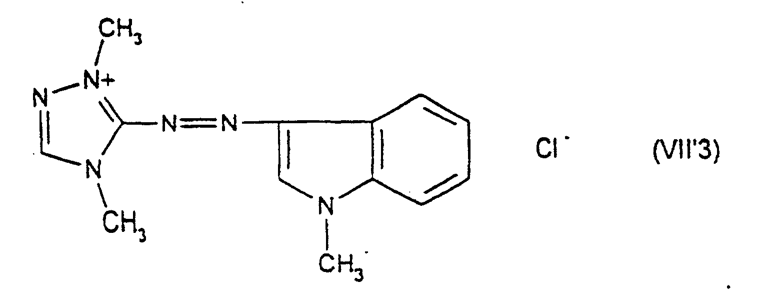

27. Zusammensetzung nach einem der vorhergehenden Ansprüche, **dadurch gekennzeichnet, dass** der oder die kationischen Direktfarbstoffe 0,001 bis 10 Gew.-% des Gesamtgewichts der gebrauchsfertigen Farbmittelzusammensetzung ausmachen.

28. Zusammensetzung nach Anspruch 27, **dadurch gekennzeichnet, dass** der oder die kationischen Direktfarbstoffe 0,05 bis 5 Gew.-% des Gesamtgewichts der gebrauchsfertigen Farbmittelzusammensetzung ausmachen.

29. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

30. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

31. Zusammensetzung nach einem der vorhergehenden Ansprüche, **dadurch gekennzeichnet, dass** sie einen pH-Wert im Bereich von 5 bis 11 aufweist.

32. Zusammensetzung nach einem der vorhergehenden Ansprüche, **dadurch gekennzeichnet, dass** sie mindestens eine Peroxidase enthält.

33. Verfahren zum Färben von Keratinfasern und insbesondere menschlichen Keratinfasern, wie dem Haar, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der vorhergehenden Ansprüche aufgebracht wird.

34. Verfahren nach Anspruch 33, **dadurch gekennzeichnet, dass** es einen vorbereitenden Schritt umfasst, der darin besteht, einerseits eine Zusammensetzung (A), die in einem zum Färben geeigneten Medium mindestens eine Oxidationsbase und mindestens einen kationischen Direktfarbstoff enthält, und andererseits eine Zusammensetzung (B) getrennt voneinander aufzubewahren, die in einem zum Färben geeigneten Medium mindestens ein Enzym vom Typ Oxidoreduktase (2 Elektronen) in Gegenwart mindestens eines Donors für das Enzym enthält, und diese bei der Anwendung zu vermischen, bevor das Gemisch auf die Fasern aufgebracht wird.

35. Vorrichtung mit mehreren Abteilungen oder "Kit" zum Färben, **dadurch gekennzeichnet, dass** sie eine erste Abteilung mit der in Anspruch 34 definierten Zusammensetzung (A) und eine zweite Abteilung mit der in Anspruch 34 definierten Zusammensetzung (B) enthält.

## Claims

1. Ready-to-use composition for the oxidation dyeing of keratin fibres, and in particular human keratin fibres such as the hair, **characterized in that** it comprises, in a medium which is suitable for dyeing:
- at least one oxidation base,
- at least cationic direct dye,
- at least one enzyme of 2-electron oxidoreductase type, and
- at least one donor for the said enzyme.

2. Composition according to Claim 1, **characterized in that** the 2-electron oxidoreductase is chosen from pyranose oxidases, glucose oxidases, glycerol oxidases, lactate oxidases, pyruvate oxidases and uricases.

3. Composition according to either of Claims 1 and 2, **characterized in that** the 2-electron oxidoreductase is chosen from uricases of animal, microbiological or biotechnological origin.

4. Composition according to any one of the preceding claims, **characterized in that** the 2-electron oxidoreductase(s) represent(s) from 0.01 to 20% by weight relative to the total weight of the ready-to-use dye composition.

5. Composition according to Claim 4, **characterized in that** the 2-electron oxidoreductase(s) represent(s) from 0.1 to 5% by weight relative to the total weight of the ready-to-use dye composition.

6. Composition according to Claim 3, **characterized in that** the donor (or substrate) for the said 2-electron oxidoreductase is chosen from uric acid and its salts.

7. Composition according to any one of the preceding claims, **characterized in that** the donor(s) represent(s) from 0.01 to 20% by weight relative to the total weight of the ready-to-use dye composition.

8. Composition according to Claim 7, **characterized in that** the donor(s) represent(s) from 0.1 to 5% by weight relative to the total weight of the ready-to-use dye composition.

9. Composition according to any one of the preceding claims, **characterized in that** the oxidation base(s) is (are) chosen from para-phenylenediamines, double bases, para-aminophenols, ortho-aminophenols and heterocyclic oxidation bases.

10. Composition according to Claim 9, **characterized in that** the para-phenylenediamines are chosen from the compounds of formula (I) below, and the addition salts thereof with an acid: 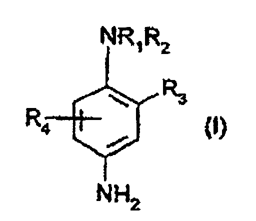 in which:
- R₁ represents a hydrogen atom, a C₁-C₄ alkyl radical, a C₁-C₄ monohydroxyalkyl radical, a C₂-C₄ polyhydroxyalkyl radical, a (C₁-C₄)-alkoxy(C₁-C₄)alkyl radical, a C₁-C₄ alkyl radical substituted with a nitrogenous group, a phenyl radical or a 4'-aminophenyl radical;
- R₂ represents a hydrogen atom, a C₁-C₄ alkyl radical, a C₁-C₄ monohydroxyalkyl radical, a C₂-C₄ polyhydroxyalkyl radical, a (C₁-C₄)-alkoxy(C₁-C₄)alkyl radical or a C₁-C₄ alkyl radical substituted with a nitrogenous group;
- R₃ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxy-alkoxy radical, an acetylamino(C₁-C₄)alkoxy radical, a C₁-C₄ mesylaminoalkoxy radical or a carbamoylamino(C₁-C₄)alkoxy radical,
- R₄ represents a hydrogen or halogen atom or a C₁-C₄ alkyl radical.

11. Composition according to Claim 10, **characterized in that** the para-phenylenediamines of formula (I) are chosen from para-phenylenediamine, paratoluylenediamine, 2-chloro-para-phenylenediamine, 2,3-dimethyl-para-phenylenediamine, 2,6-dimethyl-para-phenylenediamine, 2,6-diethyl-para-phenylene-diamine, 2,5-dimethyl-para-phenylenediamine, N,N-dimethyl-para-phenylenediamine, N,N-diethyl-para-phenylenediamine, N,N-dipropyl-paraphenylenediamine, 4-amino-N,N-diethyl-3-methylaniline, N,N-bis(β-hydroxyethyl)-para-phenylene-diamine, 4-[N,N-bis(β-hydroxyethyl)amino]-2-methylaniline, 4- [N, N-bis(β-hydroxyethyl)amino]2-chloroaniline, 2-β-hydroxyethyl-paraphenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N- (4'-aminophenyl)-para-phenylenediamine, N-phenyl-paraphenylenediamine, 2-β-hydroxyethyloxy-paraphenylenediamine, 2-β-acetylaminoethyloxy-paraphenylenediamine and N-(β-methoxyethyl)-paraphenylenediamine, and the addition salts thereof with an acid.

12. Composition according to Claim 9, **characterized in that** the double bases are chosen from the compounds of formula (II), and the addition salts thereof with an acid: 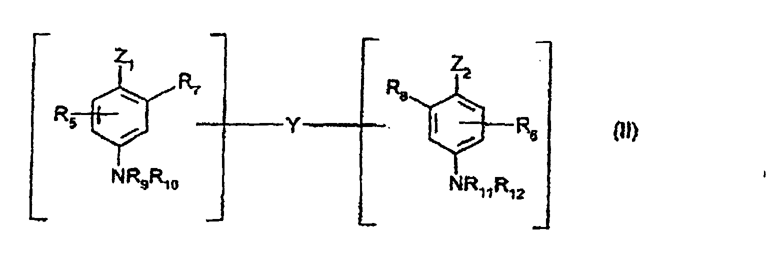 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more hetero atoms such as oxygen, sulphur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ mono-hydroxyalkyi radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical;
it being understood that the compounds of formula (II) contain only one linker arm Y per molecule.

13. Composition according to Claim 12, **characterized in that** the double bases of formula (II) are chosen from N,N'-bis(β-hydroxyethyl)-N,N'-bis(4'-aminophenyl)-1,3-diaminopropanol, N,N'-bis(β-hydroxy-ethyl)-N, N'-bis(4'-aminophenyl)ethylenediamine, N,N'-bis(4-aminophenyl)tetramethylenediamine, N,N'-bis(β-hydroxyethyl)-N,N'-bis(4-aminophenyl)-tetramethylenediamine, N,N'-bis(4-methylaminophenyl)tetramethylenediamine, N,N'-bis(ethyl) -N,N'-bis(4'-amino-3'-methylphenyl)ethylenediamine and 1,8-bis(2,5-diaminophenoxy)-3,5-dioxaoctane, and the addition salts thereof with an acid.

14. Composition according to Claim 9, **characterized in that** the para-aminophenols are chosen from the compounds of formula (III), and the addition salts thereof with an acid 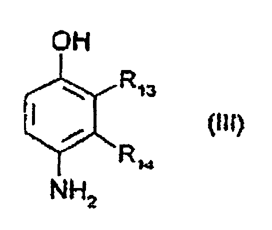 in which:
- R₁₃ represents a hydrogen or halogen atom or a C₁-C₄ alkyl, C₁-C₄ monohydroxyalkyl, (C₁-C₄)-alkoxy(C₁-C₄)alkyl, C₁-C₄ aminoalkyl or hydroxy (C₁-C₄) alkylamino (C₁-C₄) alkyl radical,
- R₁₄ represents a hydrogen or halogen atom or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy-(C₁-C₄)alkyl radical,
it being understood that at least one of the radicals R₁₃ or R₁₄ represents a hydrogen atom.

15. Composition according to Claim 14, **characterized in that** the para-aminophenols of formula (III) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

16. composition according to Claim 9, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

17. Composition according to Claim 9, **characterized in that** the heterocyclic oxidation bases are chosen from pyridine derivatives, pyrimidine derivatives, pyrazole derivatives and pyrazolopyrimidine derivatives, and the addition salts thereof with an acid.

18. Composition according to any one of the preceding claims, **characterized in that** the oxidation base(s) represent(s) from 0.0005 to 12% by weight relative to the total weight of the dye composition.

19. Composition according to Claim 18, **characterized**
**in that** the oxidation bass(s) represent(s) from 0.005 to 6% by weight relative to the total weight of the dye composition.

20. Composition according to any one of the preceding claims, **characterized in that** the cationic direct dye(s) is (are) chosen from cationic aminoanthraquinone dyes, cationic monoazo or diazo dyes and cationic naphthoquinone dyes.

21. Composition according to Claim 20, **characterized in that** the cationic direct dye(s) is (are) chosen from [8- [(p-aminophenyl)azol]-7-hydroxy-2-naphthyl]trimethylammonium chloride, 3-[(4-amino-6-bromo-5,8-dihydro-1-hydroxy-8-imino-5-oxo-2-naphthalenyl)amino]-N,N,N-trimethylbenzenaminium chloride, 7-hydroxy-8-[(2-methoxyphenyl)azo]-N,N,N-trimethyl-2-naphthalenaminium chloride, [8-[(4-amino-2-nitrophenyl)azo]-7-hydroxy-2-naphthyl]trimethylammonium chloride and 3-[(4,5-dihydro-3-methyl-5-oxo-1-phenyl-1H-pyrazol-4-yl)-azo]-N,N,N-trimethylbenzenaminium chloride.

22. Composition according to any one of Claims 1 to 19, **characterized in that** the cationic direct dye(s) is (are) chosen from:
a) the compounds of formula (V) below: 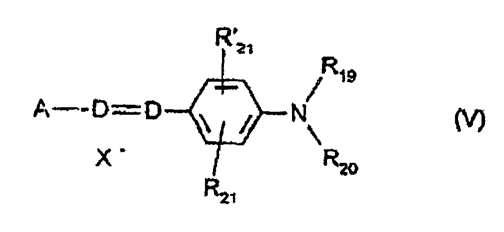 in which:
D represents a nitrogen atom or a -CH group,
R₁₉ and R₂₀, which may be identical or different, represent a hydrogen atom; a C₁-C₄ alkyl radical which can be substituted with a -CN, -OH or -NH₂ radical or form, with a carbon atom of the benzene ring, an optionally oxygenated or nitrogenous heterocycle, which can be substituted with one or more C₁-C₄ alkyl radicals; a 4'-aminophenyl radical,
R₂₁ and R'₂₁, which may be identical or different, represent a hydrogen atom, a halogen atom chosen from chlorine, bromine, iodine and fluorine, or a cyano, C₁-C₄ alkoxy or acetyloxy radical,
X⁻ represents an anion preferably chosen from chloride, methyl sulphate and acetate,
A represents a group chosen from structures A1 to A19 below: 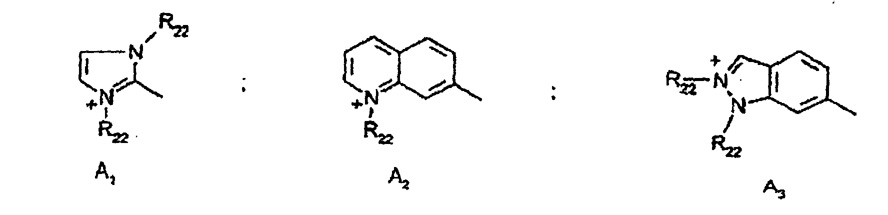 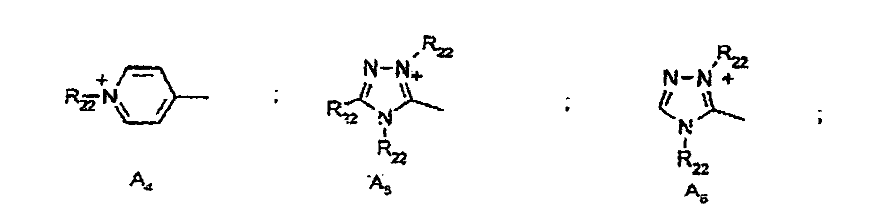 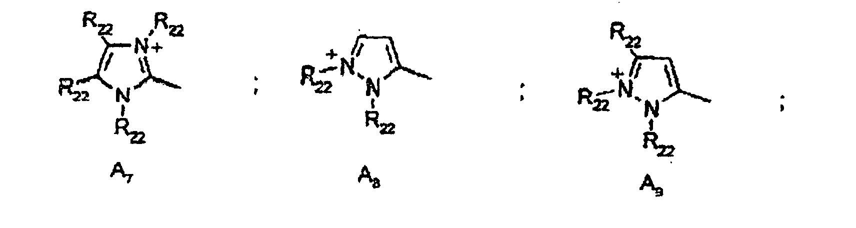 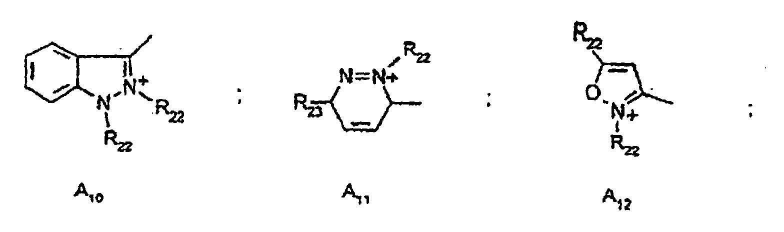 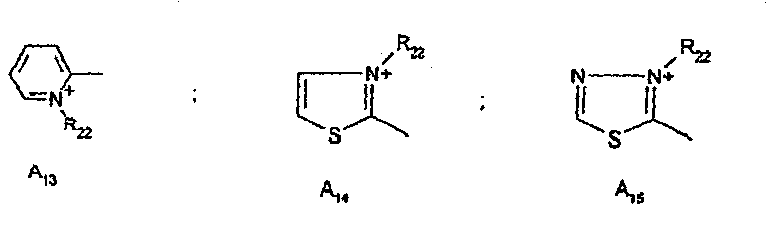 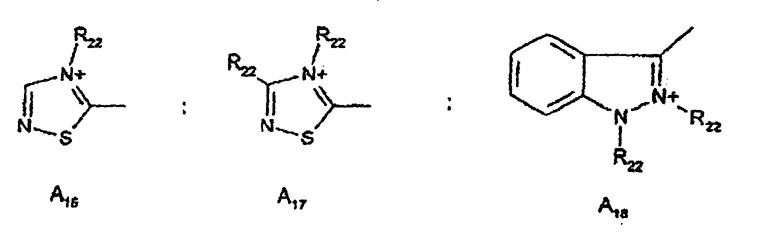 and 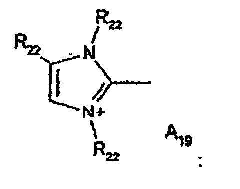 in which R₂₂ represents a C₁-C₄ alkyl radical which can be substituted with a hydroxyl radical and R₂₃ represents a C₁-C₄ alkoxy radical;
b) the compounds of formula (VI) below; 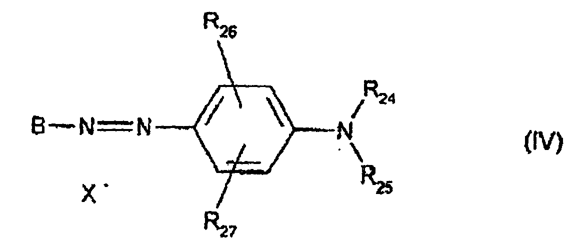 in which:
R₂₄ represents a hydrogen atom or a C₁-C₄ alkyl radical,
R₂₅ represents a hydrogen atom, an alkyl radical which can be substituted with a -CN radical or with an amino group, a 4'-aminophenyl radical or forms, with R₂₄, an optionally oxygenated and/or nitrogenous heterocycle which can be substituted with a C₁-C₄ alkyl radical,
R₂₆ and R₂₇, which may be identical or different, represent a hydrogen atom, a halogen atom such as bromine, chlorine, iodine or fluorine, a C₁-C₄ alkyl or C₁-C₄ alkoxy radical or a -CN radical,
X⁻ represents an anion preferably chosen from chloride, methyl sulphate and acetate,
B represents a group chosen from structures B1 to B6 below: 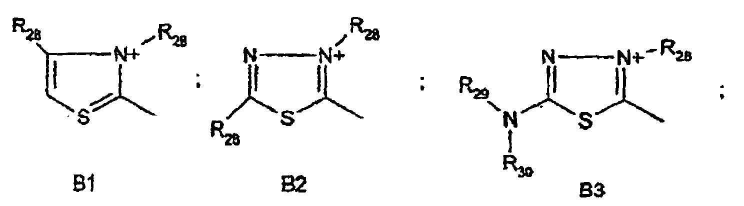 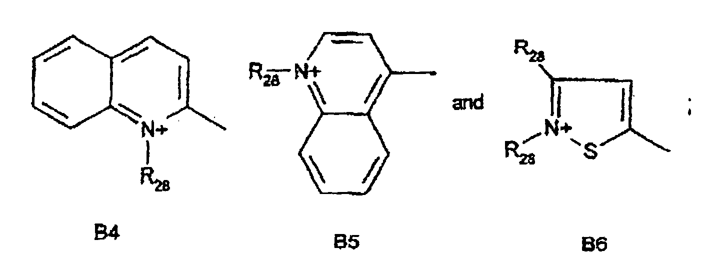 in which R₂₈ represents a C₁-C₄ alkyl radical, R₂₉ and R₃₀, which may be identical or different, represent a hydrogen atom or a C₁-C₄ alkyl radical;
c) the compounds of formulae (VII) and (VII') below: 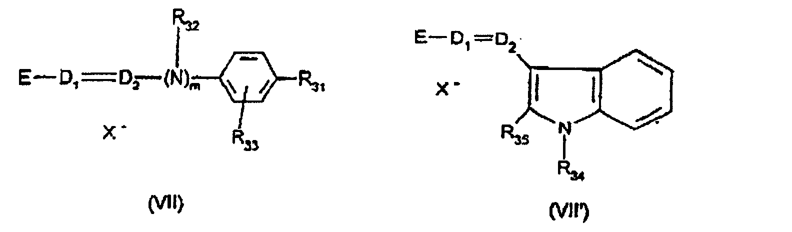 in which:
R₃₁ represents a hydrogen atom, a C₁-C₄ alkoxy radical, a halogen atom such as bromine, chlorine, iodine or fluorine, or an amino radical,
R₃₂ represents a hydrogen atom, a C₁-C₄ alkyl radical or forms, with a carbon atom of the benzene ring, a heterocycle which is optionally oxygenated and/or substituted with one or more C₁-C₄ alkyl groups,
R₃₃ represents a hydrogen atom or a halogen atom such as bromine, chlorine, iodine or fluorine,
R₃₄ and R₃₅, which may be identical or different, represent a hydrogen atom or a C₁-C₄ alkyl radical, D₁ and D₂, which may be identical or different, represent a nitrogen atom or a -CH group,
m = 0 or 1,
it being understood that when R₃₁ represents an unsubstituted amino group, then D₁ and D₂ simultaneously represent a -CH group and m = 0,
X⁻ represents an anion preferably chosen from chloride, methyl sulphate and acetate,
E represents a group chosen from structures E1 to E8 below: 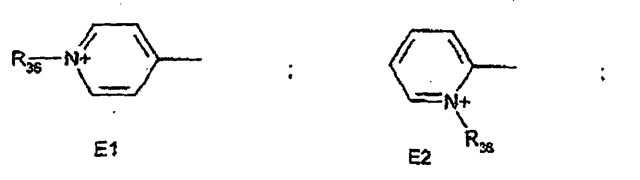 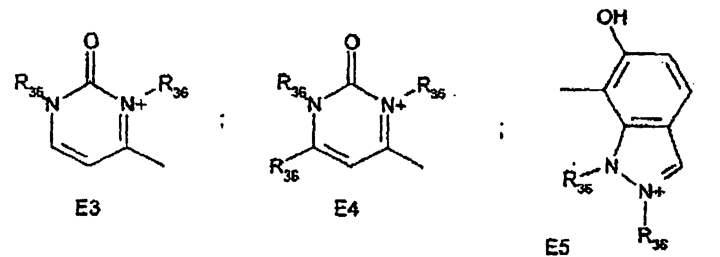 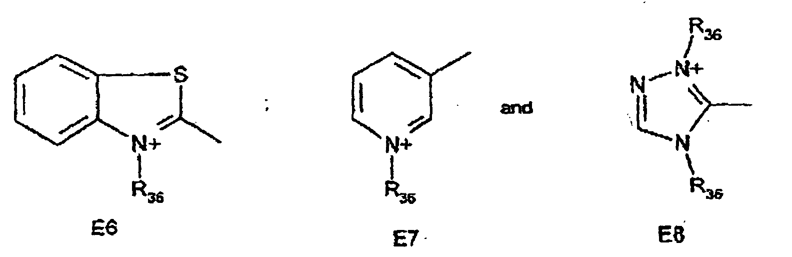
in which R₃₆ represents a C₁-C₄ alkyl radical;
when m = 0 and when D₁ represents a nitrogen atom, then E can also denote a group of structure E9 below: 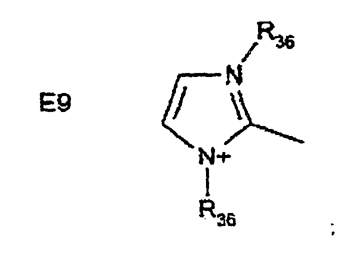 in which R₃₆ represents a C₁-C₄ alkyl radical.

23. Composition according to Claim 22, **characterized in that** the cationic direct dyes of formula (V) are chosen from the compounds corresponding to structures (VI) to (V52) below: 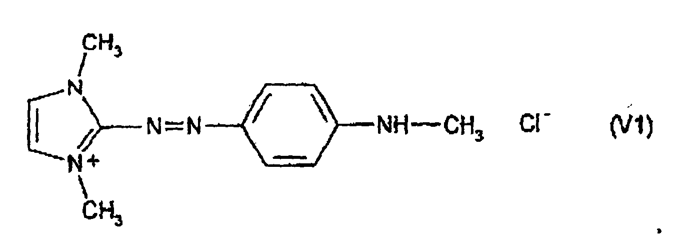 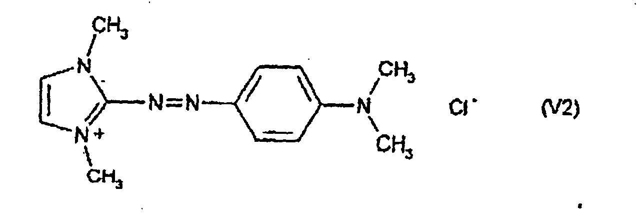 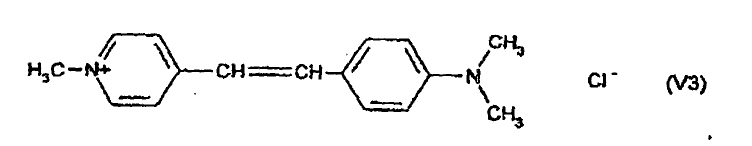 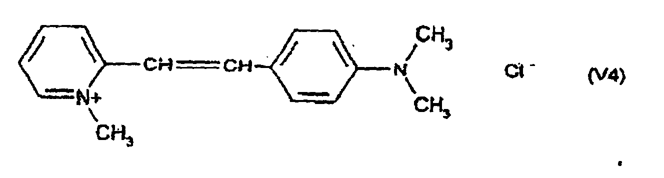 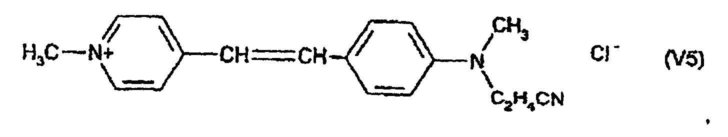 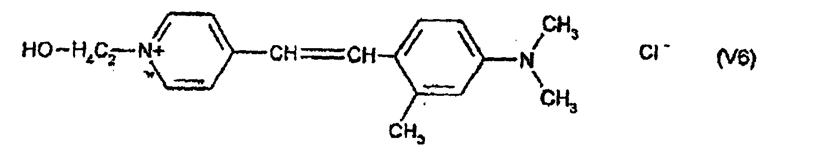 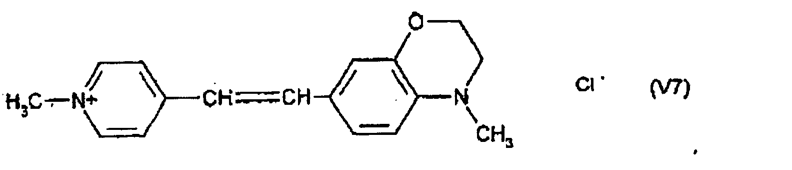 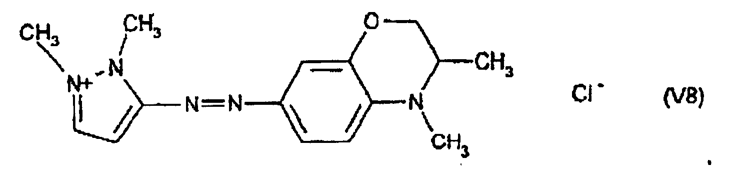 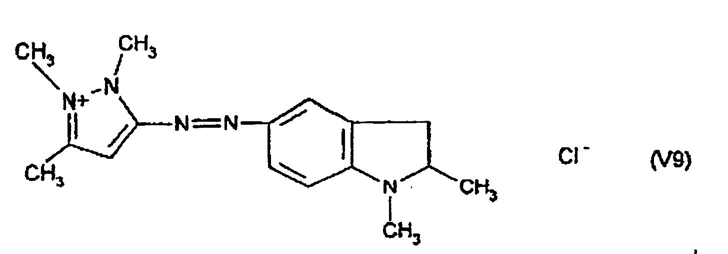 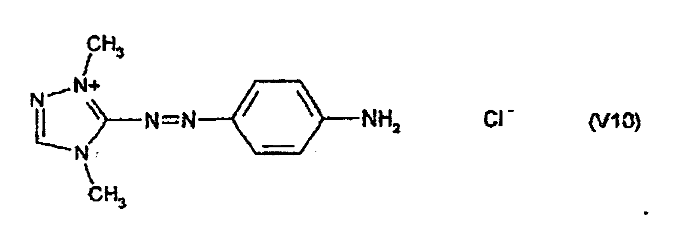 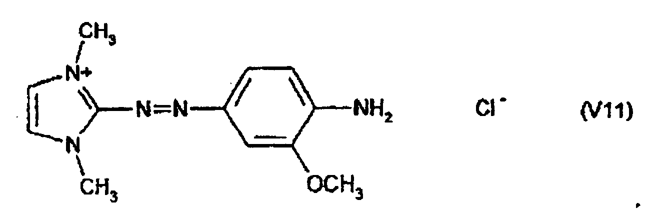 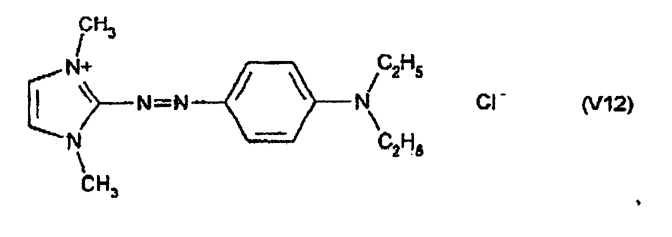 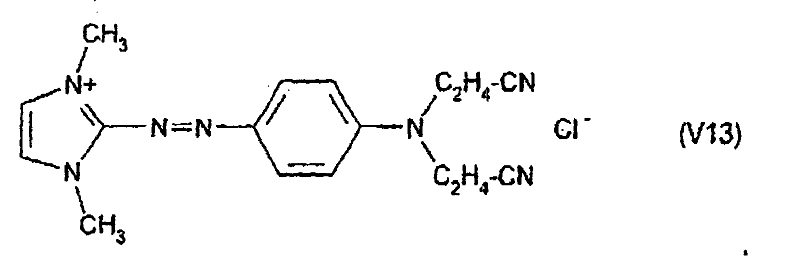 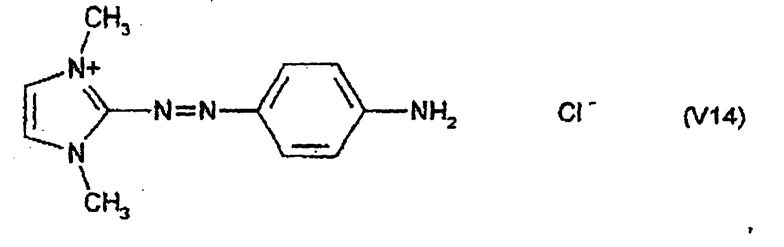 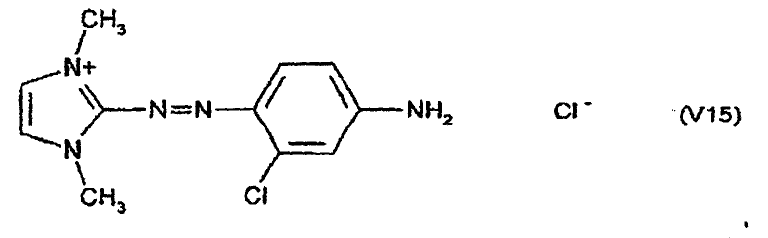 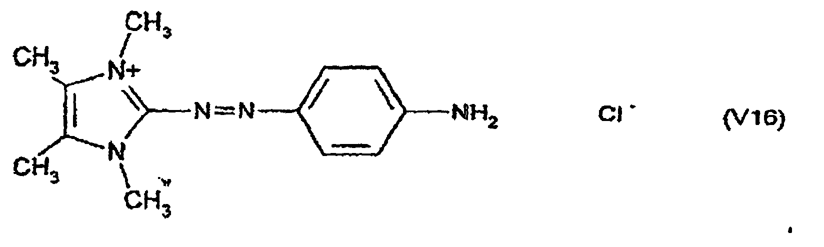 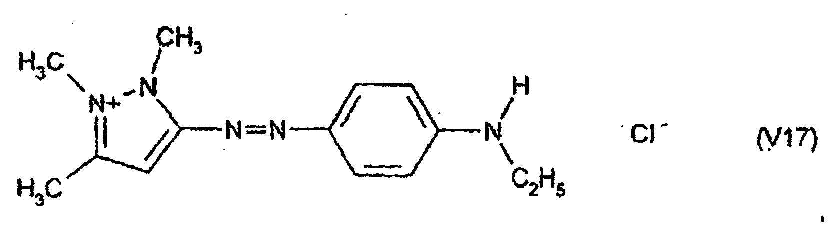 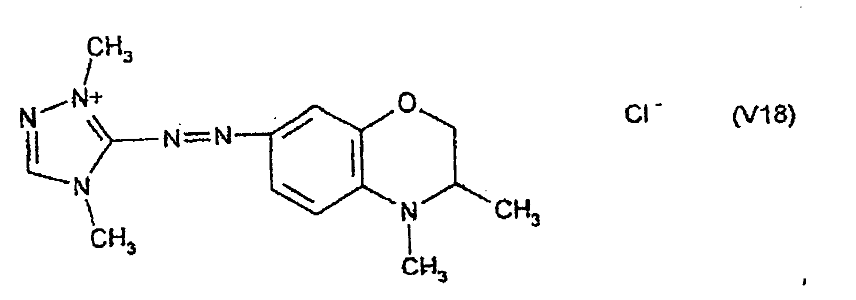 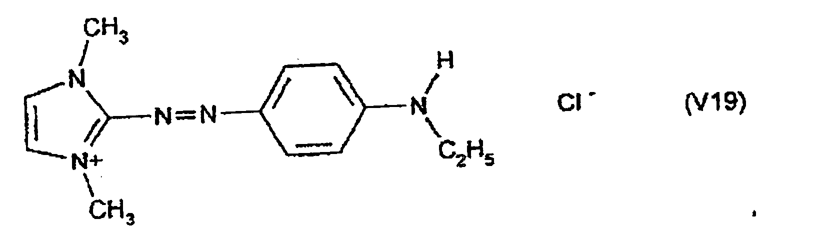 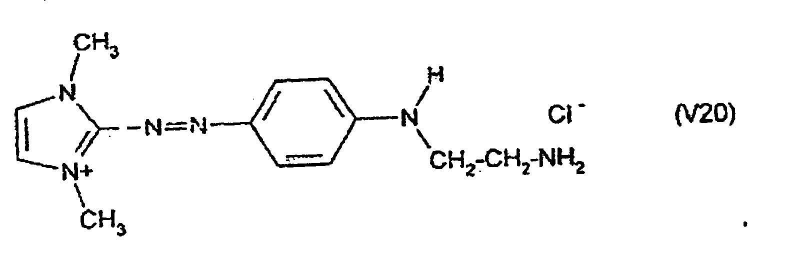 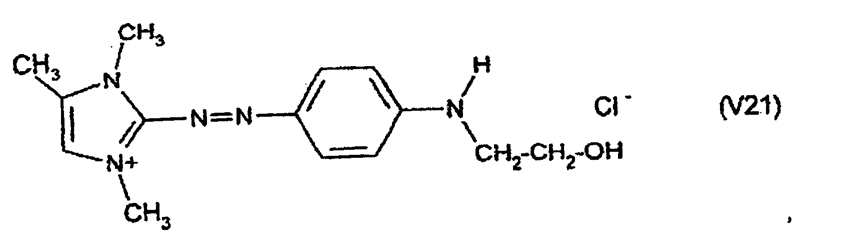 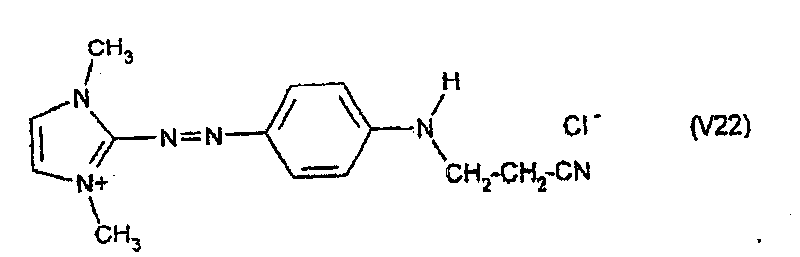 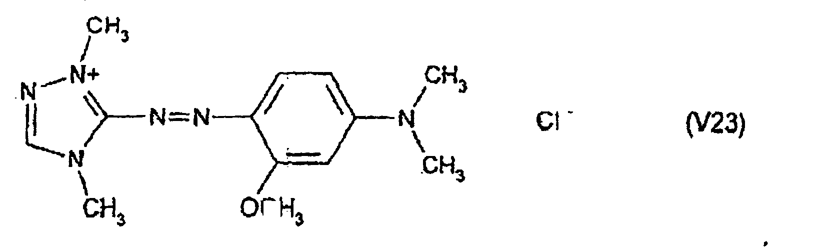 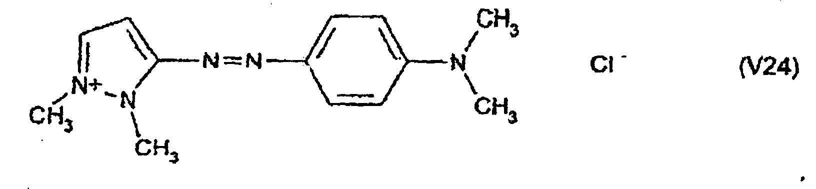 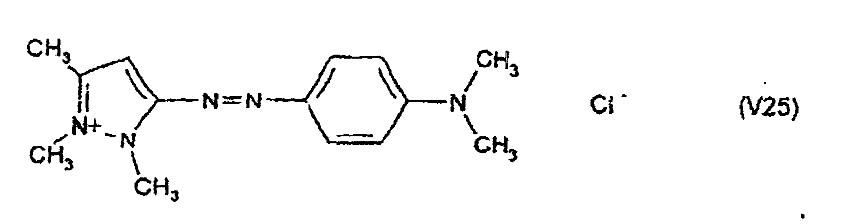 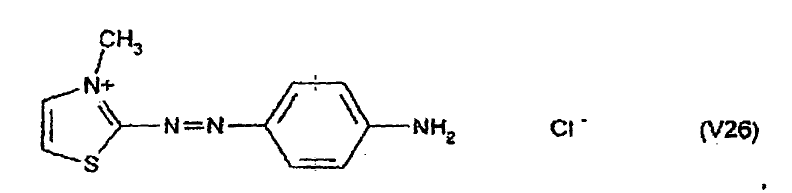 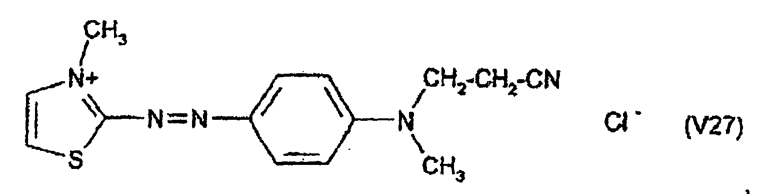 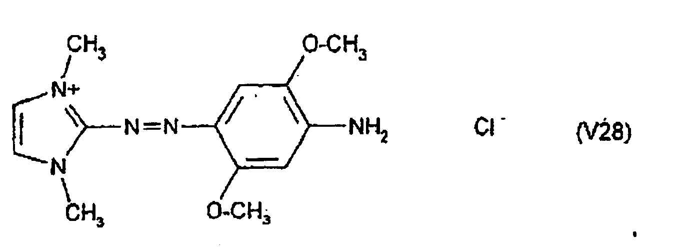 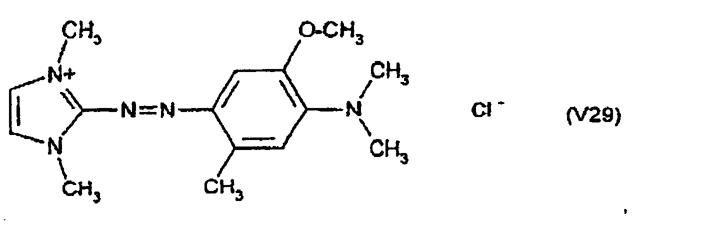 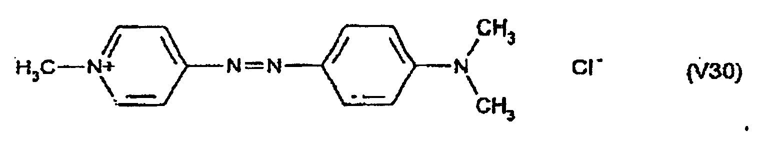 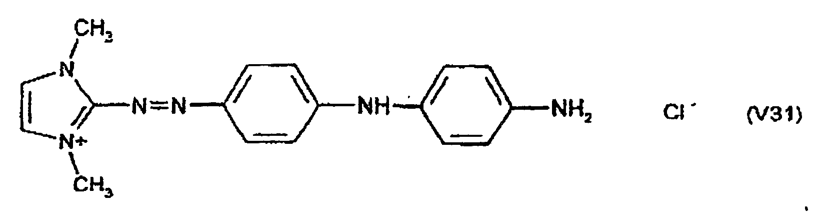 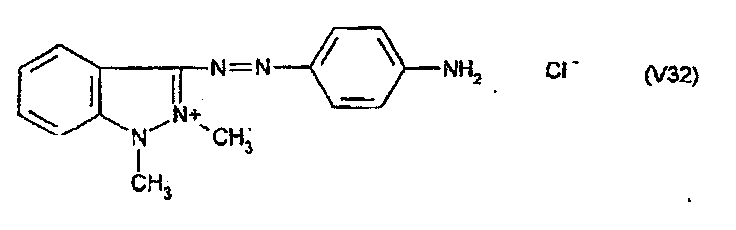 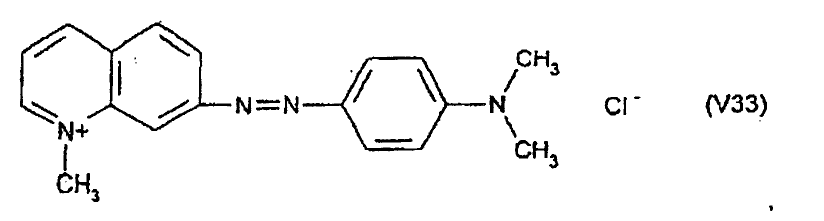 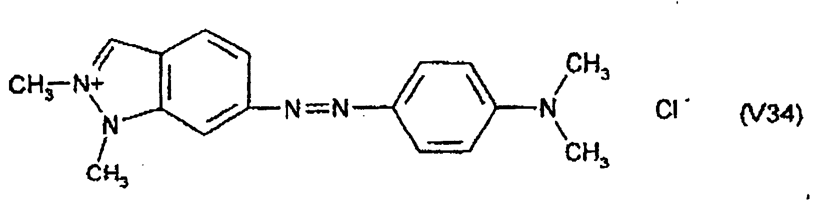 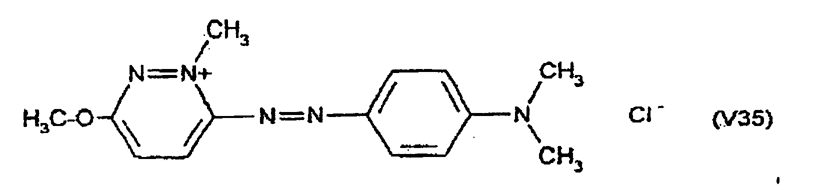 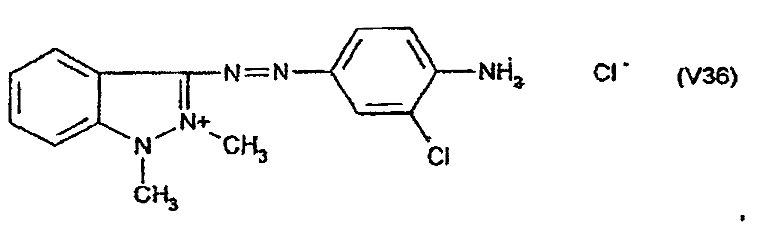 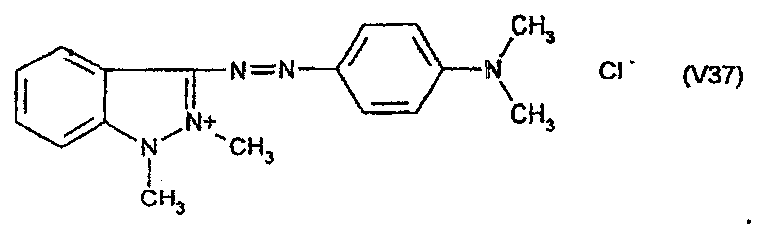 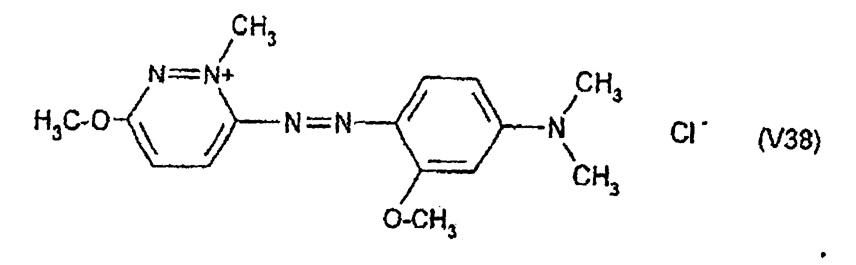 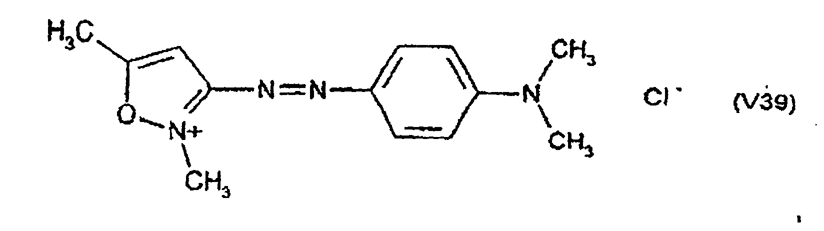 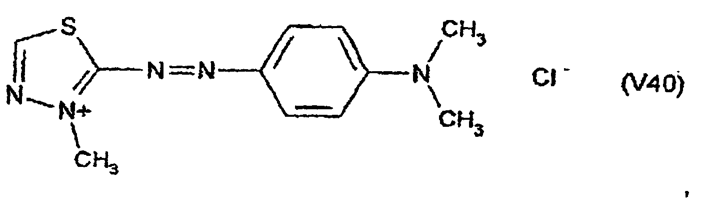 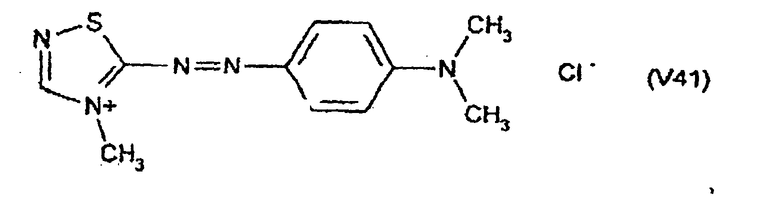 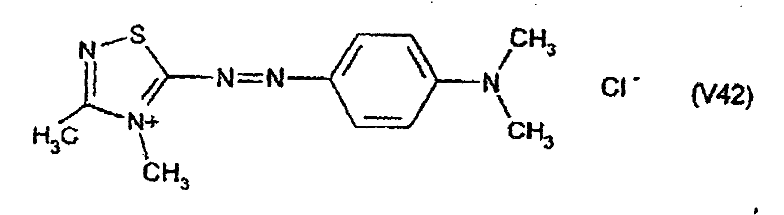 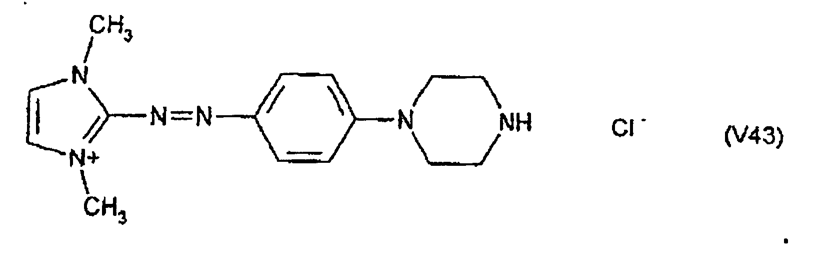 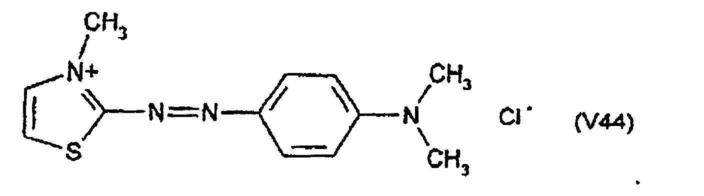 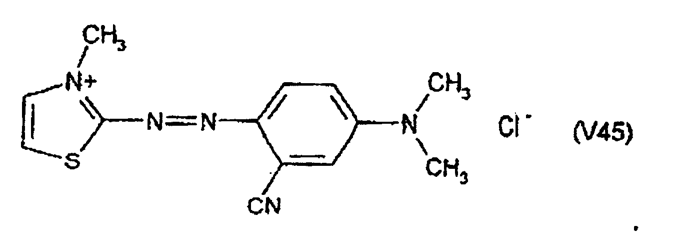 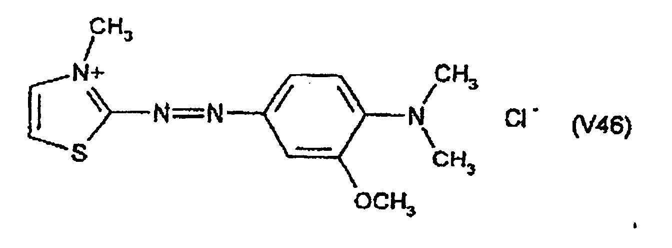 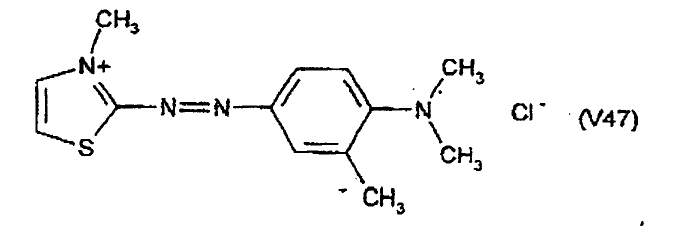 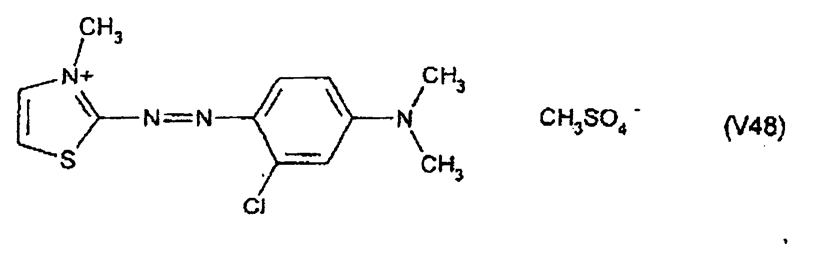 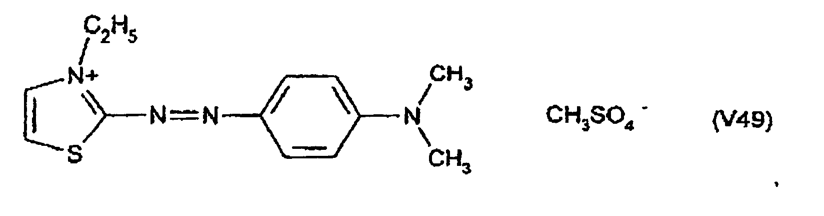 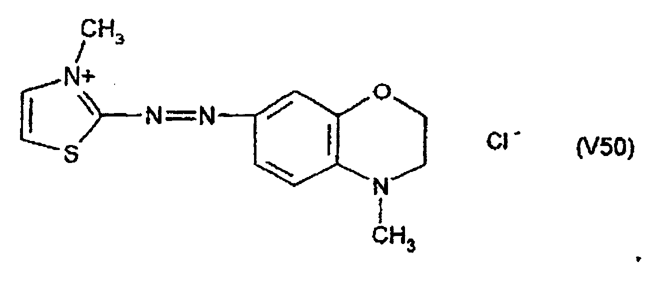 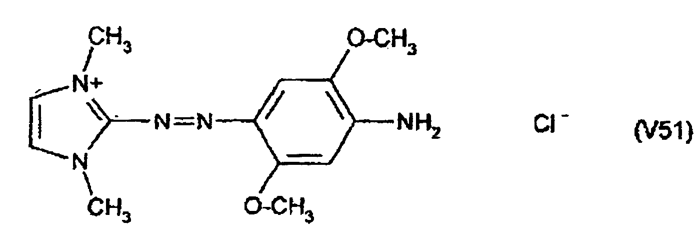 and

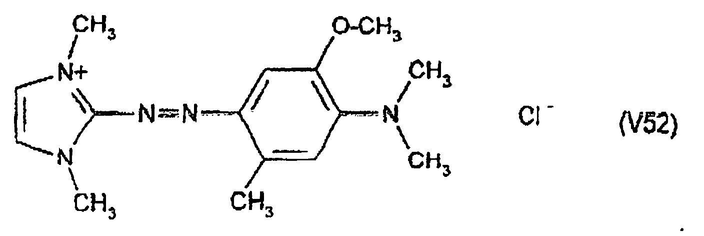

24. Composition according to Claim 22, **characterized in that** the cationic direct dyes of formula (VI) are chosen from the compounds corresponding to structures (VI1) to (VI12) below: 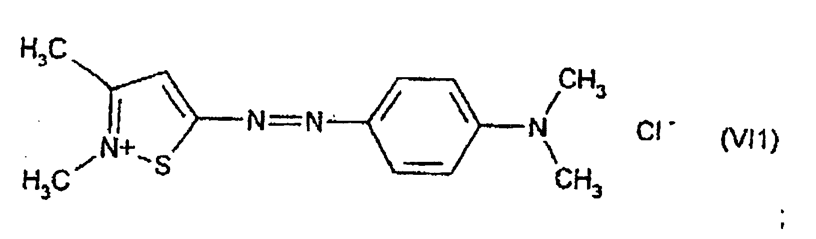 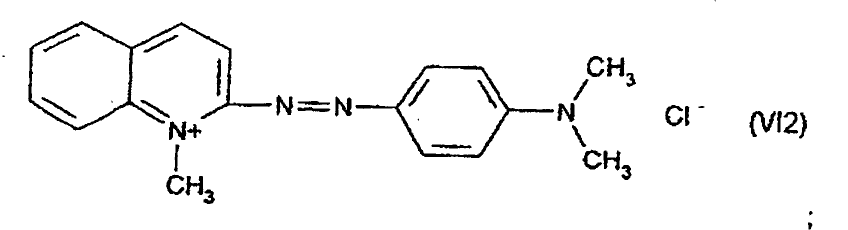 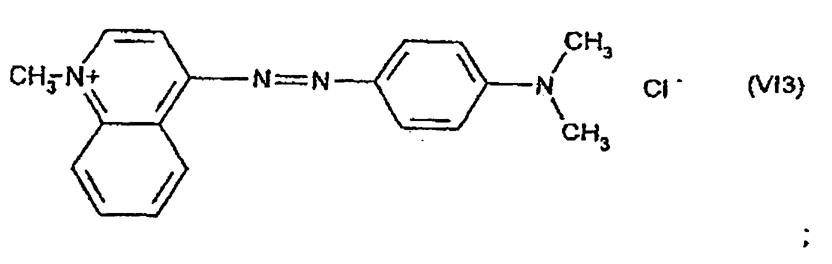 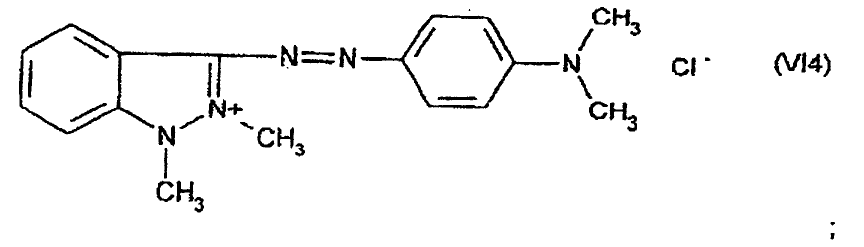 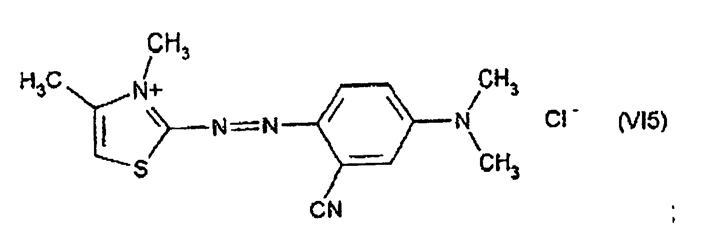 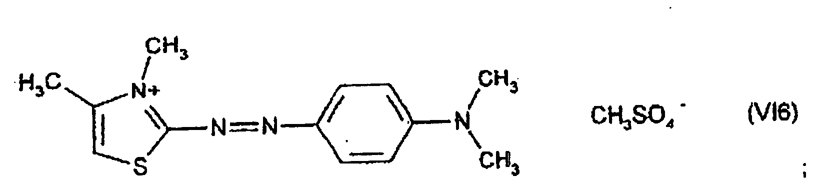 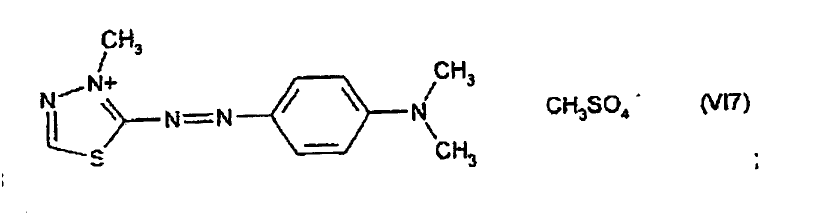 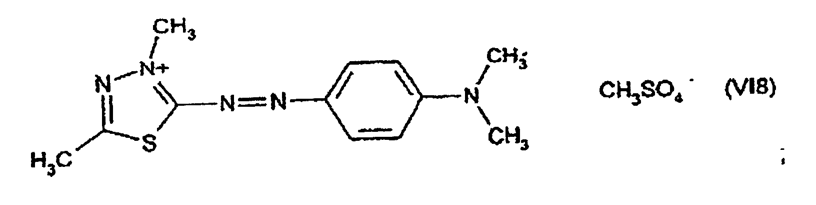 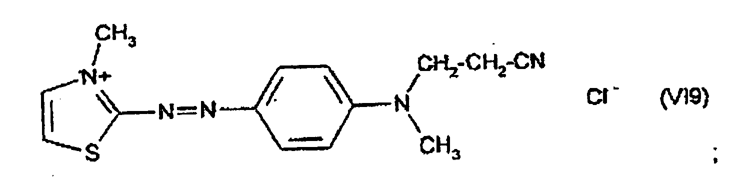 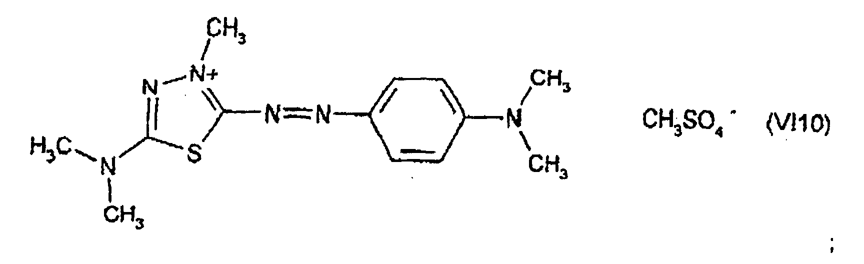 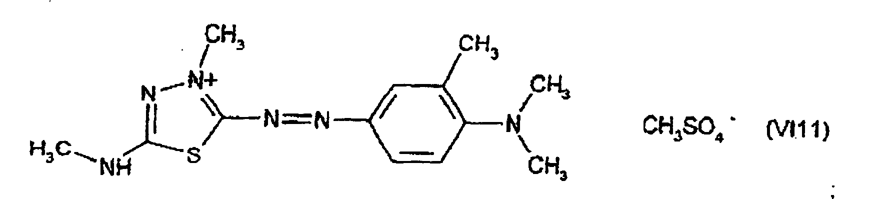 and

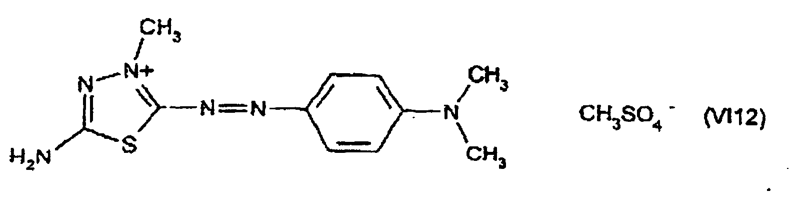

25. Composition according to Claim 22, **characterized in that** the cationic direct dyes of formula (VII) are chosen from the compounds corresponding to structures (VIII) to (VII18) below: 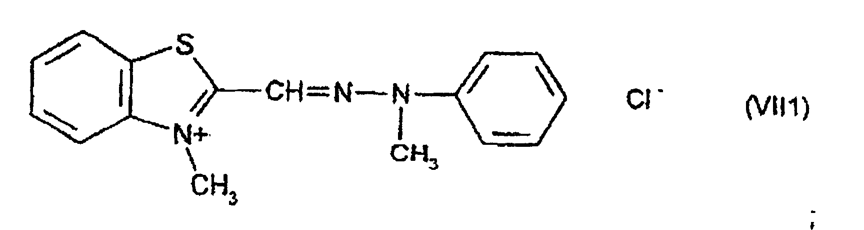 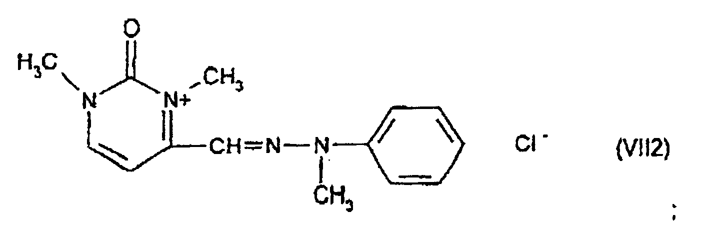 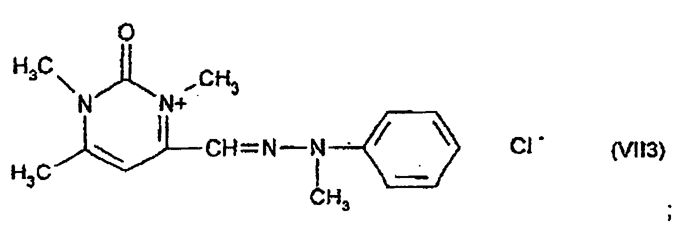 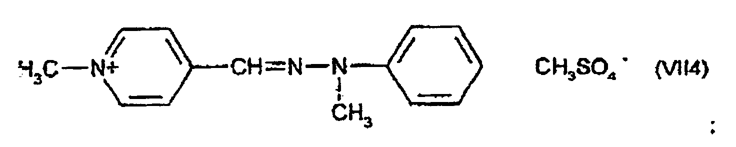 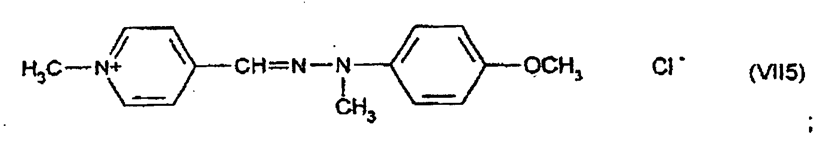 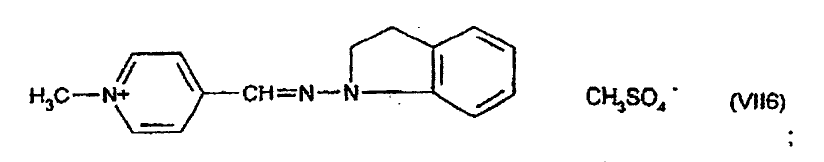 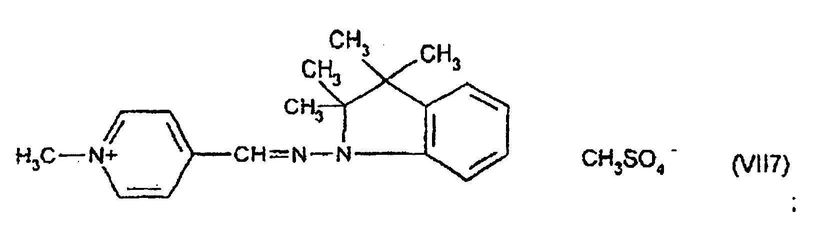 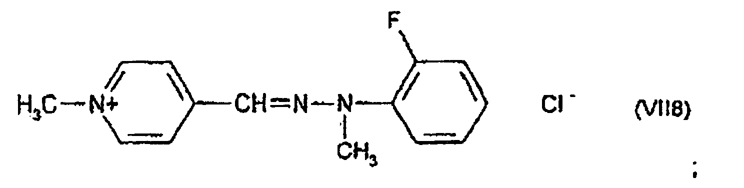 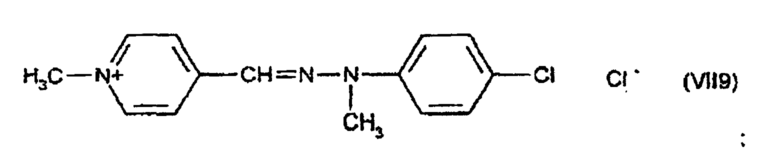 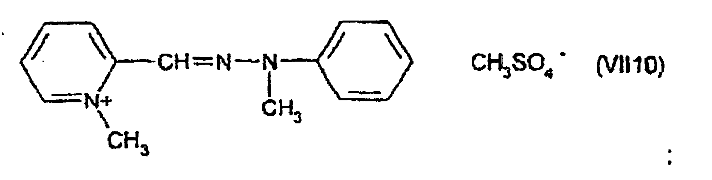 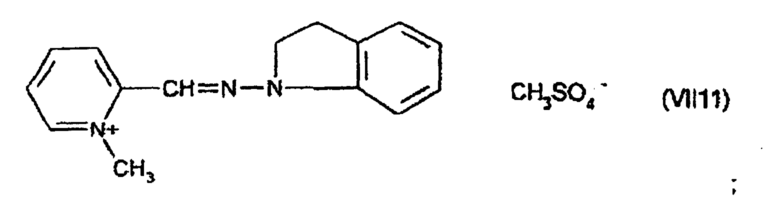 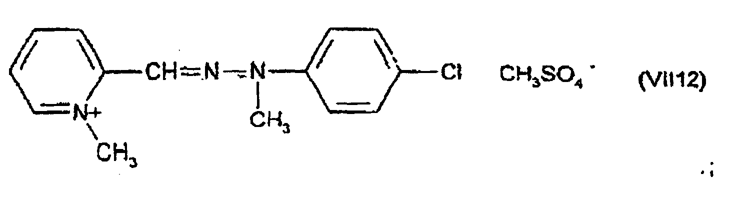 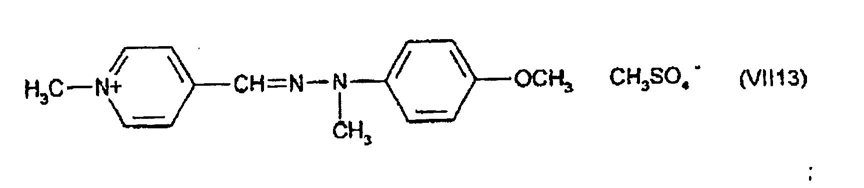 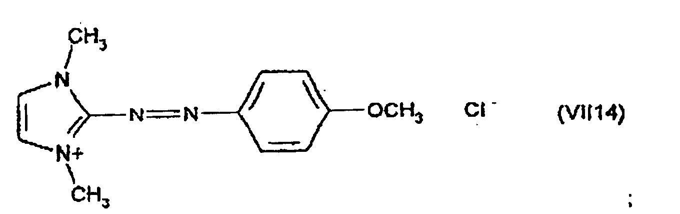 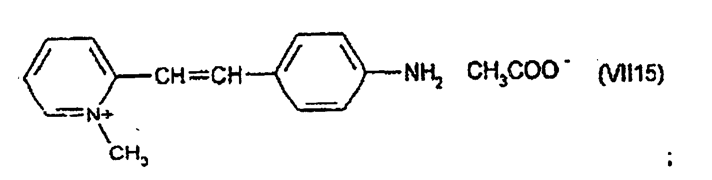 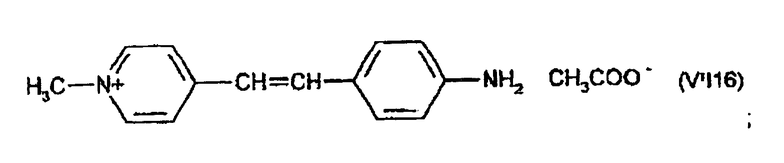 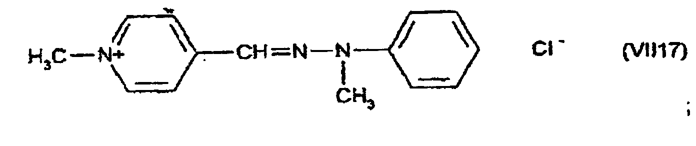 and

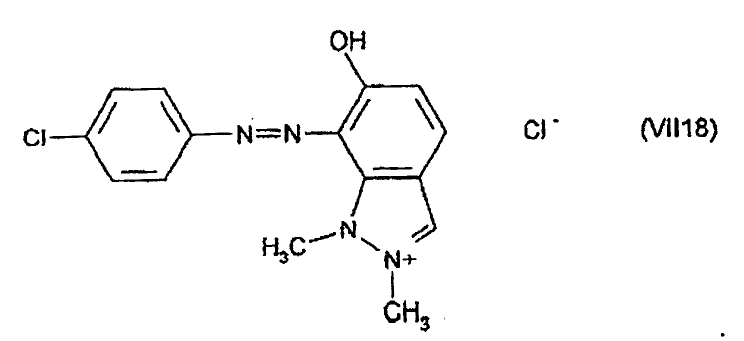

26. Composition according to Claim 22, **characterized in that** the cationic direct dyes of formula (VII') are chosen from the compounds corresponding to structures (VII'1) to (VII'3) below: 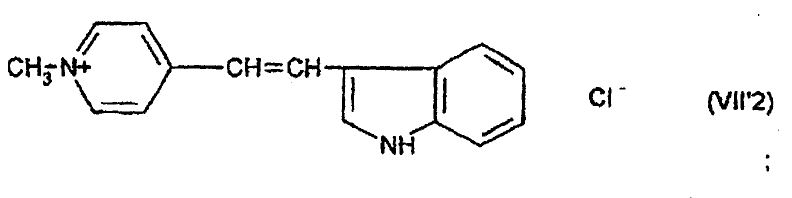 and

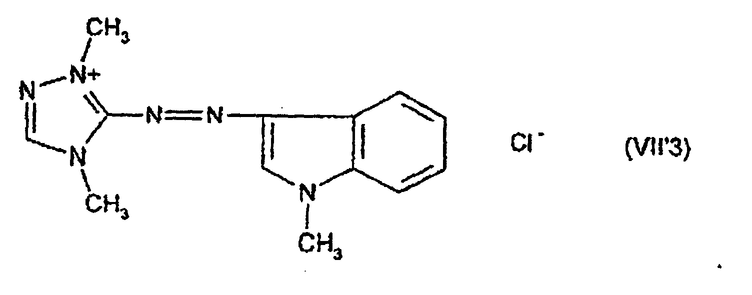

27. Composition according to any one of the preceding claims, **characterized in that** the cationic direct dye(s) represent(s) from 0.001 to 10% by weight relative to the total weight of the ready-to-use dye composition.

28. Composition according to Claim 27, **characterized in that** the cationic direct dye(s) represent(s) from 0.05 to 5% by weight relative to the total weight of the ready-to-use dye composition.

29. Composition according to any one of the preceding claims, **characterized in that** the addition salts with an acid are chosen from the hydrochlorides, hydrobromides, sulphates, tartrates, lactates and acetates.

30. Composition according to any one of the preceding claims, **characterized in that** the medium which is suitable for dyeing consists of water or a mixture of water and at least one organic solvent.

31. Composition according to any one of the preceding claims, **characterized in that** it has a pH of between 5 and 11.

32. Composition according to any one of the preceding claims, **characterized in that** it contains at least one peroxidase.

33. Process for dyeing keratin fibres, and in particular human keratin fibres such as the hair, **characterized in that** at least one ready-to-use dye composition as defined in any one of the preceding claims is applied to the said fibres, for a period which is sufficient to develop the desired coloration.

34. Process according to Claim 33, **characterized in that** it includes a preliminary step which consists in separately storing, on the one hand, a composition (A) comprising, in a medium which is suitable for dyeing, at least one oxidation base and at least one cationic direct dye, and, on the other hand, a composition (B) comprising, in a medium which is suitable for dyeing, at least one enzyme of 2-electron oxidoreductase type in the presence of at least one donor for the said enzyme, and then in mixing them together at the time of use, after which this mixture is applied to the keratin fibres.

35. Multi-compartment dyeing device or "kit", **characterized in that** it includes a first compartment comprising composition (A) as defined in Claim 34 and a second compartment comprising composition (B) as defined in Claim 34.
